# FASCICULE DE BREVET EUROPEEN

(11) **EP 3 649 214 B1**
(45) Date de publication et mention de la délivrance du brevet: **17.03.2021**
(21) Numéro de dépôt: 18742729.9
(22) Date de dépôt: 05.07.2018
(51) Int. Cl.: C09K 11/77, C07F 5/00

(54) **COMPLEXES DE TERBIUM ET DE DYSPROSIUM AVEC ANTENNE OPTIMISÉE, UTILES COMME MARQUEURS LUMINESCENTS**
TERBIUM UND DYSPROSIUMKOMPLEXE MIT OPTIMIERTER ANTENNE ZUR VERWENDUNG ALS LUMINESZENZMARKER
TERBIUM AND DYSPROSIUM COMPLEXES WITH OPTIMISED ANTENNA, USED AS LUMINESCENT MARKERS

(30) Priorité: 06.07.2017 FR 1756385
(43) Date de publication de la demande: 13.05.2020
(73) Titulaire: Ecole Normale Supérieure de Lyon, 69007 Lyon (FR); Centre National de la Recherche Scientifique, 75016 Paris (FR); Université Claude Bernard Lyon 1, 69100 Villeurbanne (FR)
(72) Inventeur: ROUX, Amandine, 38550 Le Peage de Roussillon (FR); BUI, Anh-Thy, 33600 Pessac (FR); MAURY, Olivier, 69126 Brindas (FR); ANDRAUD, Chantal, 69740 Genas (FR)
(74) Mandataire: Icosa
(86) Numéro de dépôt international: PCT/EP2018/068302
(87) Numéro de publication internationale: WO 2019/008118

(56) Documents cités:
- WO-A1-2005/058877
- JIDE XU ET AL: "Octadentate Cages of Tb(III) 2-Hydroxyisophthalamides: A New Standard for Luminescent Lanthanide Labels", JOURNAL OF THE AMERICAN CHEMICAL SOCIETY, AMERICAN CHEMICAL SOCIETY, US, vol. 133, no. 49, 14 décembre 2011 (2011-12-14), pages 19900-19910, XP002755615, ISSN: 0002-7863, DOI: 10.1021/JA2079898 [extrait le 2011-10-19] cité dans la demande
- PLACIDE ET AL.: "Two-photon multiplexing bio-imaging using a combination of Eu- and Tb-bioprobes", DALTON TRANS., vol. 44, 2015, pages 4918-4924, XP002776746, cité dans la demande

## Description

L'invention concerne le domaine technique des complexes de lanthanide. En particulier, l'invention concerne des complexes de lanthanide comportant un agent chélatant, formé d'un macrocycle ou d'un ligand, et complexant un ion lanthanide, des agents chélatants correspondants, ainsi qu'un procédé de détection d'une biomolécule à l'aide dudit complexe de lanthanide.

Certains complexes de lanthanide luminescents présentent des propriétés spectroscopiques remarquables (bandes d'émission fines caractéristiques d'un métal donné et à longue durée de vie), et sont, de ce fait, des composés à très fort potentiel pour des applications en imagerie biologique (S.V. Eliseeva, J.-C. G. Bünzli, Chem. Soc. Rev. 2010, 39, 189). Ces composés luminescents peuvent être utilisés seuls pour des applications en imagerie par microscopie de fluorescence mono- ou biphotonique, ou bien en conjonction avec un fluorophore approprié pour réaliser des expériences de FRET (Förster/Fluorescence Resonant Energy Transfer). Dans ce dernier cas, les complexes de lanthanide sont généralement sous la forme d'un complexe conjugué à une biomolécule. Ces deux techniques peuvent éventuellement être résolues en temps grâce à la durée de vie longue des lanthanides, ce qui constitue un avantage important pour améliorer la détection, en s'affranchissant de signaux de fluorescence parasites à durée de vie courte (C. P. Montgomery B. S. Murray, E. J. New, R. Pal, D. Parker, Acc. Chem. Res. 2009, 42, 925 ; E. G. Moore, A. P. S. Samuel, K. N. Raymond, Acc. Chem. Res. 2009, 42, 542).

De tels composés de lanthanide luminescents ont déjà été décrits dans la littérature, et certains sont même commercialisés par Cisbio Bioassays, PerkinElmer ou Invitrogen. Ces dernières années, des complexes de lanthanide fonctionnalisés ont été développés, afin d'optimiser leurs propriétés spectroscopiques, de déplacer leur absorption vers le visible, optimiser leur absorption biphotonique, et améliorer leur solubilité en milieu aqueux.

En particulier, le document WO 2005/058877 décrit des complexes de lanthanide basés sur des cycles 1,4,7-triazacyclononane (TACN) dont trois atomes d'azote sont substitués par des chromophores constitués d'un dérivé de pyridine éventuellement substitué par un groupe réactif, afin de faciliter la conjugaison avec des biomolécules. L'objectif est de fournir des complexes fortement fluorescents, stables et facilement greffables à une biomolécule. Ce document décrit notamment un complexe de terbium de type TACN dont les trois atomes d'azote sont substitués par un groupe 2-carboxylate-4-(2,4,6-triméthoxyphényle)-pyridine (composé 22), mais n'indique pas la brillance mesurée pour ce composé.

Par ailleurs, certains des inventeurs ont précédemment décrit des complexes de terbium basés sur des cycles 1,4,7-triazacyclononane (TACN) dont trois atomes d'azote sont substitués par des chromophores constitués d'un dérivé de pyridine (V. Placide, A. T. Bui, A. Grichine, A. Duperray, D. Pitrat, C. Andraud, O. Maury, Dalton Trans. 2015, 44, 4918). Ce document décrit en particulier un complexe de terbium de type TACN dont les trois atomes d'azote sont substitués par un groupe 2-carboxylate-4-(4-méthoxyphényle)-pyridine, qui a une absorption intense, mais un rendement quantique et une brillance faibles.

Il subsiste donc un besoin de complexes de lanthanide présentant une bonne brillance, et en particulier lorsqu'ils sont excités dans la gamme spectrale d'intérêt de 330 nm à 350 nm, et plus particulièrement aux alentours de 337 nm (longueur d'onde correspondant aux sources laser souvent utilisées dans les dosages biologiques). La structure des complexes selon l'invention rend possible des variations chimiques permettant d'obtenir avantageusement une bonne solubilité et une bonne stabilité en milieu biologique, et permettant un greffage aisé sur une biomolécule.

La brillance est définie comme le produit de l'absorption à la longueur d'onde d'intérêt et du rendement quantique d'émission, et correspond à la quantité de lumière effectivement disponible pour l'expérience d'imagerie et/ou de FRET. Une brillance optimale permet d'optimiser le rapport signal/bruit, et donc d'augmenter la sensibilité de la détection et/ou de réduire la quantité de chromophore utilisé.

Les Demandeurs proposent de nouveaux complexes de lanthanide comportant un agent chélatant, formé d'un macrocycle ou d'un ligand, complexant un ion lanthanide **Ln³⁺** choisi parmi le terbium et le dysprosium, l'agent chélatant comprenant au moins un groupe de structure (**B**) suivante : 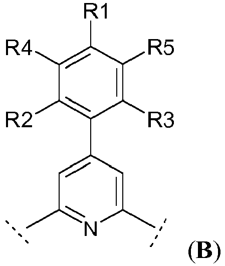 dans laquelle :
- **-R1** représente un hydrogène, un groupe **-R6**, ou un groupe électrodonneur **-E1**,
- **-R2** représente un hydrogène, un groupe **-R7**, ou un groupe électrodonneur **-E2**,
- **-R3** représente un hydrogène, un groupe **-R8**, ou un groupe électrodonneur **-E3**,
- **-R4** et **-R5**, identiques ou différents, représentent indépendamment l'un de l'autre un hydrogène ou un groupe **-R9** ou **-**O**R9**,
- **-E1**, **-E2** et **-E3** sont choisis indépendamment les uns des autres parmi les groupes **-**O**R10,** -S**R10,** -NH(CO)**R10**, -NH(CO)N**R10R'10**, -NH(CS)N**R10R'10** et -NH(CS)NH**R10,**
- **-R6**, **-R7**, **-R8**, **-R9**, **-R10** et **R'10** identiques ou différents, représentent indépendamment les uns des autres un groupe (C1-C6) alkyle, éventuellement substitué par un groupe **-X1** ou un groupe **-Y**,
- **-X1** est un groupe réactif permettant son couplage par liaison covalente à une biomolécule,
- **-Y** est un groupe hydrosolubilisant choisi parmi les groupes -SO₃⁻, -COO⁻, sulfobétaïne et -O-[(CH₂)₂-O]ₘ-CH₃, m étant un nombre entier allant de 1 à 10,
- étant entendu que :
   ∘ au moins l'un des substituants **-R2** et **-R3** n'est pas un hydrogène,
   ∘ un seul des groupes **-R1**, **-R2** et **-R3** représente un groupe électrodonneur,
   ∘ ledit agent chélatant comprend au plus un groupe réactif.

Les complexes de l'invention sont luminescents. La luminescence correspond à une émission de lumière consécutive à un apport d'énergie, en particulier un apport de lumière. Cet apport en énergie fait passer des atomes ou molécules dans un état « excité » se situant à une énergie supérieure à celle qu'ils possèdent dans leur état normal dit « fondamental ». C'est lors du retour à leur état fondamental qu'ils peuvent émettre de la lumière. De manière générale, les complexes de lanthanide sont sensibilisés par effet d'antenne et la nature de l'antenne organique contrôle l'absorption de la lumière et permet d'optimiser la luminescence du lanthanide (S. V. Eliseeva, J.-C. Bünzli, Chem. Soc. Rev. 2010, 39, 189).

Les complexes selon l'invention ont l'avantage d'avoir une brillance élevée, notamment due à un rendement quantique très élevé, en particulier en milieu hydrophile et notamment en milieu aqueux. Sans vouloir être lié par une quelconque théorie, les inventeurs pensent que l'amélioration du rendement quantique, et l'optimisation de la brillance, des complexes de l'invention est due à la combinaison des effets électroniques et stériques des antennes (i.e. des groupes (**B**))**.** La présence d'un seul groupe électrodonneur permet d'améliorer significativement le rendement quantique. Ainsi, de manière surprenante, notamment par rapport au document WO-2005/058877, les inventeurs ont observé une chute drastique du rendement quantique lorsque plusieurs groupes électrodonneurs sont présents, comparé à un même complexe ne comportant qu'un seul groupe électrodonneur. De plus, il apparait que l'introduction de groupements en position R2 et/ou R3 permet vraisemblablement d'éviter la rotation interne de l'antenne autour de la liaison pyridine-phényle qui contribue à augmenter les désexcitations non radiatives et conduit à une diminution du rendement quantique. Ainsi, l'encombrement stérique des groupes **-R2** et/ou - **R3,** qui ne sont pas tous les deux des hydrogènes dans les complexes de l'invention, bloquerait cette rotation et serait donc important pour la brillance finale du complexe.

Par ailleurs, les complexes selon l'invention peuvent être pourvus d'un groupe réactif, pour être éventuellement conjugués par une liaison covalente à une molécule d'intérêt, telle qu'une biomolécule. Les complexes selon l'invention peuvent également être pourvus d'un ou plusieurs groupes hydrosolubilisants, ce qui permet alors d'améliorer leur solubilité en milieu hydrophile, et en particulier en milieu aqueux.

Dans le cadre de l'invention, on entend par « alkyle » une chaîne hydrocarbonée saturée, qui peut être linéaire ou ramifiée. A titre d'exemples de groupes alkyles comprenant de 1 à 6 atomes de carbone, on peut citer les groupes méthyle, éthyle, n-propyle, iso-propyle, n-butyle, iso-butyle, tert-butyle, sec-butyle, n-pentyle, iso-pentyle, tert-pentyle, neo-pentyle, sec-pentyle, et n-hexyle.

De manière préférée dans le cadre de l'invention, le lanthanide **Ln** est le terbium. Les complexes de terbium sont en effet très lumineux.

Avantageusement, les complexes de lanthanide selon l'invention comprennent un groupe réactif, noté **-X**, **-X1** ou **-X2**, permettant son couplage par liaison covalente à une biomolécule. D'après la définition même de l'agent chélatant formant le complexe, un seul groupe réactif peut être présent.

Par « groupe réactif », on entend un groupe comprenant une fonction qui permet un greffage covalent sur une fonction susceptible de réagir et présente sur une biomolécule (amine, alcool, thiol, acide carboxylique, insaturation...). Les différentes fonctions permettant une telle bio-conjugaison sont bien connues de l'homme de l'art et sont décrites, par exemple, dans Bioconjugate Techniques, G.T. Hermanson, 1996, 137-166.

Par « biomolécule », on entend les molécules d'intérêt biologique qu'il peut être avantageux de marquer grâce à un complexe luminescent. A titre d'exemple de biomolécules, on peut citer les protéines, les peptides, les anticorps, les antigènes, les brins d'ADN, la biotine et la streptavidine. Les protéines sont les biomolécules qui seront le plus souvent liées aux complexes de l'invention.

Dans le cadre de l'invention, le groupe réactif peut être choisi parmi -COOH, -NH₂, un acrylamide, une amine activée, un ester activé, un aldhéhyde, un halogénure d'alkyle, un anhydride, une aniline, un azide, une aziridine, un acide carboxylique, un diazoalcane, un haloacétamide, une halotriazine, une hydrazine, un imido ester, un isocyanate, un isothiocyanate, un maléimide, un halogénure de sulfonyle, un thiol, une cétone, un halogénure d'acide, un ester d'hydroxysuccinimidyle, un ester de succinimidyle, un ester d'hydroxysulfosuccinimidyle, un azidonitrophényle, un azidophényle, un 3-(2-pyridyl dithio)-propionamide, un glyoxal, une triazine, un groupe acétylénique, et les groupes de formule : 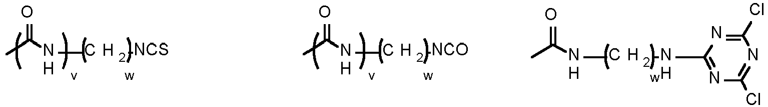 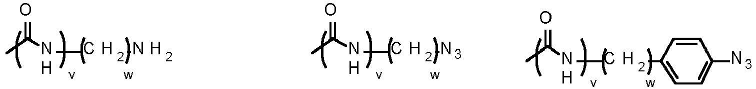 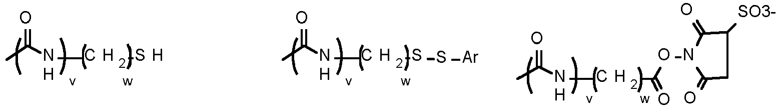 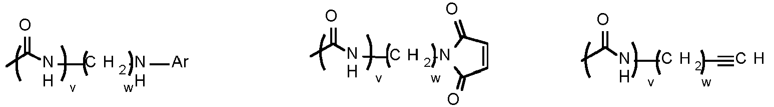 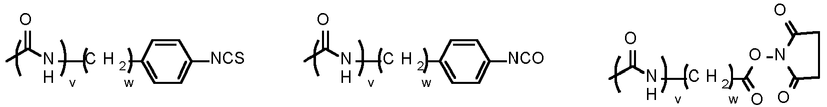 dans lesquels **w** est un entier appartenant à la gamme allant de 0 à 8 et v est égal à 0 ou 1, et **Ar** est un hétérocycle à 5 ou 6 chaînons saturé ou insaturé, comprenant 1 à 3 hétéroatomes, éventuellement substitué par un atome d'halogène ; les groupes réactifs choisis parmi -COOH, -NH₂, les esters de succinimidyle, les haloacétamides, les azides, les hydrazines, les isocyanates et les maléimides étant préférés.

De préférence, les complexes selon l'invention comprennent au moins un et au maximum 15 groupes hydrosolubilisants, notés **-Y**, et avantageusement au moins 2, et de préférence de 2 à 9 groupes hydrosolubilisants, en particulier de 2 à 5, et de préférence 2 ou 3.

Par « groupe hydrosolubilisant », on entend un groupe qui comprend une fonction qui permet d'améliorer la solubilité en milieu hydrophile.

Dans le cadre de l'invention, le ou les groupes hydrosolubilisants **-Y** peuvent être choisis parmi les groupes -SO₃⁻, -COO⁻, sulfobétaïne et -O-[(CH₂)₂-O]ₘ-CH₃, m étant un nombre entier allant de 1 à 10, de préférence m=3. Selon un mode de réalisation préféré, le ou les groupes hydrosolubilisants sont choisis parmi les groupes -O-[(CH₂)₂-O]ₘ-CH₃, m étant un nombre entier allant de 1 à 10, de préférence m=3.

Par « sulfobétaïne », on entend un groupement bétaïne comportant une fonction -SO₃⁻ en tant que fonction porteuse de la charge négative. Un groupe bétaïne est défini comme un groupement zwittérionique dans lequel l'atome portant la charge positive ne porte pas d'atome d'hydrogène et n'est pas adjacent à l'atome porteur de la charge négative. Dans le cadre de l'invention, on entend par sulfobétaïne, par exemple, les groupements zwittérioniques associant un cation ammonium, ou iminium aromatique, généralement pyridinium, imidazollium, et un groupement anionique de type sulfonate. Le cation et l'anion sont espacés par au moins un chainon CH₂, et de préférence par une chaine alkyle bivalente (également nommé alkylène) comprenant de 1 à 4, voire de 1 à 6, atomes de carbone. A titre d'exemple de groupe sulfobétaïne, on peut citer les groupes de formule : 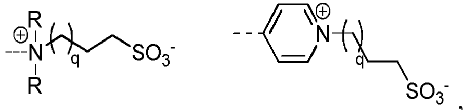 avec R qui représente un groupe alkyle de 1 à 6 atomes de carbone, et de préférence un méthyle ou éthyle, et q qui est égal à 1, 2, 3, 4, 5 ou 6, et de préférence qui est égal à 1 ou 2. Le groupe -N(CH₃)₂⁺-(CH₂)₃-SO₃⁻ est préféré.

Lorsque les complexes selon l'invention comprennent un ou plusieurs groupes hydrosolubilisants, ceux-ci peuvent être sur n'importe quel substituant du groupe phényle du groupe de structure (**B**), i.e. sur **-R1**, **-R2, -R3, -R4** et/ou **-R5**, ou plus précisément sur **-R6**, **-R7**, **-R8**, **-R9**, **-R10** et/ou **-R'10**. De manière préférée, les groupes hydrosolubilisants sont en position ortho et/ou para par rapport au groupement pyridinyle du groupe de structure (**B**), i.e. sur **-R6**, **-R7**, **-R8**, **-R10** et/ou **-R'10**, et de préférence en para.

Selon un premier mode de réalisation, les complexes de lanthanide selon l'invention sont choisis parmi les complexes de lanthanide de formule **(I)** : 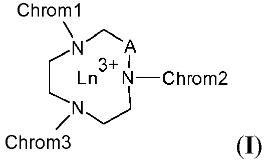 dans lesquels :
- **Ln** est un lanthanide choisi parmi Tb et Dy,
- **-A-** représente -CH₂- ou -CH(**L_{A}-X2**)-,
- **-L_{A}-** est une liaison covalente, ou un groupe (C1-C20) alkylène, linéaire ou ramifié, contenant éventuellement une ou plusieurs doubles ou triples liaisons, et éventuellement substitué par un à trois groupes -SO₃H, ou **-L_{A}-** est un groupe (C5-C8) cycloalkylène ou un groupe (C6-C14) arylène,
- **-X2** est un groupe réactif permettant son couplage par liaison covalente à une biomolécule,
- **-Chrom1**, **-Chrom2** et **-Chrom3** sont identiques ou différents et choisis indépendamment les uns des autres parmi les groupes de formule **(B1)** : 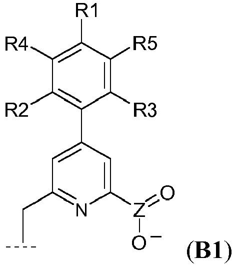
- **-R1**, **-R2**, **-R3**, **-R4**, et **-R5** sont tels que définis pour le groupe de formule (**B**),
- **-Z-** représente -C- ou **-P(R_{Z})-**, et
- **-R_{Z}** représente un groupe phényle, benzyle, méthyle, éthyle, propyle, n-butyle, sec-butyle, iso-butyle ou tert-butyle, et de préférence un groupe phényle ou méthyle,
- étant entendu que si **-A-** est -CH**(L_{A}-X2)**-, alors aucun des groupes **-Chrom1**, **-Chrom2** et **-Chrom3** de formule (**B1**) ne comprend de groupe **-X1**.

De préférence, dans les complexes de formule **(I)**, **-A-** représente -CH₂-, et/ou **-Z-** représente **-C-**, et/ou **-R4** représente un hydrogène, et/ou **-R5** représente un hydrogène dans **-Chrom1**, **-Chrom2** et **-Chrom3**.

De préférence, dans les complexes de formule **(I)**, **-R4** et **-R5** représentent des hydrogènes dans **-Chrom1**, **-Chrom2** et **-Chrom3**.

Selon un mode de réalisation préféré, les complexes de lanthanide **(I)** sont tels que **-A-**représente -CH₂-, **-Z-** représente -C- et **-R4** = **-R5** = H dans **-Chrom1**, **-Chrom2** et **-Chrom3**.

Selon un mode de réalisation, **-Chrom1**, **-Chrom2** et **-Chrom3** sont identiques dans les complexes (**I**). Ces complexes seront nommés (**la**). Ces complexes (**la**) présentent alors l'avantage d'être plus faciles à synthétiser.

Selon un mode de réalisation, dans les complexes (**Ia**), **-R1** représente un groupe électrodonneur **-E1** tel que défini pour les groupes de formule (**B**), notamment choisi parmi les groupes -O**R10** et -NH(CO)**R10**, **-R10** étant tel que défini pour les groupes de formule (**B**). De préférence, **-R1** représente un groupe **-**O**R10**, et en particulier -OMe ou -OPEG, dans les complexes (**Ia**).

Selon un mode de réalisation, dans les complexes (**Ia**), **-R2** est un groupe **-R7** tel que défini pour les groupes de formule (**B**). De préférence, dans les complexes (**Ia**), **-R2** représente -Me.

Selon un mode de réalisation, dans les complexes (**Ia**), **-R3** est avantageusement un hydrogène ou un groupe **-R8** tel que défini pour les groupes de formule (**B**), préférentiellement -Me. De préférence, **-R3** est un hydrogène dans les complexes (**Ia**).

Selon un mode de réalisation particulier des complexes de formule (**I**), **-Chrom1**, **-Chrom2** et **-Chrom3** sont identiques et tels que **-R1** représente un groupe **-E1**, notamment choisi parmi les groupes **-**O**R10** et -NH(CO)**R10**, **-R2** est un groupe **-R7**, et en particulier -Me, et **-R3** est un hydrogène ou un groupe **-R8**, de préférence -Me, **-E1**, **-R7**, **-R8** et **-R10** étant tels que définis pour les groupes de formule (**B**).

Selon un mode de réalisation particulier des complexes de formule **(I)**, **-Chrom1**, **-Chrom2** et **-Chrom3** sont identiques et tels que **-R1** représente un groupe **-**O**R10, -R2** est un groupe **-R7**, et en particulier -Me, et **-R3** est un hydrogène, **-R7** et **-R10** étant tels que définis pour les groupes de formule (**B**).

Selon un autre mode de réalisation, **-Chrom1** et **-Chrom2** sont identiques et **-Chrom3** est différent de **-Chrom1** et de **-Chrom2**. Ces complexes seront nommés **(Ib)**.

Selon un mode particulier de réalisation, les complexes de lanthanide **(Ib)** sont tels que :
- **-A-** représente -CH₂-,
- **-Chrom1** et **-Chrom2** sont identiques et sont de structure **(B2)** : 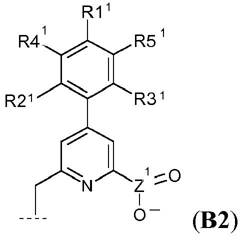 avec **-R1¹**, **-R2¹**, **-R3¹**, **-R4¹**, **-R5¹** et **-Z¹-** tels que définis respectivement pour **R1**, **-R2**, **-R3**, **-R4**, **-R5** et **-Z-** dans les groupes de formule (**B**), étant entendu que **Chrom1** et **-Chrom2** ne comprennent pas de groupe réactif, et
- **-Chrom3** est différent de **-Chrom1** et de **-Chrom2** et est de structure (**B3**) : 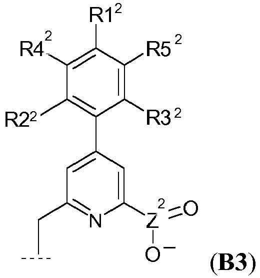 avec **-R1²**, **-R2²**, **-R3²**, **-R4²**, **-R5²** et **-Z²-** tels que définis respectivement pour **R1**, **-R2**, **-R3**, **-R4**, **-R5** et **-Z-** dans les groupes de formule (**B**), étant entendu que **-Chrom3** comprend un groupe réactif.

Selon un mode de réalisation, dans les complexes (**Ib**), **-R1¹** représente un groupe -O**R10¹**, de préférence -OMe ou -OPEG, ou un groupe -NH(CO)**R10¹**, **-R10¹** étant tel que défini pour **-R10** dans le groupe de formule (**B**). De préférence, dans les complexes (**Ib**), **-R1¹** représente un groupe -O**R10¹**, de préférence -OMe ou -OPEG, **-R10¹** étant tel que défini pour **-R10** dans le groupe de formule (**B**).

Dans les complexes (**Ib**), **-R1²** représente avantageusement un groupe -NH(CO)**R10²**, ou, de préférence -O**R10²**, avec **R10²** qui est un groupe -O(C1-C6) alkyle substitué par un groupe **-X1**.

Dans les complexes (**Ib**), **-R2¹** et **-R2²**, identiques ou différents, représentent avantageusement un groupe **-R7** tel que défini pour les groupes de formule (**B**). De préférence, dans les complexes (**Ib**), **-R2¹** et **-R2²** sont identiques et représentent, préférentiellement, -Me.

Dans les complexes (**Ib**), **-R3¹** et **-R3²**, identiques ou différents, représentent un hydrogène ou un groupe **-R8** tel que défini pour les groupes de formule (**B**). De préférence, dans les complexes (**Ib**), **R3¹** et **-R3²** sont identiques et représentent -Me ou, préférentiellement, un hydrogène.

Selon un mode de réalisation particulier, dans les complexes **(Ib), -R1¹** représente un groupe -O**R10¹**, de préférence -OMe ou -OPEG, et **-R1²** représente -O**R10²**, de préférence un groupe - O(C1-C6) alkyle substitué par un groupe **-X1**, **-R10¹** et **-R10²** étant tel que défini pour **-R10** dans les groupes de formule (**B**).

Selon un mode de réalisation particulier, dans les complexes **(Ib), -R2¹** et **-R2²** sont identiques et représentent un groupe **-R7,** et **-R3¹** et **-R3²** sont identiques et représentent un hydrogène ou un groupe **-R8** tel que -Me, les groupes **-R7** et **-R8** étant tels que définis pour les groupes de formule (**B**).

Selon un mode de réalisation particulier, dans les complexes (**Ib**), **-R2¹** et **-R2²** sont identiques et représentent un groupe **-R7** tel que défini pour les groupes de formule (**B**), et préférentiellement -Me, et **-R3¹** et **-R3²** sont identiques et représentent un hydrogène.

Selon un mode de réalisation particulier des complexes de formule **(Ib), -R1¹** représentent un groupe -O**R10¹**, de préférence -OMe ou -OPEG, **-R1²** représente un groupe -O**R10²**, de préférence un groupe -O(C1-C6) alkyle substitué par un groupe **-X1**, **-R2¹** et **-R2²** sont identiques et représentent un groupe **-R7**, de préférence -Me et **-R3¹** et **-R3²** sont identiques et représentent un hydrogène, **-R1¹**, **-R1²**, **-R2¹**, **-R2²**, **R3¹**, **-R3²** et **-R10¹** tels que définis respectivement pour **-R1**, - **R1**, **-R2**, **-R2**, **R3**, **-R3** et **-R10** pour les groupes de formule (**B**).

Avantageusement, dans les groupes de formule (**B**), un des groupes **-R2** ou **-R3** représente un hydrogène. De même, dans les groupes de formule (**B1**), un des groupes **-R2¹** ou **-R3¹** représente avantageusement un hydrogène. De même, dans les groupes de formule (**B2**), un des groupes - **R2²** ou **R3²** représente avantageusement un hydrogène.

En particulier, **-Chrom1**, **-Chrom2** et **-Chrom3** sont identiques ou différents, et choisis indépendamment les uns des autres parmi les groupes de formule (**B4**) suivante : 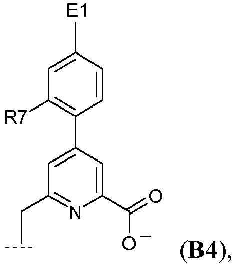 avec **-R7** et **-E1** tels que définis pour les groupes de formule (**B**), **-R7** représentant de préférence -Me, et **-E1** représentant de préférence un groupe **-**O**R10** ou -NH(CO)**R10** éventuellement substitué par un groupe -Y ou un groupe **-X1**, **R10** étant tel que défini pour les groupes de formule (**B**).

Selon un mode de réalisation particulier, les complexes de lanthanide (**I**) selon l'invention sont tels que :
- **-R1¹** est un groupe -O(C1-C6) alkyle substitué par un groupe **-Y**,
- **-R1²** est un groupe -O(C1-C6) alkyle substitué par un groupe **-X1**,
- **-R2¹** et **-R2²**, identiques ou différents, représentent un groupe **-R7** tel que défini pour les groupes de structure (**B**), et sont de préférence identiques et représentent, préférentiellement, un groupe (C1-C6) alkyle, notamment -Me, et
- **-R3¹**, **-R3²**, **-R4¹**, **-R4²**, **-R5¹** et **-R5²** sont des hydrogènes.

Selon ce mode particulier de réalisation, les complexes de lanthanide sont de formule (**Ib'**) : 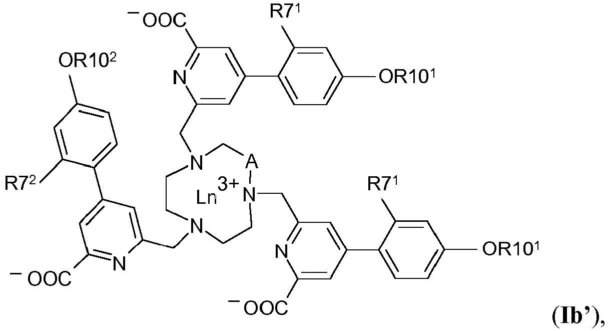 avec **A** tel que défini pour les complexes (**I**), **-R7**¹ et **-R7²** tels que définis pour **-R7** pour les groupes de formule (**B**), **-R10¹** représentant un groupe (C1-C6) alkyle substitué par un groupe **-X1** et **-R10²** représentant un groupe (C1-C6) alkyle substitué par un groupe **-Y**.

Selon un mode de réalisation particulier de l'invention, les complexes de lanthanide (**I**) sont choisis parmi les complexes suivants :

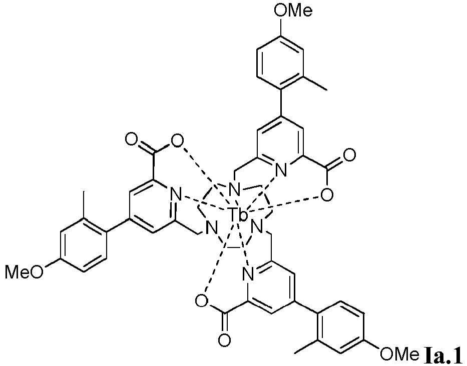

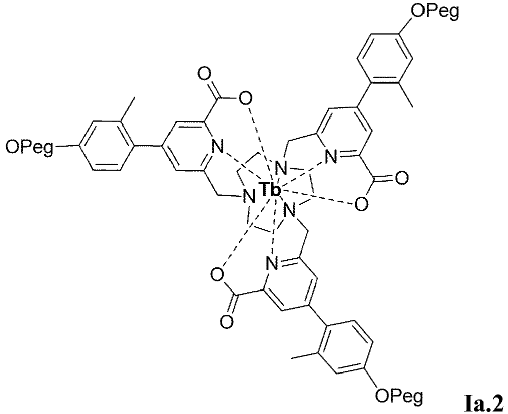

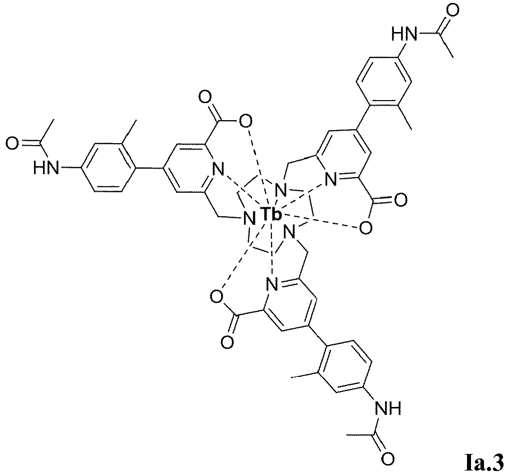

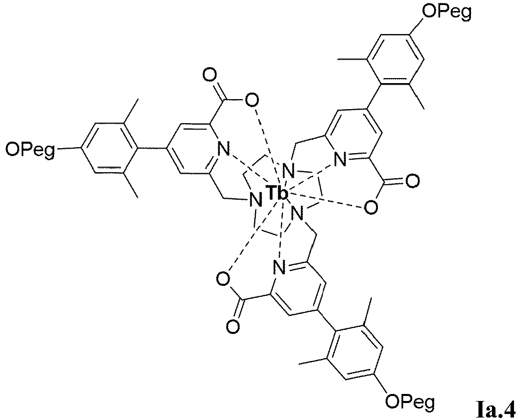

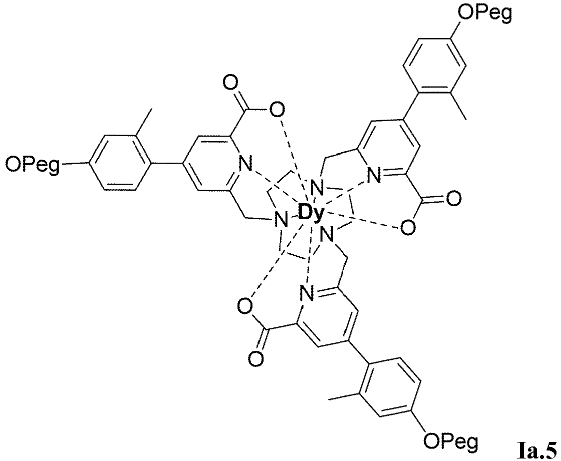

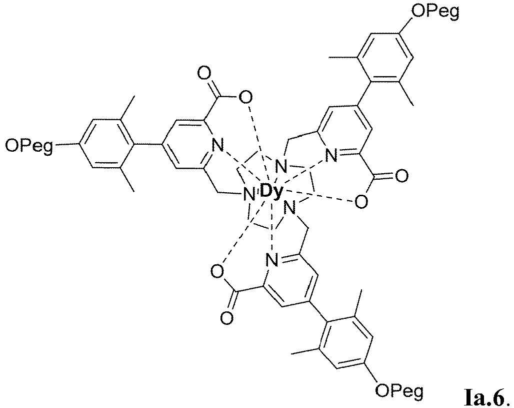

Un complexe de lanthanide particulièrement préféré est le complexe (**Ia.2**).

Selon un second mode de réalisation, les complexes de lanthanide selon l'invention sont choisis parmi les complexes de lanthanide de formule (**II**) : 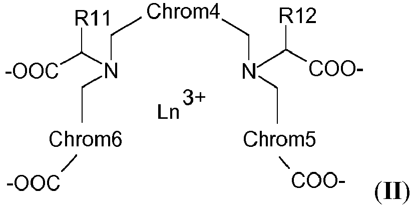 dans lesquels :
- **Ln** est un lanthanide choisi parmi Tb et Dy,
- **-R11** et **-R12,** identiques ou différents, sont choisis indépendamment l'un de l'autre parmi **-X1**, **-Y,** un atome d'hydrogène ou un groupe (C1-C6) alkyle,
   **-Chrom4-**, **-Chrom5-**, et **-Chrom6-**, identiques ou différents, représentent indépendamment l'un de l'autre un groupe de formule (**B**), tel que défini dans le cadre de l'invention.

De préférence, **-R4** et/ou **-R5** représente un hydrogène dans **-Chrom4-**, **-Chrom5-** et **-Chrom6-**. De préférence, **-R4** et **-R5** représentent des hydrogènes dans **-Chrom4-**, **-Chrom5-** et - **Chrom6-**.

Selon un mode de réalisation, **-Chrom4-**, **-Chrom5-** et **-Chrom6-** sont identiques. Ces complexes seront nommés (**IIa**)**.**

Dans les complexes (**IIa**), **-R1** représente avantageusement un groupe électrodonneur **-E1** tel que défini pour les groupes de formule (**B**), notamment choisi parmi les groupes **-**O**R10** et -NH(CO)**R10**, **-R10** étant tel que défini pour les groupes de formule (**B**). De préférence, **-R1** représente un groupe **-OR10,** et en particulier -OMe ou -OPEG dans les complexes (**IIa**)**.**

Dans les complexes (**IIa**), **-R2** est avantageusement un groupe **-R7** tel que défini pour les groupes de formule (**B**). De préférence, **-R2** représente -Me dans les complexes (**IIa**).

Dans les complexes (**IIa**), **-R3** est avantageusement un hydrogène ou un groupe **-R8** tel que défini pour les groupes de formule (**B**), préférentiellement -Me. De préférence, **-R3** est un hydrogène dans les complexes (**IIa**).

Selon un autre mode de réalisation, les complexes de lanthanide (**II**), nommés (**IIb**), sont tels que :
- **-Chrom5-** et **-Chrom6-** sont identiques et sont de structure (**B10**) : 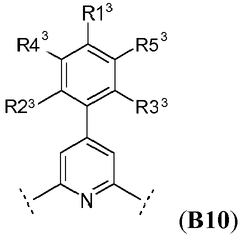 avec **-R1³**, **-R2³**, **-R3³**, **-R4³**, et **-R5³** tels que définis respectivement pour **-R1**, **-R2**, **-R3**, **-R4**, et **-R5** dans les groupes de structure (**B**), étant entendu que **-Chrom5-** et **-Chrom6-** ne comprennent pas de groupe réactif, et
- **-Chrom4-** est différent de **-Chrom5-** et de **-Chrom6-** et est de structure (**B11**) : 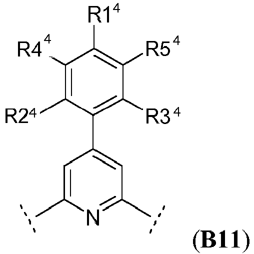 avec **-R1⁴**, **-R2⁴**, **-R3⁴**, **-R4⁴** et **-R5⁴** tels que définis respectivement pour **-R1**, **-R2**, **-R3**, **-R4** et **-R5** dans les groupes de structure (**B**), étant entendu que **-Chrom4-**comprend un groupe réactif -X1 permettant son couplage par liaison covalente à une biomolécule.

Selon un autre mode de réalisation, les complexes de lanthanide (**II**), nommés (**IIc**) sont tels que :
- **-Chrom4-** et **-Chrom6-** sont identiques et sont de structure **(B12)** : 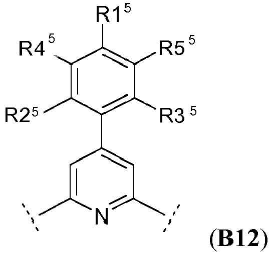 avec **-R1⁵**, **-R2⁵**, **-R3⁵**, **-R4⁵** et **-R5⁵** tels que définis respectivement pour **-R1**, **-R2**, **-R3**, **-R4** et **-R5** dans les groupes de formule (**B**), étant entendu que **-Chrom4-** et **-Chrom6-** ne comprennent pas de groupe réactif, et
- **-Chrom5-** est différent de **-Chrom4-** et de **-Chrom6-** et est de structure (**B13**) : 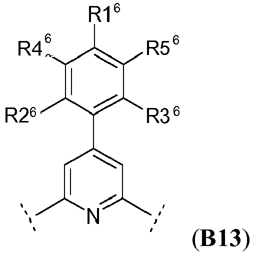 avec **-R1⁶, -R2⁶, -R3⁶, -R4⁶** et **-R5⁶** tels que définis respectivement pour **-R1**, **-R2, -R3**, **-R4** et **-R5** dans les groupes de formule (**B**), étant entendu que **-Chrom5-** comprend un groupe réactif -X1 permettant son couplage par liason covalente à une biomolécule.

Avantageusement, dans les complexes (**IIb**) et (**IIc**), **-R1³** et **-R1⁵**, lorsqu'ils sont présents, sont des groupes -O(C1-C6) alkyle ou -NH(CO)(C1-C6) alkyle éventuellement substitué par un groupe **-Y**. De préférence dans les complexes (**IIb**) et (**IIc**)**, -R1³** et **-R1⁵** sont des groupes -O(C1-C6) alkyle éventuellement substitué par un groupe **-Y**, et en particulier -OMe ou -OPEG.

Avantageusement dans les complexes **(IIb)** et **(IIc)**, **-R1⁴** et **-R1⁶**, lorsqu'ils sont présents, représentent un groupe -O(C1-C6) alkyle ou NH(CO)(C1-C6) alkyle substitué par un groupe **-X1**. De préférence dans les complexes **(IIb)** et **(IIc)**, **-R1⁴** et **-R1⁶** sont des groupes -O(C1-C6) alkyle éventuellement substitué par un groupe **-X1**.

Dans les complexes (**IIb**) et (**IIc**), **R2³**, **-R2⁴**, **-R2⁵** et **-R2⁶,** lorsqu'ils sont présents, peuvent être identiques ou différents, et représenter un groupe -R7 tel que défini pour les groupes de formule (**B**). De préférence dans les complexes (**IIb**) et (**IIc**), **R2³**, **-R2⁴**, **-R2⁵** et **-R2⁶**, lorsqu'ils sont présents, sont identiques et représentent, préférentiellement -Me.

Dans les complexes (**IIb**) et (**IIc**), **R2³**, **-R2⁴**, **-R2⁵,** et **-R2⁶**, lorsqu'ils sont présents, peuvent être identiques ou différents et représenter un hydrogène ou un groupe **-R8** tel que défini pour les groupes de formule (**B**), préférentiellement -Me. De préférence, **R2³**, **-R2⁴**, **-R2⁵**, **-R2⁶**, lorsqu'ils sont présents, sont identiques et représentent, préférentiellement un hydrogène.

Dans les complexes (**IIb**) et (**IIc**), **R3³**, **-R3⁴**, **-R3⁵** et **-R3⁶**, lorsqu'ils sont présents, peuvent être identiques ou différents et représenter un hydrogène ou un groupe **-R8** tel que défini pour les groupes de formule (**B**), préférentiellement -Me. De préférence, **R3³**, **-R3⁴**, **-R3⁵** et **-R3⁶,** lorsqu'ils sont présents, sont identiques et représentent un hydrogène.

De préférence dans les complexes (**IIb**) et (**IIc**), **-R3³**, **-R3⁴**, **-R3⁵** et **-R3⁶,** lorsqu'ils sont présents, sont identiques et représentent avantageusement des hydrogènes, et **-R2³**, **-R2⁴**, **-R2⁵** et - **R2⁶**, lorsqu'ils sont présents, sont identiques et représentent chacun un groupe **-R7**, tel que défini pour les groupes de formule (**B**), de préférence -Me.

En particulier, **Chrom4-, -Chrom5-** et **-Chrom6-** sont identiques ou différents, et choisis indépendamment les uns des autres parmi les groupes de formule (**B14**) suivante : 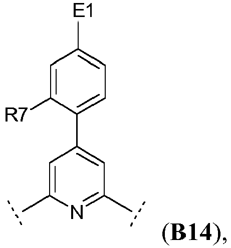 avec **-R7** et **-E1** tels que définis pour les groupes de formule (**B**), et **-R7** représentant de préférence -Me et **-E1** représentant de préférence un groupe **-**O**R10** ou -NCO(N**R10**) éventuellement substitué par un groupe **-Y** ou un groupe **-X1**, **R10** étant tel que défini pour les groupes de formule (**B**).

Selon un mode de réalisation particulier de l'invention, les complexes de lanthanide (**IIb**) sont tels que :
- **-R1³** est un groupe -O(C1-C6) alkyle éventuellement substitué par un groupe **-Y**,
- **-R1⁴** est un groupe -O(C1-C6) alkyle substitué par un groupe **-X1**,
- **-R2³** et **-R2⁴** sont identiques ou différents, de préférence identiques, et représentent un groupe -R7, et en particulier un groupe (C1-C6) alkyle, et
- **-R3³**, **R3⁴**, **-R4³**, **-R4⁴**, **-R5³** et **-R5⁴** sont des hydrogènes.

Selon un mode de réalisation particulier de l'invention, les complexes de lanthanide (**IIc**) sont tels que :
- **-R1⁵** est un groupe -O(C1-C6) alkyle éventuellement substitué par un groupe **-Y**,
- **-R1⁶** est un groupe -O(C1-C6) alkyle substitué par un groupe **-X1**,
- **-R2⁵** et **-R2⁶** sont identiques ou différents, de préférence identiques, et représentent un groupe -R7, et en particulier un groupe (C1-C6) alkyle, et
- **-R3⁵**, **R3⁶, -R4⁵, -R4⁶, -R5⁵** et **-R5⁶** sont des hydrogènes.

L'invention a également pour objet les agents chélatants correspondant aux composés de lanthanide définis dans le cadre de l'invention, et ce quelle que soit leur variante de réalisation décrite. L'invention concerne aussi de tels macrocycles chélatants sous leur forme protégée selon la formule (**III**) et à de tels ligands chélatants sous leur forme protégée selon la formule (**IV**).

L'invention concerne également les agents chélatants de formule (**III**) : 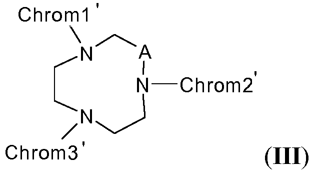 avec **-Chrom1', -Chrom2'**, et **-Chrom3'**, identiques ou différents, et choisis indépendamment les uns des autres parmi les groupes de formule (**B5**) : 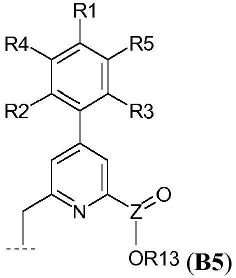 avec **R1**, **R2**, **R3**, **R4** et **R5** tels que définis pour les complexes de lanthanide (**I**), et **-R13** un groupe protecteur des fonctions acide (i.e. -Z(O)OH), tel qu'un alkyle, et notamment un méthyle, ou sous la forme d'un sel.

L'invention a aussi pour objet les agents chélatants de formule (**IV**) : 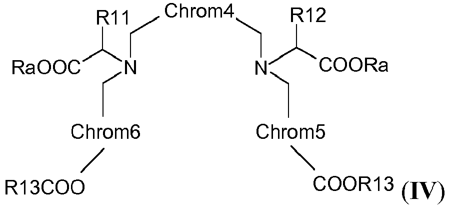 avec **-Chrom4-**, **-Chrom5-** et **-Chrom6-** tels que définis pour les complexes de lanthanide de formule (**II**), et **-R13** un groupe protecteur des fonctions acide, tel qu'un alkyle, et notamment un méthyle, ou sous la forme d'un sel.

La préparation des complexes (**I**) et (**II**) selon l'invention met en œuvre des techniques et des réactions classiques connues de l'homme de l'art. Ils peuvent, notamment, être obtenus selon des procédés analogues à ceux décrits dans les exemples.

Les réactions de complexation sont, en général, menées avec un sel du lanthanide souhaité, notamment un chlorure, un nitrate ou un triflate, dans un solvant dissociant, notamment un alcool tel que le méthanol, le THF ou l'acétonitrile, ou encore dans un mélange de solvants, en présence généralement d'un carbonate, à une température comprise entre 25 et 80 °C, pendant une durée variant de 30 minutes à quelques heures.

Les composés (**III**) et (**IV**) sont d'accès relativement aisé et classique, et peuvent être obtenus à un coût de préparation raisonnable. Les réactifs sont en effet disponibles dans le commerce ou facilement accessibles.

Les groupes fonctionnels éventuellement présents dans les composés de formule (**III**) et (**IV**), et dans les intermédiaires réactionnels, peuvent être protégés au cours des synthèses, soit sous forme permanente soit sous forme temporaire, par des groupes protecteurs qui assurent une synthèse univoque des composés attendus. Les réactions de protection et de déprotection sont effectuées selon des techniques bien connues de l'homme de l'art. Par groupe protecteur temporaire, on entend les groupes protecteurs tels que ceux décrits dans Protective Groups in Organic Synthesis, Greene T. W. et Wuts P. G. M., ed. John Wiley & Sons, 2006**,** et dans Protecting Groups, Kocienski P. J. 1994, Georg Thieme Verlag.

Selon la formule (**I**) est décrite la famille de complexes de lanthanide issus de la coordination d'un ligand macrocyclique de formule (**III**) après déprotection et libération des fonctions acide - Z(O)OH (acide dialkyle ou diaryle phosphinique lorsque **-Z-** = -P(**Rz**)- ou acide carboxylique lorsque **-Z-** = -C-).

Selon un premier mode de réalisation, **-Chrom1', -Chrom2',** et **-Chrom3'** sont identiques. Selon ce mode de réalisation, l'agent chélatant de formule (**III**) peut être obtenu par alkylation d'un cycle de type TACN.3HCl avec un intermédiaire de formule (**B6**) : 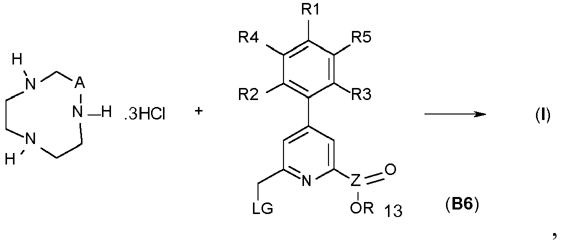 avec **-A-**, **-R1**, **-R2**, **-R3**, **-R4**,**-R5** et **-R13** tels que définis pour les agents chélatants (**III**), et **-LG** représentant un groupe partant.

Par « groupe partant », on entend un groupe apte à se détacher du reste de la molécule dans les conditions réactionnelles, de sorte qu'un autre réactif réagisse avec le reste de la molécule pour former une nouvelle liaison avec l'atome de carbone portant initialement le groupe partant. La nature du groupe partant n'est pas limitée, et tout groupe partant connu de l'homme du métier peut convenir. A titre d'exemples, on peut citer les sulfonates, comme le mésylate ou le tosylate.

L'intermédiaire (**B6**) peut être préparé, selon des techniques bien connues de l'homme du métier, à partir de l'intermédiaire (**B7**) suivant : 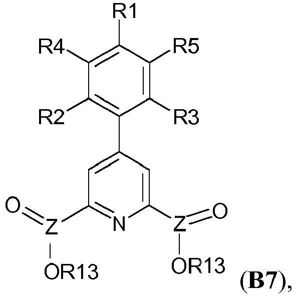 avec **-R1**, **-R2**, **-R3**, **-R4**, **-R5**, -**R13** et **-Z-** tels que définis pour l'intermédiaire (**B6**).

L'intermédiaire (**B7**) peut être synthétisé à partir du réactif (**B9**), par formation d'un acide boronique (**B8**), suivie d'une réaction de Suzuki avec un dialkyl-4-halogéno-2,6-pyridinedicarboxylate : 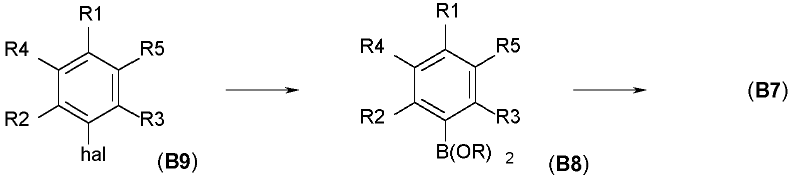 avec **-R1**, **-R2**, **-R3**, **-R4** et **-R5** tels que définis pour l'intermédiaire (**B7**) et **-hal** représentant un halogène, comme -Br, -Cl ou -I.

Selon un second mode de réalisation, **-Chrom1'** et **-Chrom2'** sont identiques, et **-Chrom3'** est différent de **-Chrom1'** et **-Chrom2'.** Selon ce mode de réalisation, l'agent chélatant de formule (**III**) peut être préparé par alkylation de l'intermédiaire (**III'**) avec l'intermédiaire de formule (**B6"**), avec **-R1", -R2"**, **-R3"**, **-R4"**, **-R5"**, **-R13"**, **-Z"** et **-LG"** tels que définis respectivement pour **-R1**, **-R2**, **-R3**, **-R4**, **-R5**, **-R13**, **-Z** et **-LG** pour l'agent chélatant de formule (**III**) :

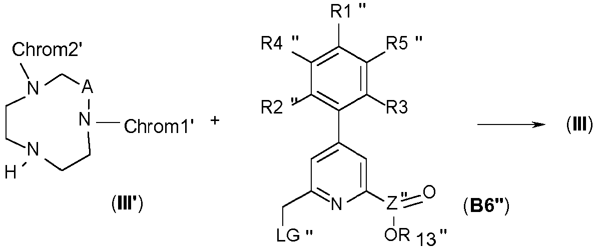

L'intermédiaire (**III'**) peut être préparé par alkylation d'un cycle de type TACN.3HCl avec l'intermédiaire de formule (**B6'**), avec **-R1'**, **-R2'**, **-R3'**, **-R4'**, **-R5'**, **-R13'**, **-Z'** et **-LG'** tels que définis respectivement pour **-R1**, **-R2**, **-R3**, **-R4**, **-R5**, **-R13**, **-Z** et **-LG** pour l'agent chélatant (**III**) :

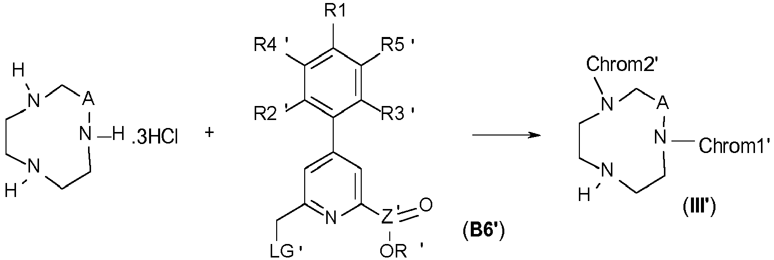

Avantageusement, une des trois fonctions amine du cycle de type TACN.3HCl est protégée, via un groupe protecteur des fonctions amine, préalablement à l'étape d'alkylation avec l'intermédiaire de formule (**B6'**) ; une étape de déprotection subséquente permettant de générer l'intermédiaire (**III'**)

Les intermédiaires (**B6'**) et (**B6"**) peuvent être obtenus de manière analogue à l'intermédiaire (**B6**).

Selon la formule (**II**) est décrite la famille de complexes de lanthanide issus de la coordination d'un ligand chélatant de formule (**IV**) après déprotection et libération des fonctions acide carboxylique. Pour la synthèse de ces composés, on pourra notamment se référer aux demandes WO 2013/026790 et WO 2013/092992 de Takalo.

L'invention concerne enfin un procédé de détection d'une biomolécule comprenant la détection de la luminescence d'un conjugué de ladite biomolécule avec un complexe luminescent tel que défini dans le cadre de l'invention, comprenant un groupe réactif, et obtenu par couplage de ladite biomolécule avec ledit complexe luminescent sur son groupe réactif.
La **figure 1** présente le spectre d'absorption et d'émission du complexe **Ia.4** dans l'eau à température ambiante.
La **figure 2** présente le spectre d'émission du complexe **Ia.6** dans un mélange méthanol/éthanol 4 :1 à température ambiante.
La **figure 3** représente des images de cellules fixées au PFA et marquées par le complexe **Ia.2** (excitation biphotonique λₑₓ = 720 nm), obtenues avec l'émission dans le visible du complexe **Ia.2** (image de gauche) ou obtenue en lumière transmise DIC (image de droite).
La **figure 4** représente des images de cellules fixées au PFA et marquées par le complexe **Ia.5** (excitation biphotonique λₑₓ = 720 nm), obtenues avec l'émission dans le visible du complexe Ia.5 (images a et c) ou obtenue en lumière transmise DIC (images b et d).

Les exemples ci-dessous permettent d'illustrer l'invention, mais n'ont aucun caractère limitatif.

### Exemples

Les abréviations ci-dessous sont utilisées dans les exemples qui vont suivre : DCM : dichlorométhane ; DMF : diméthylformamide ;DMSO : diméthylsulfoxide ; Ms : mésyle ; n.d. : non déterminé ; PBS : phosphate buffered saline ; PEG : polyéthylène glycol ; PFA : paraformaldéhyde ; quant. : quantitatif ; TACN : Triazacyclononane ; THF : Tétrahydrofurane ; Ts : tosyle

### Informations Générales :

Les spectres RMN (¹H, ¹³C) ont été enregistrés sur un appareil Bruker Avance à une fréquence de 300 MHz ou 500,10 MHz, et 75 MHz ou 125,75 MHz, pour ¹H et ¹³C respectivement. Les déplacements chimiques (δ) sont exprimés en partie par million (ppm) par rapport au triméthylsilane utilisé comme référence interne et en utilisant les solvants indiqués. Les constantes de couplage (J) sont exprimées en Hz et les notations suivantes sont utilisées : s (singulet), br (broad), d (doublet), t (triplet), dd (doublet de doublet), m (multiplet). Les spectres de masse haute résolution ont été enregistrés au centre commun de spectrométrie de masse de Lyon (Université Claude Bernard Lyon, Lyon, France) sur un appareil MicroTOFQII équipé avec une source ESI positive. Les chromatographies sur couche mince ont été réalisées sur des plaques de gel de silice sur feuilles d'aluminium (silica gel, Fluka) et révélées à l'aide d'une lampe UV (λ = 254 ou 365 nm) ou bien par coloration. Les purifications ont été réalisées par colonne chromatographique sur gel de silice (silica gel 0,035-0,070 mm, 60A). Les solvants utilisés pour les réactions ont été achetés chez Aldrich ou Acros Organics en tant que solvants secs ou extra sec et stockés sur tamis moléculaire 3Å et les réactifs utilisés ont été achetés chez Aldrich, Acros Organics ou Alfa Aesar. Les spectres d'absorption UV/Vis ont été enregistrés sur un spectromètre JASCO V670 ; les spectres d'émission sur un spectrofluorimètre JOBIN-YVON fluorolog 3. Les temps de rétention ont été réalisés sur un appareil Agilent Technologies 1260 équipé d'une colonne aWaters XBridge RP-C18 (3.5 µm, 4.6 × 100 mm). Le système chromatographique utilisé est formiate d'ammonium 0.25M-MeCN (v/v) comme éluants : isocratique 15% MeCN (2 min), gradient linéaire 15 à 100% MeCN (16 min), isocratique 100% MeCN (4 min), à un débit de 1mL.min⁻¹, détection UV à 210 et 252 nm. Les tests d'imagerie cellulaire ont été réalisés à partir d'une solution concentrée en complexe dans le DMSO afin d'obtenir une concentration finale en complexe de l'ordre de 1.10⁻⁵ M dans le milieu cellulaire (PBS) avec < 1% DMSO. Les cellules T24 sont préalablement fixées au PFA. Les spectres d'absorption biphotonique ont été réalisés avec un microscope confocal à balayage laser LSM710 NLO (Cari Zeiss).

### A. PREPARATION DES COMPLEXES

### Exemple 1 : Complexes d'Europium Ia-Ie

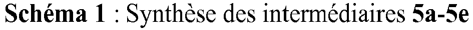

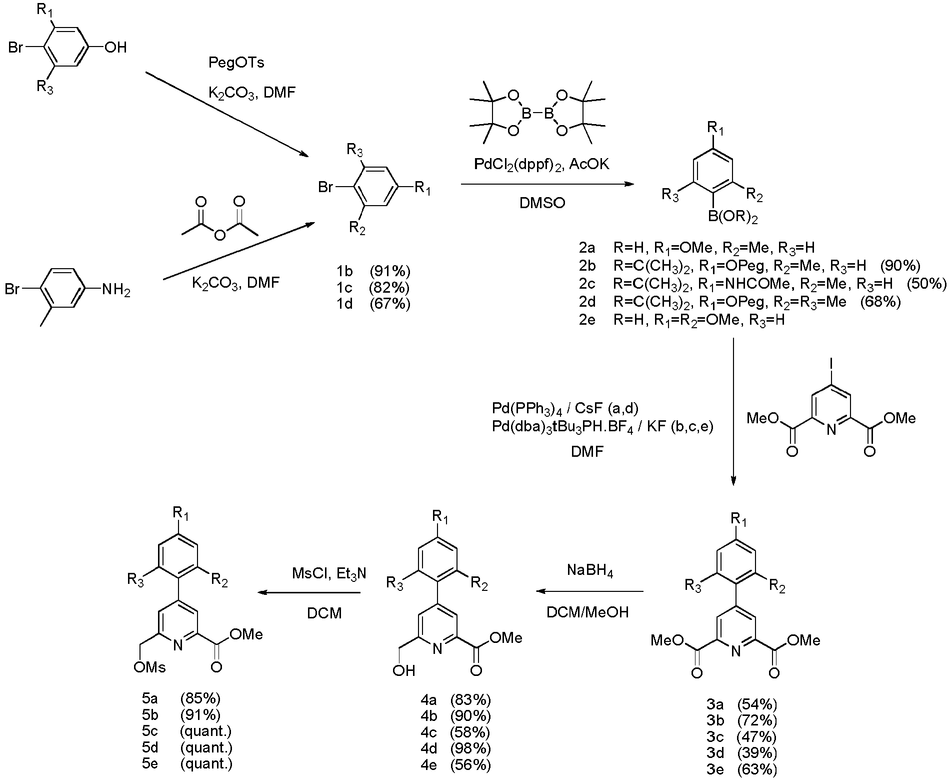

Les composés **la** (4-bromo-3-méthylanisole), **1e** (1-bromo-3,5-diméthoxybenzène) et **2e** (acide 2,4-dimethoxyphenylboronique) sont disponibles commercialement. Les composés **1b** et **1d** sont préparés à partir du phénol correspondant, tandis que le composé **1c** est préparé à partir de l'aniline correspondante. Les acides boroniques **2a-2d** sont ensuite obtenus selon une procédure bien connue de l'homme du métier. Les composés **3a-e** sont obtenus par une réaction de couplage de Suzuki avec du diméthyl-4-iododipicolinate. Puis, une réaction de réduction puis de mésylation permet d'isoler les composés **5a-e.**

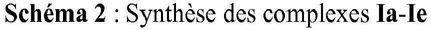

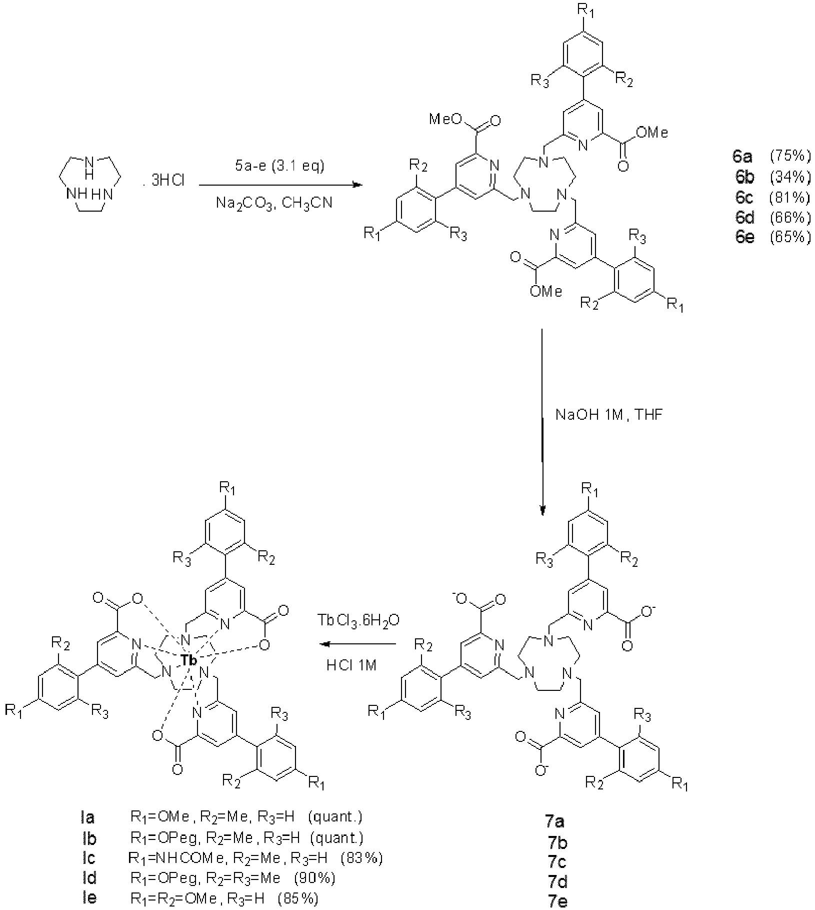

Une réaction d'alkylation du TACN.3HCl par les dérivés mésylates **5a-e**, dans des conditions classiques d'alkylation, suivie d'une étape de déprotection des fonctions acide carboxylique, permettent d'obtenir les complexes de lanthanide **Ia-Ie**.

### Composé 1b

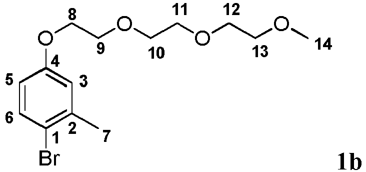

Le 4-bromo-3-methylphenol (1.0 g, 5.3 mmol) et K₂CO₃ (3,7 g, 27 mmol) sont séchés sous vide puis solubilisés dans la DMF sèche (20 mL). Après 30 minutes, le triethylene glycol methyl ether tosylate (2.55 g, 8 mmol) est ajouté dans le schlenck. Le mélange réactionnel est laissé sous agitation à 80°C pendant 5 jours. La solution est ensuite filtrée sur fritté P3, rincée avec CH₂Cl₂ et concentrée sous vide. Le milieu est dilué dans AcOEt/Et₂O et lavée avec H₂O et NaClₛₐₜ. La phase organique est séchée sur Na₂SO₄, filtrée et évaporée. Le produit brut est ensuite purifié sur colonne chromatographique (SiO₂, CH₂Cl₂/AcOEt, 90/10 à 80/20) pour donner le produit pur sous forme d'huile incolore (1.62 g, 91%). ***R_{f}*** (SiO₂, CH₂Cl₂/AcOEt, 80/20) = 0.53 ; **¹H NMR (300 MHz, CDCl₃, δ):** 7.38 (d, 1H, J = 8.7 Hz, H⁶), 6.81 (d, 1H, J = 2.9 Hz, H³), 6.62 (dd, 1H, J₃ = 8.7 Hz, J₄= 2.9 Hz, H⁵), 4.08 (t, 2H, J= 4.6 Hz, H⁸), 3.83 (t, 2H, J= 4.6 Hz, H⁹), 3.75-3.53 (m, 8H, H¹⁰/H¹¹/H¹²/H¹³), 3.38 (s, 3H, H¹⁴), 2.35 (s, 3H, H⁷) ; **¹³C NMR (100 MHz, CDCl₃, δ):** 158.2 (C⁴), 138.9 (C²), 132.9 (C⁶), 117.4 (C³), 115.8 (C¹), 113.8 (C⁵), 72.1 (C¹³), 71.0 (C¹⁰), 70.8 (C¹¹), 70.8 (C¹²), 69.8 (C⁹), 67.8 (C⁸), 59.2 (C¹⁴), 23.3 (C⁷) ; **HRMS (ESI)** calculée pour C₁₄H₂₁BrNaO₄ 355.0515. Exp 355.0520 [M+Na]⁺.

### Composé 1c

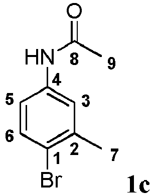

Dans un ballon, l'anhydride acétique (305 µL, 3.22 mmol) est ajouté à une solution de 4-bromo-3-methylaniline (500 mg, 2.69 mmol) dans CH₂Cl₂ sec sous Argon. Le mélange est agité à température ambiante sous Argon jusqu'à la réaction complète (suivi CCM, éther de pétrole/acétate d'éthyle, 80/20). Après 4h, le mélange est lavé avec H₂O (3x). La phase organique est séchée sur Na₂SO₄, filtrée sur coton et évaporée pour obtenir un solide blanc (506 mg, 82%). ***R_{f}*** (SiO₂, CH₂Cl₂/AcOEt, 80/20) = 0.46 ; **¹H NMR (300 MHz, CDCl₃, δ):** 7.45-7.42 (m, 2H, H⁶/H³), 7.19 (dd, 1H, J₃= 8.6 Hz, J₄= 2.4 Hz, H⁵), 2.36 (s, 3H, H⁷), 2.16 (s, 3H, H⁹) ; **¹³C NMR (75 MHz, CDCl₃, δ):** 169.0 (C⁸), 138.5 (C⁴), 137.2 (C¹), 132.6 (C³), 122.4 (C⁶), 119.6 (C²), 119.2 (C⁵), 24.5 (C⁹), 23.1 (C⁷) ; **HRMS (ESI)** calculée pour C₉H₁₁BrNO 228.0024. Exp 228.0019 [M+H]⁺.

### Composé 1d

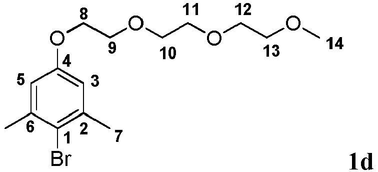

Le 4-bromo-3,5-dimethylphenol (1.30 g, 6.46 mmol) et K₂CO₃ (4.46 g, 9.68 mmol) sont séchés sous vide puis solubilisés dans la DMF sèche (12 mL). Un bullage d'Argon est effectué pendant 10 minutes et le triethylene glycol methyl ether tosylate (3.08 g, 9.68 mmol) est ajouté dans le schlenck. Le mélange réactionnel est laissé sous agitation à 80°C pendant 3 jours. Une extraction CH₂Cl₂/H₂O est ensuite effectuée, puis la phase organique est séchée avec NaClₛₐₜ et Na₂SO₄, filtrée et évaporée. Le produit brut est ensuite purifié sur colonne chromatographique (SiO₂, CH₂Cl₂/AcOEt, 100/0 à 80/20) pour donner une huile du produit pur (1.5 g, 67%). ***R_{f}*** (SiO₂, CH₂Cl₂/AcOEt, 95/5) = 0.38 ; **¹H NMR (300 MHz, CDCl₃, δ):** 6.66 (s, 2H, H³), 4.07 (t, 2H, J= 4.7 Hz, H⁸), 3.82 (t, 2H, J= 4.7 Hz, H⁹), 3.74-3.52 (m, 8H, H¹⁰/H¹¹/H¹²/H¹³), 3.37 (s, 3H, H¹⁴), 2.36 (s, 6H, H⁷) ; **¹³C NMR (75 MHz, CDCl₃, δ):** 157.4 (C⁴), 139.2 (C¹), 118.5 (C^{2/6}), 114.7 (C^{3/5}), 72.1 (C¹³), 71.0 (C¹⁰), 70.8 (C¹¹), 70.7 (C¹²), 69.8 (C⁹), 67.7 (C⁸), 59.2 (C¹⁴), 24.1 (C⁷) ; **HRMS (ESI)** calculée pour C₁₅H₂₃BrNaO₄ 369.0672. Exp 369.0664 [M+Na]⁺.

### Composé 2a

Le composé 2a a été préparé selon le protocole décrit dans *"*Substituent Effects on Oxidation-Induced Formation of Quinone Methidesfrom Arylboronic Ester Precursors ", Sheng Cao, Robin Christiansen, and Xiaohua Peng, Chem. Eur. J., 2013, 19, 9050 - 9058.

### Composé 2b

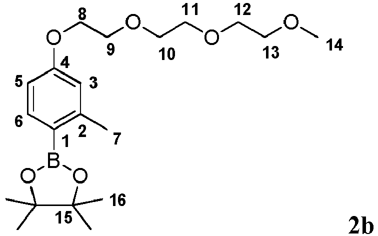

L'acétate de potassium sec (442 mg, 8.46 mmol) est solubilisé dans le DMSO sec (15 mL) sous Argon puis le bis(dipicolinato)diboron (572 mg, 2.25 mmol) et **1b** (500 mg, 1.50 mmol) sont ajoutés. La solution est placée sous bullage d'Argon pendant 20 minutes puis PdCl₂(dppf)₂ (77 mg, 0.11 mmol) est ajouté. Le mélange réactionnel est mis à chauffer à 90°C sous Argon et sous agitation pendant 16h. Une extraction en série H₂O/Et₂O est ensuite effectuée. Les phases organiques sont rassemblées, lavées avec NaClₛₐₜ, séchées sur Na₂SO₄, filtrées et évaporées sous vide. Le produit brut est ensuite purifié sur colonne (SiO₂, CH₂Cl₂/AcOEt, 90/10 à 80/20) afin d'obtenir le produit pur sous forme d'huile jaune (516 mg, 90%). ***R_{f}*** (SiO₂, CH₂Cl₂/AcOEt, 80/20) = 0.57 ; **¹H NMR (500 MHz, CDCl₃, δ):** 7.69 (d, 1H, J = 8.1 Hz, H⁶), 6.72-6.69 (m, 2H, H³/H⁵), 4.13 (t, 2H, J= 4.7 Hz, H⁸), 3.85 (t, 2H, J= 4.7 Hz, H⁹), 3.75-3.53 (m, 8H, H¹⁰/H¹¹/H¹²/H¹³), 3.38 (s, 3H, H¹⁴), 2.50 (s, 3H, H⁷), 1.32 (s, 12H, H¹⁶) ; **¹³C NMR (125 MHz, CDCl₃, δ):** 161.1 (C⁴), 147.3 (C²), 138.0 (C⁶), 120.6 (C¹), 116.4 (C³), 110.9 (C⁵), 83.3 (C¹⁵), 72.1 (C¹³), 71.0 (C¹⁰), 70.8 (C¹¹), 70.8 (C¹²), 69.9 (C⁹), 67.2 (C⁸), 59.2 (C¹⁴), 25.1 (C¹⁶), 22.6 (C⁷) ; **HRMS (ESI)** calculée pour C₂₀H₃₃BNaO₆ 403.2262. Exp 403.2255 [M+Na]⁺.

### Composé 2c

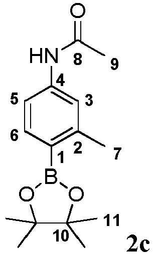

L'acétate de potassium (515 mg, 5.25 mmol) est mis à séché sous vide/Argon pendant 1h puis le bis(pinacolato)diboron (668 mg, 2.63 mmol), le DMSO sec (6 mL) et **1c** (400 mg, 1.75 mmol) sont ajoutés. Un bullage d'Argon dans le mélange est effectué pendant 20 min puis PdCl₂(dppf)₂.CH₂Cl₂ (100 mg, 0.12 mmol) est ajouté et le schlenck est fermé. La solution est mise à chauffer à 90°C sous Argon et sous agitation pendant 19h. Après retour à température ambiante, le mélange est filtré sur fritté P3 et rincé avec Et₂O. On procède ensuite à une extraction en série avec H₂O/Et₂O. Les phases organiques sont rassemblées, séchées sur Na₂SO₄, filtrées sur coton et évaporées. L'évaporation lente du CH₂Cl₂ donne lieu à la formation de cristaux qui sont lavés plusieurs fois au CH₂Cl₂ et séchés sous vide. La RMN ¹H montre l'obtention du produit attendu pur (240 mg, 50%). ***R_{f}*** (SiO₂, CH₂Cl₂/AcOEt, 80/20) = 0.61 ; **¹H NMR (300 MHz, CDCl₃, δ):** 7.72 (d, 1H J= 8 Hz, H⁵), 7.33 (s, 1H, H³), 7.30 (d, 1H, J= 8 Hz, H⁶), 7.18 (s, 1H, NH), 2.51 (s, 3H, H⁷), 2.16 (s, 3H, H⁹), 1.33 (s, 12H, H¹¹) ; **¹³C NMR (75 MHz, CDCl₃, δ):** 168.7 (C⁸), 146.4 (C²), 140.3 (C⁴), 137.1 (C⁶), 120.6 (C³), 115.9 (C⁵), 83.4 (C¹⁰), 25.1 (C⁹), 25.0 (C¹¹), 22.4 (C⁷) ; **HRMS (ESI)** calculée pour C₁₅H₂₃BNO₃ 276.1768. Exp 276.1762 [M+H]⁺.

### Composé 2d

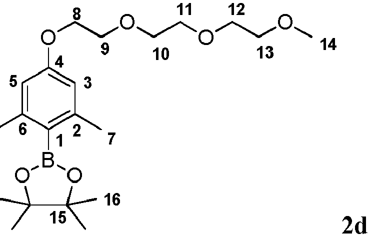

L'acétate de potassium sec (830 mg, 8.46 mmol) est solubilisé dans le DMSO sec (10 mL) sous Argon puis le bis(dipicolinato)diboron (1.07 g, 4.23 mmol) et **1d** (979 mg, 2.82 mmol) sont ajoutés. La solution est placée sous bullage d'Argon pendant 20 minutes puis PdCl₂(dppf)₂.CH₂Cl₂ (161 mg, 0.20 mmol) est ajouté. Le mélange réactionnel est mis à chauffer à 90°C sous Argon et sous agitation pendant 40h. Une extraction en série H₂O/Et₂O est ensuite effectuée. Les phases organiques sont rassemblées, lavées avec NaClₛₐₜ, séchées sur Na₂SO₄, filtrées et évaporées sous vide. Le produit brut est ensuite purifié sur colonne chromatographique (SiO₂, CH₂Cl₂/AcOEt, 95/5 à 80/20) afin d'obtenir le produit pur sous forme d'huile (748 mg, 68%). ***R_{f}*** (SiO₂, CH₂Cl₂/AcOEt, 80/20) = 0.43 ; **¹H NMR (300 MHz, CDCl₃, δ):** 6.51 (s, 2H, H³), 4.09 (t, 2H, J= 4.7 Hz, H⁸), 3.82 (t, 2H, J= 4.7 Hz, H⁹), 3.74-3.53 (m, 8H, H¹⁰/H¹¹/¹²/H¹³), 3.37 (s, 3H, H¹⁴), 2.37 (s, 6H, H⁷), 1.36 (s, 12H, H¹⁶) ; **¹³C NMR (75 MHz, CDCl₃, δ):** 159.7 (C⁴), 144.5 (C^{2/6}), 113.2 (C^{3/5}), 83.5 (C¹⁵), 72.1 (C¹³), 70.9 (C¹⁰), 70.8 (C¹¹), 70.7 (C¹²), 69.9 (C⁹), 67.1 (C⁸), 59.1 (C¹⁴), 25.0 (C¹⁶), 22.7 (C⁷) ; **HRMS (ESI)** calculée pour C₂₁H₃₅BNaO₆ 417.2423. Exp 417.2417 [M+Na]⁺.

### Composé 3a

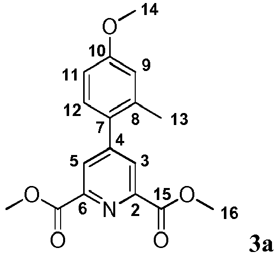

Dans un schlenck, le diméthyl-4-iododipicolinate (300 mg, 0.934 mmol) et l'acide 2-méthyl,4-méthoxyphenylboronic (186 mg, 1.121 mmol) sont solubilisés dans la DMF sèche (15 mL) sous Argon. Après 30 minutes de bullage d'Argon dans le milieu, le fluorure de césium (355 mg, 2.335 mmol) et Pd(PPh₃)₄ (cat.) sont ajoutés. Le milieu réactionnel est laissé sous agitation à 90°C sous Argon pendant 17h. Le mélange est ensuite dilué dans CH₂Cl₂ puis extrait avec H₂O. La phase chlorée est évaporée et la phase aqueuse est extraite de nouveau avec AcOEt. La phase organique est lavée avec NaClₛₐₜ., séchée sur Na₂SO₄, filtrée et évaporée sous vide. Le produit brut obtenu est purifié sur 2 colonnes chromatographiques (SiO₂, CH₂Cl₂/acétone, 90/10 puis SiO₂, CH₂Cl₂/AcOEt 98/2 à 95/5) afin d'obtenir le produit pur (159 mg, 54%). ***R_{f}***(SiO₂, CH₂Cl₂/acétone, 80/20) = 0.82 **; ¹H NMR (300 MHz, CDCl₃, δ):** 8.28 (s, 2H, H^{3/5}), 7.24-7.20 (m, 1H, H¹²), 6.87-6.85 (m, 2H, H¹¹/H⁹), 4.04 (s, 6H, H¹⁶), 3.86 (s, 3H, H¹⁴), 2.32 (s, 3H, H¹³) ; **¹³C NMR (125 MHz, CDCl₃, δ):** 165.5 (C¹⁵), 160.5 (C¹⁰), 152.6 (C⁴), 148.4 (C^{2/6}), 137.0 (C⁸), 131.0 (C¹²), 128.8 (C⁷), 121.7 (C^{3/5}), 116.6 (C⁹), 112.1 (C¹¹), 55.5 (C¹⁴), 53.4 (C¹⁶), 20.8 (C¹³) ; **HRMS (ESI)** calculée C₁₇H₁₇NNaO₅ 338.0999. Exp 338.0990 [M+Na]⁺.

### Composé 3b

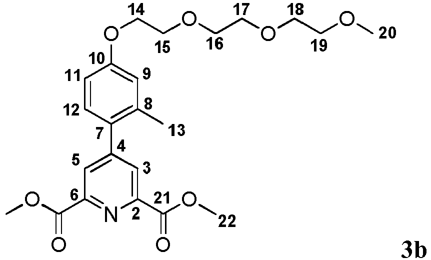

Le dimethyl 4-iodo-2,6-pyridinedicarboxylate sec (344 mg, 1.07 mmol) est solubilisé dans la DMF sèche (12 mL) et **2b** (448 mg, 1.18 mmol) est ajouté sous Argon. La solution est placée sous bullage d'Argon pendant 30 minutes puis le fluorure de potassium (205 mg, 3.53 mmol) et Pd(dba)₃tBu₃PH.BF₄ (43 mg, 0.08 mmol) sont ajoutés. Le mélange réactionnel est placé à 80°C sous agitation et sous Argon pendant 40h. Une extraction en série AcOEt/H₂O est ensuite effectuée. Les phases organiques sont lavées avec NaClₛₐₜ, séchées sur Na₂SO₄, filtrées et évaporées sous vide. Le produit brut est ensuite purifié sur colonne chromatographique (SiO₂, CH₂Cl₂/acétone, 95/5 à 90/10) pour obtenir le produit pur sous forme de solide blanc (343 mg, 72%). ***R_{f}*** (SiO₂, CH₂Cl₂/acétone, 90/10) = 0.45 ; **¹H NMR (500 MHz, CDCl₃, δ):** 8.26 (s, 2H, H^{3/5}), 7.19 (d, 1H, J = 8.3 Hz, H¹²), 6.87 (d, 1H, J = 2.3 Hz, H⁹), 6.86 (dd, 1H, J₃ = 8.3 Hz, J₄= 2.3 Hz, H¹¹), 4.17 (t, 2H, J = 4.7 Hz, H¹⁴), 4.03 (s, 6H, H²²), 3.86 (t, 2H, J = 4.7 Hz, H¹⁵), 3.76-3.54 (m, 8H, H¹⁶/H¹⁷/H¹⁸/H¹⁹), 3.38 (s, 3H, H²⁰), 2.30 (s, 3H, H¹³) ; **¹³C NMR (125 MHz, CDCl₃, δ):** 165.5 (C²¹), 159.7 (C¹⁰), 152.6 (C⁴), 148.3 (C^{2/6}), 136.9 (C⁸), 130.9 (C¹²), 130.1 (C⁷), 128.7 (C^{3/5}), 117.3 (C⁹), 112.7 (C¹¹), 72.1 (C¹⁹), 71.0 (C¹⁶), 70.9 (C¹⁷), 70.8 (C¹⁸), 69.8 (C¹⁵), 67.7 (C¹⁴), 59.2 (C²⁰), 53.4 (C²²), 20.8 (C¹³) ; **HRMS (ESI)** calculée pour C₂₃H₃₀NO₈ 448.1966. Exp 448.1959 [M+H]⁺.

### Composé 3c

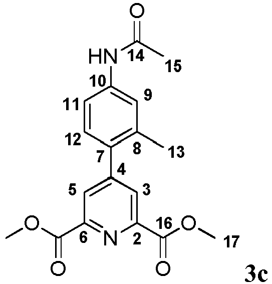

**2c** (200 mg, 0.73 mmol) et le dimethyl 4-iodo-2,6-pyridinedicarboxylate (212 mg, 0.66 mmol) sont préalablement séchés sous vide/Argon puis la DMF sèche (15 mL) est ajoutée. La solution est dégazée pendant 20 min sous Argon et le fluorure de potassium (126 mg, 2.18 mmol) et Pd(dba)₃tBu₃PH.BF₄ (27 mg, 0.05 mmol) sont ajoutés. Le mélange est placé à 80°C sous Argon pendant 3 jours. Après retour à température ambiante, du CH₂Cl₂ est ajouté et la phase organique est lavée avec H₂O (3x) et NaCl saturé (1x). La phase organique est séchée sur Na₂SO₄, filtrée et évaporée sous vide. Après évaporation lente d'un mélange DCM/MeOH, des cristaux sont obtenus et rincés avec un minimum de CH₂Cl₂ afin d'obtenir le produit pur (107 mg, 47%). ***R_{f}*** (SiO₂, CH₂Cl₂/MeOH, 95/5) = 0.55 ; **¹H NMR (300 MHz, CDCl₃, δ):** 8.25 (s, 2H, H^{3/5}), 7.96 (s, 1H, NH), 7.55 (s, 1H, H⁹), 7.48 (d, 1H, J= 8.3 Hz, H¹¹), 7.19 (d, 1H, J= 8.3 Hz, H¹²), 4.00 (s, 6H, H¹⁷), 2.26 (s, 3H, H¹³), 2.19 (s, 3H, H¹⁵) ; **¹³C NMR (75 MHz, CDCl₃, δ):** 168.9 (C¹⁴), 165.3 (C¹⁶), 152.3 (C⁴), 148.3 (C^{2/6}), 139.2 (C¹⁰), 136.2 (C⁸), 133.0 (C⁷), 130.2 (C¹²), 128.6 (C^{3/5}), 121.9 (C⁹), 117.8 (C¹¹), 53.3 (C¹⁷), 24.7 (C¹⁵), 20.6 (C¹³) ; **HRMS (ESI)** calculée pour C₁₈H₁₉N₂O₅ 343.1288. Exp 343.1289 [M+H]⁺.

### Composé 3d

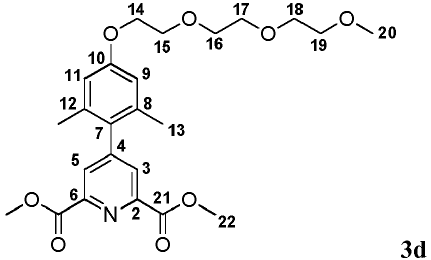

Le dimethyl 4-iodo-2,6-pyridinedicarboxylate sec (144 mg, 0.45 mmol) est solubilisé dans la DMF sèche (5 mL) et **2d** (195 mg, 0.50 mmol) est ajouté sous Argon. La solution est placée sous bullage d'Argon pendant 20 minutes puis le fluorure de césium (171 mg, 0.11 mmol) et Pd(PPh₃)₄ (52 mg, 0.05 mmol) sont ajoutés. Le mélange réactionnel est placé à 80°C sous agitation et sous Argon pendant 5 jours. Une extraction en série AcOEt/H₂O est ensuite effectuée. Les phases organiques sont lavées avec NaClₛₐₜ, séchées sur Na₂SO₄, filtrées et évaporées sous vide. Le produit brut est ensuite purifié sur colonne chromatographique (SiO₂, CH₂Cl₂/acétone, 100% à 80/20) pour obtenir le produit pur sous forme d'huile (85 mg, 39%). ***R_{f}*** (SiO₂, CH₂Cl₂/acétone, 95/5) = 0.41 ; **¹H NMR (300 MHz, CDCl₃, δ):** 8.08 (s, 2H, H^{3/5}), 6.66 (s, 2H, H⁸), 4.11 (t, 2H, J= 4.6 Hz, H¹⁴), 3.99 (s, 6H, H²²), 3.83 (t, 2H, J= 4.6 Hz, H¹⁵), 3.72-3.50 (m, 8H, H¹⁶/H¹⁷/H¹⁸/H¹⁹), 3.34 (s, 3H, H²⁰), 1.94 (s, 6H, H¹³) ; **¹³C NMR (75 MHz, CDCl₃, δ):** 165.2 (C²¹), 158.7 (C¹⁰), 152.5 (C⁴), 148.7 (C^{2/6}), 136.5 (C^{8/12}), 130.0 (C⁷), 129.6 (C^{3/5}), 114.0 (C^{9/11}), 72.0 (C¹⁹), 70.9 (C¹⁶), 70.7 (C¹⁷), 70.6 (C¹⁸), 69.7 (C¹⁵), 67.4 (C¹⁴), 59.0 (C²⁰), 53.2 (C²²), 21.0 (C¹³).

### Composé 3e

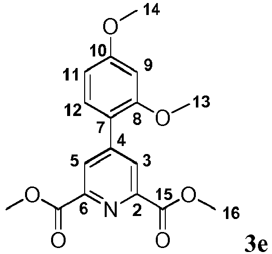

Dans un schlenck, le diméthyl-4-iododipicolinate (200 mg, 0.623 mmol) et l'acide 2,4-diméthoxyphenylboronic (125 mg, 0.685 mmol) sont solubilisés dans la DMF sèche (7 mL) sous Argon. Après 30 minutes de bullage d'Argon dans le milieu, le fluorure de potassium (119 mg, 2.056 mmol) et Pd(dba)₃tBu₃PH.BF₄ (36 mg, 0.062 mmol) sont ajoutés. Le milieu réactionnel est laissé sous agitation à 80°C sous Argon pendant 19h. Le mélange est ensuite dilué dans un mélange AcOEt/Et₂O puis extrait avec H₂O puis NaClₛₐₜ. La phase organique est ensuite séchée sur Na₂SO₄, filtrée et évaporée sous vide. Le solide brun obtenu est finalement purifié sur colonne chromatographique (SiO₂, AcOEt/EP, 20/80 à 60/40, dépôt solide) afin d'obtenir le produit pur sous forme d'une solide beige (131 mg, 63%). ***R_{f}***(SiO₂, EP/AcOEt, 60/40) = 0.73 ; **¹H NMR (300 MHz, CDCl₃, δ):** 8.50 (s, 2H, H^{3/5}), 7.40 (d, 1H, J = 8.5 Hz, H¹²), 6.63 (dd, 1H, J₃ = 8.4 Hz, J₄= 2.4 Hz, H¹¹), 6.58 (d, 1H, J = 2.4 Hz, H⁹), 4.04 (s, 6H, H¹⁶), 3.88 (s, 3H, H¹³), 3.87 (s, 3H, H¹⁴) ; **¹³C NMR (100 MHz, CDCl₃, δ):** 165.8 (C¹⁵), 162.5 (C¹⁰), 158.2 (C⁸), 149.2(C⁴), 148.1(C^{2/6}), 131.6(C¹²), 128.4(C^{3/5}), 118.6(C⁷), 105.5(C⁹), 99.2(C¹¹), 55.8(C¹³), 55.7(C¹⁴), 53.4 (C¹⁶) ; **HRMS (ESI)** calculée pour C₁₇H₁₇NNaO₆ 354.0948. Exp 354.0937 [M+Na]⁺.

### Composé 4a

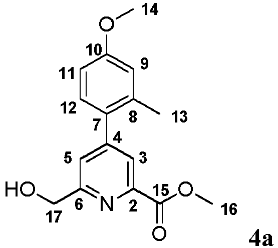

**3a** (180 mg, 0.571 mmol) est solubilisé dans un mélange CH₂Cl₂/MeOH (2/3 mL) puis refroidi à 0°C. NaBH₄ (42 mg, 1.1 mmol) est ensuite ajouté et après 30 minutes, le milieu réactionnel est placé à température ambiante pendant 4h. La réaction est ensuite quenchée avec une solution HCl 1M. La phase organique est lavée avec H₂O (2x) puis NaClₛₐₜ, séchée sur Na₂SO₄, filtrée et évaporée. Le produit brut est ensuite purifié sur colonne chromatographique (SiO₂, CH₂Cl₂/acétone 80/20 à 65/35) afin d'obtenir le produit pur sous forme de cristaux jaune pâle (136 mg, 83%). **Rf** (SiO₂, CH₂Cl₂/acétone, 80/20) = 0.25 ; **¹H NMR (500 MHz, CDCl₃, δ):** 8.01 (s, 1H, H³), 7.46 (s, 1H, H⁵), 7.17 (d, 1H, J = 8.8 Hz, H¹²), 6.85-6-81 (m, 2H, H¹¹/H⁹), 4.89 (d, 2H, J = 4.6 Hz, H¹⁷), 4.01 (s, 3H, H¹⁶), 3.85 (s, 3H, H¹⁴), 3.31 (t, 1H, J = 4.6 Hz, OH), 2.29 (s, 3H, H¹³) ; **¹³C NMR (125 MHz, CDCl₃, δ):** 165.9 (C¹⁵), 160.1 (C⁶, C¹⁰), 151.8 (C⁴), 147.1 (C²), 136.9 130.9 (C⁷), 130.8 (C¹²), 125.0 (C³), 124.6 (C⁵), 116.5 (C⁹), 111.9 (C¹¹), 64.9 (C¹⁷), 55.5 (C¹⁴), 53.1 (C¹⁶), 20.8 (C¹³) ; HRMS (ESI) calculée pour C₁₆H₁₈NO₄ 288.1230. Exp 288.1220 [M+H]⁺. Calc pour C₁₆H₁₇NNaO₄ 310.1050. Exp 310.1038 [M+Na]⁺.

### Composé 4b

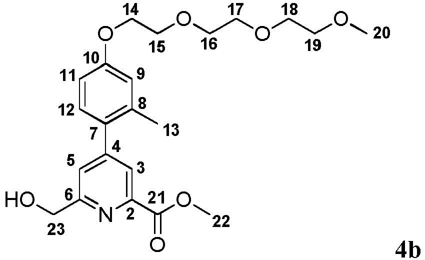

**3b** (320 mg, 0.72 mmol) est solubilisé dans un mélange CH₂Cl₂/MeOH (2.5 mL/4.5 mL). Après avoir placé le milieu à 0°C, NaBH₄ (30 mg, 0.79 mmol) est ajouté et après 30 minutes, la solution est mise à température ambiante sous agitation pendant 3h. La réaction est quenchée par ajout d'une solution HCl 1M. Puis une extraction CH₂Cl₂/H₂O est effectuée. La phase organique est lavée avec NaClₛₐₜ, séchée sur Na₂SO₄, filtrée et évaporée. Une purification sur colonne chromatographique (SiO₂, CH₂Cl₂/MeOH, 98/2 à 97/3) est effectuée afin d'obtenir le produit pur sous forme d'huile incolore (270 mg, 90%). ***R_{f}*** (SiO₂, CH₂Cl₂/MeOH, 97/3) = 0.22 ; **¹H NMR (500 MHz, CDCl₃, δ):** 7.99 (d, 1H, J = 0.9 Hz, H³), 7.47 (s, 1H, H⁵), 7.15 (d, 1H, J = 8.4 Hz, H¹²), 6.85 (d, 1H, J = 2.3 Hz, H⁹), 6.83 (dd, 1H, J₃= 8.4 Hz, J₄= 2.3 Hz, H¹¹), 4.89 (d, 2H, J = 3.9 Hz, H²³), 4.16 (t, 2H, J= 4.7 Hz, H¹⁴), 3.99 (s, 3H, H²²), 3.87 (t, 2H, J= 4.7 Hz, H¹⁵), 3.72-3.54 (m, 8H, H¹⁶/H¹⁷/H¹⁸/H¹⁹), 3.51 (m, 1H, OH), 3.37 (s, 3H, H²⁰), 2.27 (s, 3H, H¹³) ; **¹³C NMR (125 MHz, CDCl₃, δ):** 165.9 (C²¹), 160.3 (C⁶), 159.3 (C¹⁰), 151.8 (C⁴), 147.1 (C²), 136.8 (C⁸), 131.0 (C⁷), 130.8 (C¹²), 125.0 (C³), 124.6 (C⁵), 117.2 (C⁹), 112.5 (C¹¹), 72.1 (C¹⁹), 71.0 (C¹⁶), 70.9 (C¹⁷), 70.8 (C¹⁸), 69.9 (C¹⁵), 67.6 (C¹⁴), 64.9 (C²³), 59.2 (C²⁰), 53.1 (C²²), 20.8 (C¹³) ; **HRMS (ESI)** calculée pour C₂₂H₃₀NO₇ 420.2017. Exp 420.2015 [M+H]⁺.

### Composé 4c

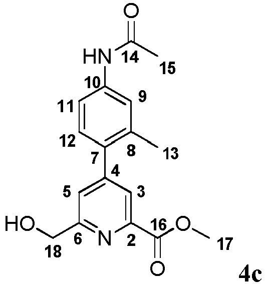

**3c** est solubilisé dans un mélange CH₂Cl₂/MeOH et NaBH₄ est ajouté à 0°C. Après 5 minutes, la solution est laissée à température ambiante et la monoréduction est suivie par CCM (SiO₂, DCM/MeOH, 95/5). Au bout de 2h30, la réaction est quenchée par HCl 1M. La phase organique est ensuite lavée avec H₂O et NaClₛₐₜ, puis séchée sur Na₂SO₄, filtrée et évaporée. Le produit brut est purifié sur colonne chromatographique (SiO₂, DCM/MeOH, 95/5) et une poudre blanche est obtenue (56 mg, 58%). ***R_{f}*** (SiO₂, CH₂Cl₂/MeOH, 95/5) = 0.30 ; **¹H NMR (300 MHz, CDCl₃, δ):** 7.98 (s, 1H, H³), 7.48 (m, 2H, H⁴/H⁹/NH), 7.42 (d, 1H, J= 8.3 Hz, H¹¹), 7.17 (d, 1H, J= 8.3 Hz, H¹²), 4.90 (s, 2H, H¹⁸), 4.00 (s, 6H, H¹⁷), 3.57 (s, 1H, OH), 2.26 (s, 3H, H¹³), 2.20 (s, 3H, H¹⁵); **¹³C NMR (75 MHz, CDCl₃, δ):** 168.7 (C¹⁴), 165.8 (C¹⁶), 160.4 (C⁶), 151.4 (C⁴), 147.1 (C²), 138.6 (C¹⁰), 136.2 (C⁸), 134.2 (C⁷), 130.2 (C¹²), 124.7 (C³), 124.4 (C⁵), 121.9 (C⁹), 117.7 (C¹¹), 64.9 (C¹⁸), 53.1 (C¹⁷), 24.8 (C¹⁵), 20.6 (C¹³) ; **HRMS (ESI)** calculée pour C₁₇H₁₉N₂O₄ 315.1339. Exp 315.1342 [M+H]⁺.

### Composé 4d

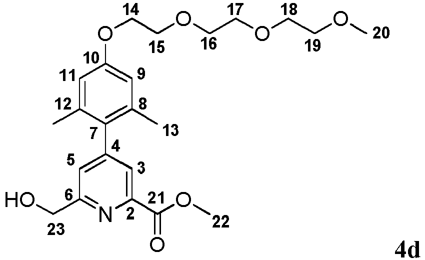

**3d** (136 mg, 0.29 mmol) est solubilisé dans un mélange CH₂Cl₂/MeOH (50/50, 4 mL). Après avoir placé le milieu à 0°C, NaBH₄ (42 mg, 1.11 mmol) est ajouté (coloration marron de la solution) et après 20 minutes, la solution est mise à température ambiante sous agitation pendant 6h. La réaction est quenchée par ajout d'une solution HCl 1M (4 mL). Puis une extraction CH₂Cl₂/H₂O est effectuée. La phase organique est lavée avec NaClₛₐₜ, séchée sur Na₂SO₄, filtrée et évaporée. Une purification sur colonne chromatographique (SiO₂, CH₂Cl₂/AcOEt, 70/30 à 50/50 puis 10% MeOH) est effectuée afin d'obtenir le produit pur (123 mg, 98%). ***R_{f}*** (SiO₂, CH₂Cl₂/AcOEt, 80/20) = 0.32 ; **¹H NMR (300 MHz, CDCl₃, δ):** 8.08 (s, 1H, H³), 7.79 (s, 1H, H⁵), 6.63 (s, 2H, H⁹), 4.87 (s, 2H, H²³), 4.09 (t, 2H, J= 4.6 Hz, H¹⁴), 3.93 (s, 3H, H²²), 3.81 (t, 2H, J= 4.6 Hz, H¹⁵), 3.72-3.49 (m, 8H, H¹⁶/H¹⁷/H¹⁸/H¹⁹), 3.33 (s, 3H, H²⁰), 1.93 (s, 6H, H¹³) ; **¹³C NMR (75 MHz, CDCl₃, δ):** 165.6 (C²¹), 161.0 (C⁶), 158.2 (C¹⁰), 151.4 (C⁴), 147.1 (C²), 136.4 (C^{8/12}), 130.9 (C⁷), 125.2 (C^{3/5}), 113.6 (C^{9/11}), 71.8 (C¹⁹), 70.7 (C¹⁶), 70.5 (C¹⁷), 70.4 (C¹⁸), 69.6 (C¹⁵), 67.2 (C¹⁴), 64.6 (C²³), 58.9 (C²⁰), 52.7 (C²²), 20.8 (C¹³) ; **HRMS (ESI)** calculée pour C₂₃H₃₂NO₇434.2173. Exp 434.2158 [M+H]⁺.

### Composé 4e

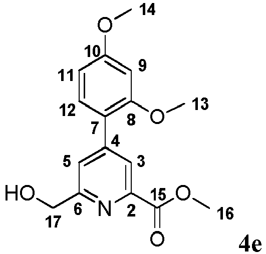

**3e** (120 mg, 0.36 mmol) est solubilisé dans un mélange CH₂Cl₂/MeOH (2/3 mL) puis refroidi à 0°C. NaBH₄ (15 mg, 0.40 mmol) est ensuite ajouté et après 15 minutes, le milieu réactionnel est placé à température ambiante pendant 4h. La réaction est ensuite quenchée avec une solution HCl 1M. La phase organique est lavée avec H₂O (2x) puis NaClₛₐₜ, séchée sur Na₂SO₄, filtrée et évaporée. Le produit brut est ensuite purifié sur colonne chromatographique (SiO₂, CH₂Cl₂/acétone 80/20) afin d'obtenir le produit pur sous forme d'un solide beige (53 mg, 56%). ***R_{f}*** (SiO₂, CH₂Cl₂/acétone, 80/20) = 0.17 ; **¹H NMR (300 MHz, CDCl₃, δ):** 8.21 (s, 1H, H³), 7.67 (s, 1H, H⁵), 7.33 (d, 1H, J = 8.3 Hz, H¹²), 6.60 (dd, 1H, J₃= 8.3 Hz, J₄= 2.4 Hz, H¹¹), 6.57 (d, 1H, J = 2.4 Hz, H⁹), 4.87 (d, 2H, J = 4.5 Hz, H¹⁷), 4.00 (s, 3H, H¹⁶), 3.87 (s, 3H, H¹³), 3.84 (s, 3H, H¹⁴), 3.42 (br, 1H, OH).

### Composé 5a

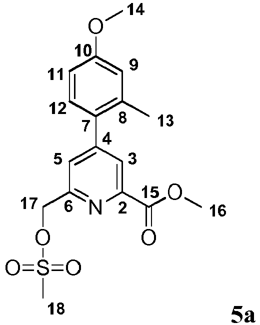

**4a** (126 mg, 0.439 mmol) est solubilisé dans CH₂Cl₂ sec (4 mL) et la triéthylamine (180 µL, 1.317 mmol) est ajoutée. Après avoir placé le milieu à 0°C, le chlorure de mésyle (50 µL, 0.702 mmol) est ajouté et après 5 minutes, le mélange réactionnel est placé à température ambiante pendant 30 minutes. La phase organique est lavée avec H₂O, puis séchée sur Na₂SO₄, filtrée et évaporée pour donner une huile jaune utilisée sans purification supplémentaire pour l'étape suivante (137 mg, 85%). ***R_{f}*** (SiO₂, CH₂Cl₂/MeOH, 95/5) = 0.83 ; **¹H NMR (300 MHz, CDCl₃, δ):** 8.08 (s, 1H, H³), 7.62 (s, 1H, H⁵), 7.18 (m, 1H, H¹²), 6.86-6.82 (m, 2H, H¹¹/H⁹), 5.47 (s, 2H, H¹⁷), 4.02 (s, 3H, H¹⁶), 3.85 (s, 3H, H¹⁴), 3.16 (s, 3H, H¹⁸), 2.30 (s, 3H, H¹³).

### Composé 5b

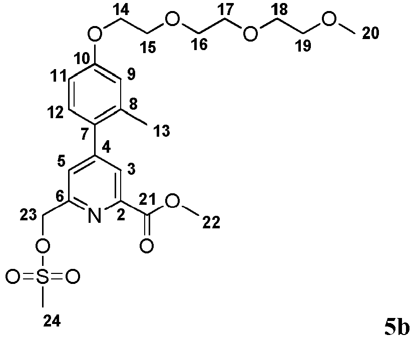

**4b** (282 mg, 0.67 mmol) est solubilisé dans CH₂Cl₂ (8 mL) puis la triéthylamine (280 µL, 2.02 mmol) est ajoutée. Le milieu est placé à 0°C puis le chlorure de mésyle (80 µL, 1.08 mmol) est ajouté. Après 5 minutes, le mélange réactionnel est placé à température ambiante pendant 30 minutes. La phase organique est lavée avec H₂O puis les phases aqueuses sont réextraites par CH₂Cl₂. Les phases organiques sont rassemblées, séchées sur Na₂SO₄, filtrées et évaporées sous vide. Le produit brut est purifié sur colonne chromatographique (SiO₂, CH₂Cl₂/MeOH, 98/2 à 96/4) afin d'obtenir le composé pur sous la forme d'une huile jaune pâle (304 mg, 91%). ***R_{f}*** (SiO₂, CH₂Cl₂/MeOH, 95/5) = 0.49 ; **¹H NMR (500 MHz, CDCl₃, δ):** 8.08 (d, 1H, J = 1.5 Hz, H³), 7.62 (d, 1H, J = 1.5 Hz, H⁵), 7.16 (d, 1H, J = 8.3 Hz, H¹²), 6.86 (d, 1H, J = 2.6 Hz, H⁹), 6.84 (dd, 1H, J₃= 8.3 Hz, J₄= 2.6 Hz, H¹¹), 5.46 (s, 2H, H²³), 4.17 (t, 2H, J= 4.7 Hz, H¹⁴), 4.01 (s, 3H, H²²), 3.88 (t, 2H, J= 4.7 Hz, H¹⁵), 3.76-3.55 (m, 8H, H¹⁶/H¹⁷/H¹⁸/H¹⁹), 3.38 (s, 3H, H²⁰), 3.16 (s, 3H, H²⁴), 2.28 (s, 3H, H¹³) ; **¹³C NMR (125 MHz, CDCl₃, δ):** 165.6 (C²¹), 159.5 (C¹⁰), 154.4 (C⁶), 152.4 (C⁴), 147.8 (C²), 136.9 (C⁸), 130.8 (C¹²), 130.5 (C⁷), 125.90 (C³), 125.88 (C⁵), 117.3 (C⁹), 112.6 (C¹¹), 72.1 (C¹⁹), 71.2 (C²³), 70.9 (C¹⁶), 70.8 (C¹⁷), 70.1 (C¹⁸), 69.9 (C¹⁵), 67.8 (C¹⁴), 59.2 (C²⁰), 53.3 (C²²), 38.3 (C²⁴), 20.8 (C¹³) ; **HRMS (ESI)** calculée pour C₂₃H₃₂NO₉S 498.1792. Exp 498.1779 [M+H]⁺.

### Composé 5c

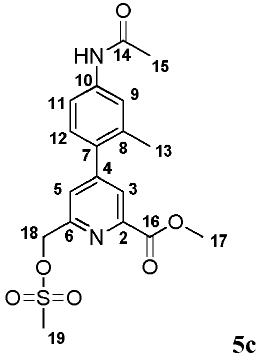

**4c** (56 mg, 0.18 mmol) est solubilisé dans CH₂Cl₂ (4 mL) puis la triéthylamine 75 µL, 0.53 mmol) est ajoutée. Le mélange est placé à 0°C et le chlorure de mésyle (21 µL, 0.27 mmol) est ajouté. Après 5 minutes, la réaction est placée à température ambiante sous agitation pendant 40 minutes. Un lavage de la phase organique avec H₂O est ensuite effectué et les phases aqueuses sont réextraites avec CH₂Cl₂. Les phases organiques sont rassemblées, séchées sur Na₂SO₄, filtrées et évaporées sous vide. La RMN ¹H montre l'obtention du produit pur de manière quantitative (70 mg) et celui-ci est utilisé sans purification supplémentaire pour l'étape suivante. ***R_{f}*** (SiO₂, CH₂Cl₂/MeOH, 95/5) = 0.64 ; **¹H NMR (300 MHz, CDCl₃, δ):** 8.04 (s, 1H, H³), 7.89 (s, 1H, NH), 7.59 (s, 1H, H⁴⁵), 7.51 (s, 1H, H⁹), 7.46 (d, 1H, J= 8.3 Hz, H¹¹), 7.16 (d, 1H, J= 8.3 Hz, H¹²), 5.43 (s, 2H, H¹⁸), 3.99 (s, 6H, H¹⁷), 3.14 (s, 3H, H¹⁹), 2.25 (s, 3H, H¹³), 2.19 (s, 3H, H¹⁵).

### Composé 5d

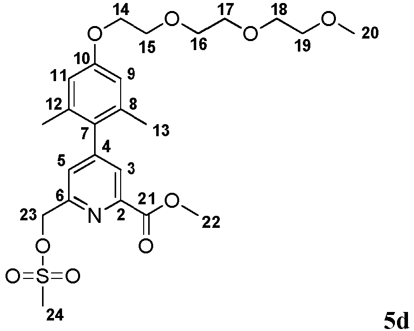

**4d** (123 mg, 0.28 mmol) est solubilisé dans CH₂Cl₂ (5 mL) puis la triéthylamine (192 µL, 1.38 mmol) est ajoutée. Le milieu est placé à 0°C puis le chlorure de mésyle (53 µL, 0.68 mmol) est ajouté. Après 5 minutes, le mélange réactionnel est placé à température ambiante pendant 30 minutes. La phase organique est lavée avec H₂O puis les phases aqueuses sont réextraites par CH₂Cl₂. Les phases organiques sont rassemblées, séchées sur Na₂SO₄, filtrées et évaporées sous vide. Le produit obtenu est utilisé sans purification supplémentaire pour l'étape suivante. ***R_{f}*** (SiO₂, CH₂Cl₂/MeOH, 95/5) = 0.76 ; **¹H NMR (300 MHz, CDCl₃, δ):** 7.90 (s, 1H, H³), 7.45 (s, 1H, H⁵), 6.66 (s, 2H, H⁹), 5.44 (s, 2H, H²³), 4.12 (t, 2H, J= 4.6 Hz, H¹⁴), 3.97 (s, 3H, H²²), 3.83 (t, 2H, J= 4.6 Hz, H¹⁵), 3.74-3.51 (m, 8H, H¹⁶/H¹⁷/H¹⁸/H¹⁹), 3.35 (s, 3H, H²⁰), 3.13 (s, 3H, H²⁴), 1.95 (s, 6H, H¹³) ; **¹³C NMR (75 MHz, CDCl₃, δ):** 165.3 (C²¹), 158.6 (C¹⁰), 154.7 (C⁶), 152.3 (C⁴), 148.0 (C²), 136.5 (C^{8/12}), 130.4 (C⁷), 126.6/126.5 (C³/C⁵), 113.9 (C^{9/11}), 71.9 (C¹⁹), 70.9 (C¹⁶), 70.7 (C¹⁷), 70.6 (C¹⁸), 69.7 (C¹⁵), 67.4 (C¹⁴), 59.0 (C²⁰), 53.1 (C²²), 38.1 (C²³), 31.6 (C²⁴), 21.0 (C¹³).

### Composé 5e

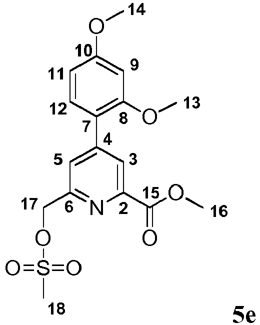

**4e** (60 mg, 0.198 mmol) est solubilisé dans CH₂Cl₂ (3 mL) et la triéthylamine (83 µL, 0.593 mmol) )est ajoutée. Après avoir placé le milieu à 0°C, le chlorure de mésyle (24 µL, 0.317 mmol) est ajouté et après 5 minutes, le mélange réactionnel est placé à température ambiante pendant 30 minutes. La phase organique est lavée avec H₂O, puis séchée sur Na₂SO₄, filtrée et évaporée pour donner une huile jaune utilisée sans purification supplémentaire pour l'étape suivante (76 mg, quant.). ***R_{f}*** (SiO₂, CH₂Cl₂/MeOH, 95/5) = 0.76 ; **¹H NMR (300 MHz, CDCl₃, δ):** 8.29 (s, 1H, H³), 7.86 (s, 1H, H⁵), 7.35 (d, 1H, J = 8.4 Hz, H¹²), 6.61 (dd, 1H, J₃= 8.4 Hz, J₄= 2.3 Hz, H¹¹), 6.57 (d, 1H, J = 2.3 Hz, H⁹), 5.46 (s, 2H, H¹⁷), 4.02 (s, 3H, H¹⁶), 3.87 (s, 3H, H¹³), 3.85 (s, 3H, H¹⁴), 3.14 (s, 3H, H¹⁸).

### Composé 6a

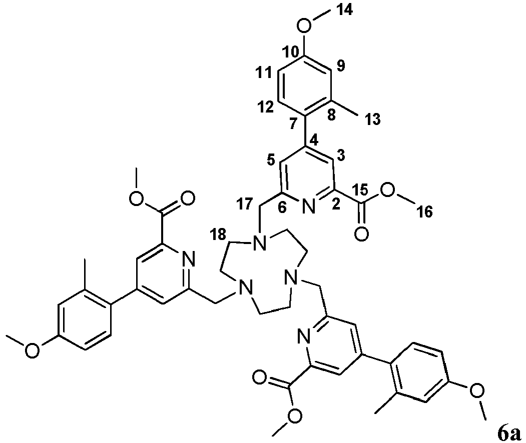

Le triazacyclononane (27 mg, 0.115 mmol) et le carbonate de sodium (122 mg, 1.15 mmol) sont solubilisés dans l'acétonitrile sec (7 mL). Après 10 minutes à température ambiante, **5a** (130 mg, 0.356 mmol) est ajouté et le mélange réactionnel est chauffé à 60°C sous agitation pendant 24h puis à température ambiante pendant 24h. Le mélange est ensuite filtré sur fritté P3, rincé avec CH₃CN sec et les eaux mères sont évaporées. Le produit brut est purifié sur colonne chromatographique (Al₂O₃, CH₂Cl₂/MeOH, 99/1 à 98/2) afin d'obtenir le produit pur sous forme d'un solide jaune pâle (81 mg, 75%). ***R_{f}*** (Al₂O₃, CH₂Cl₂/MeOH, 95/5) = 0.58 ; **¹H NMR (500 MHz, CDCl₃, δ):** 7.94 (s, 3H, H³), 7.68 (s, 3H, H⁵), 7.09 (d, 3H, J = 8.2 Hz, H¹²), 6.79-6.75 (m, 6H, H⁹/H¹¹), 3.98 (s, 9H, H¹⁶), 3.97 (s, 6H, H¹⁷), 3.83 (s, 9H, H¹⁴), 2.92 (s, 12H, H¹⁸), 2.23 (s, 9H, H¹³) ; **¹³C NMR (125 MHz, CDCl₃, δ):** 166.2 (C¹⁵), 161.1 (C⁶), 159.9 (C¹⁰), 151.2 (C⁴), 147.3 (C²), 136.7 (C⁸), 131.1 (C⁷), 130.8 (C¹²), 127.0 (C⁵), 124.7 (C³), 116.3 (C⁹), 111.8 (C¹¹), 64.8 (C¹⁷), 56.0 (C¹⁸), 55.4 (C¹⁴), 53.1 (C¹⁶), 20.9 (C¹³) ; **HRMS (ESI)** calculée pour C₅₄H₆₂N₆O₉ 469.2284. Exp 469.2273 [M+2H]⁺⁺.

### Composé 6b

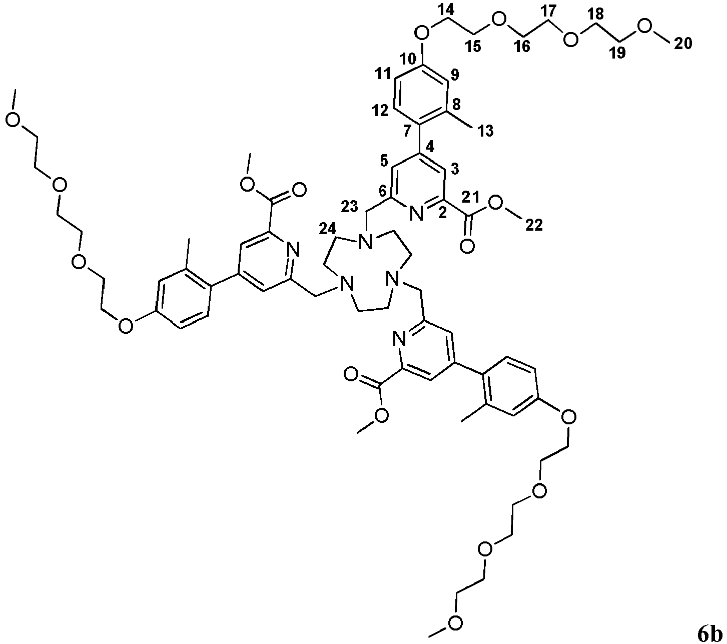

Le triazacyclononane (93 mg, 0.39 mmol) et le carbonate de sodium (414 mg, 3.91 mmol) sont solubilisés dans l'acétonitrile sec (24 mL) sous Argon. Après 10 minutes à température ambiante, **5b** (289 mg, 1.21 mmol) est ajouté et le mélange réactionnel est chauffé à 60°C sous agitation pendant 24h puis à température ambiante pendant 24h. Le mélange est ensuite filtré sur fritté P3, rincé avec CH₃CN sec et les eaux mères sont évaporées. Le produit brut est purifié sur colonne chromatographique (Al₂O₃, CH₂Cl₂/MeOH, 99/1 à 95/5) afin d'obtenir le produit pur sous forme d'une huile jaune (176 mg, 34%). **¹H NMR (500 MHz, CDCl₃, δ):** 7.92 (d, 3H, J = 1.1 Hz, H³), 7.67 (s, 3H, H⁵), 7.07 (d, 1H, J = 8.4 Hz, H¹²), 6.81 (d, 1H, J = 2.4 Hz, H⁹), 6.77 (dd, 1H, J₃= 8.4 Hz, J₄= 2.4 Hz, H¹¹), 4.14 (t, 6H, J= 4.6 Hz, H¹⁴), 3.96 (br s, 15H, H²³/ H²²), 3.86 (t, 6H, J= 4.6 Hz, H¹⁵), 3.74-3.53 (m, 24H, H¹⁶/H¹⁷/H¹⁸/H¹⁹), 3.37 (s, 9H, H²⁰), 2.91 (br s, 12H, H²⁴), 2.21 (s, 9H, H¹³) ; **¹³C NMR (125 MHz, CDCl₃, δ):** 166.2 (C²¹), 161.0 (C⁶), 159.2 (C¹⁰), 151.1 (C⁴), 147.3 (C²), 136.7 (C⁸), 131.3 (C⁷), 130.7 (C¹²), 127.0 (C⁵), 124.7 (C³), 117.0 (C⁹), 113.8 (C¹¹), 72.1 (C¹⁹), 71.0 (C¹⁶), 70.8 (C¹⁷), 70.7 (C¹⁸), 69.8 (C¹⁵), 67.6 (C¹⁴), 64.7 (C²³), 59.2 (C²⁰), 55.9 (C²⁴), 53.0 (C²²), 20.8 (C¹³) ; **HRMS (ESI)** calculée pour C₇₂H₉₈N₆O₁₈ 667.3463 Exp 667.34558 [M+2H]²⁺.

### Composé 6c

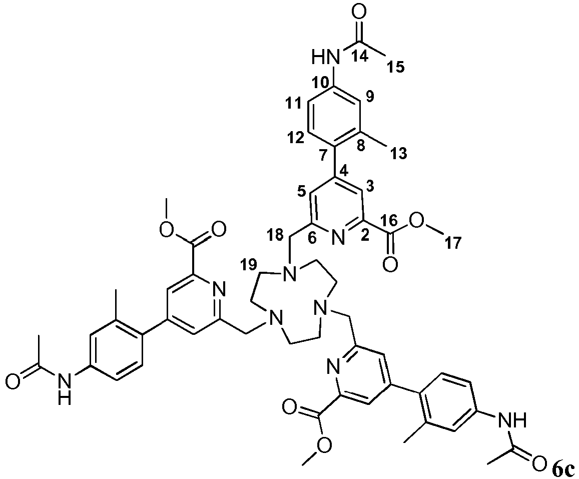

**5c** sec (70 mg, 0.18 mmol) est solubilisé dans l'acétonitrile sec (3 mL) et Na₂CO₃ (67 mg, 0.63 mmol) est ajouté ainsi que le triazacyclononane (13.6 mg, 0.06 mmol). Après bullage d'Argon dans le milieu pendant 5 min, la mélange réactionnel est mis à chauffer à 60°C pendant 5 jours. Le mélange est ensuite filtré sur fritté P3, rincé avec CH₃CN sec et les eaux mères sont évaporées. Le produit brut est purifié sur colonne chromatographique (Al₂O₃, CH₂Cl₂/MeOH, 96/4) afin d'obtenir le produit pur sous forme d'une poudre blanche (47 mg, 81%). **¹H NMR (300 MHz, CDCl₃, δ):** 8.94 (s, 3H, NH), 7.88 (s, 3H, H³), 7.54-7.45 (m, 9H, H⁵/H⁹/H¹¹), 7.07 (d, 1H, J= 8.3 Hz, H¹²), 3.98 (m, 15H, H¹⁸/ H¹⁷), 2.87 (s, 12H, H¹⁹), 2.25 (s, 9H, H¹³), 2.09 (s, 9H, H¹⁵) ; **¹³C NMR (75 MHz, MeOD, δ):** 171.8 (C¹⁴), 166.9 (C¹⁶), 162.4 (C⁶), 152.8 (C⁴), 148.3 (C²), 140.7 (C¹⁰), 136.9 (C⁸), 134.9 (C⁷), 131.0 (C¹²), 128.6 (C³), 124.4 (C⁵), 123.0 (C⁹), 118.9 (C¹¹), 65.0 (C¹⁸), 57.0 (C¹⁹), 53.3 (C¹⁷), 24.05 (C¹⁵), 20.9 (C¹³) ; **HRMS (ESI)** calculée pour C₅₇H₆₄N₉O₉ 1018.4822. Exp 1018.4782 [M+H]⁺ ; t_{R}= 8.33 min (méthode HPLC-MM-ACN, H₂O/CH₃CN 85/15 à 0/100 en 16 min).

### Composé 6d

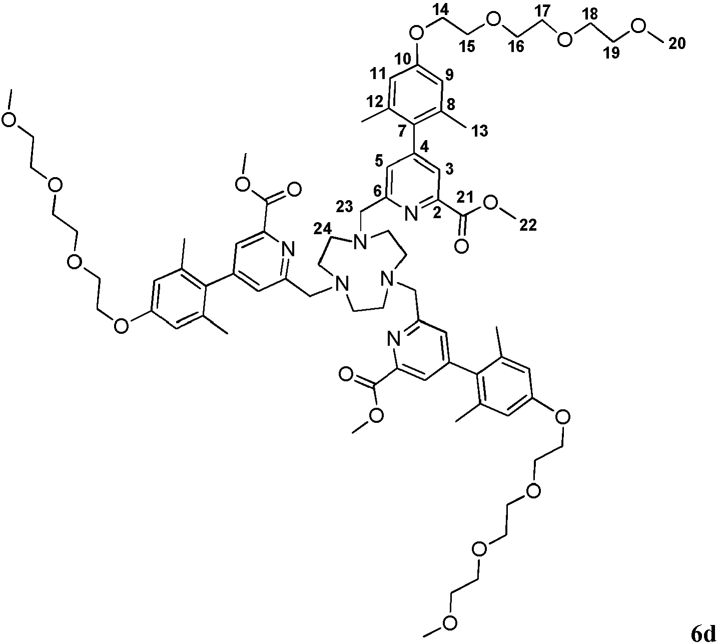

Le triazacyclononane (21.5 mg, 0.09 mmol) et le carbonate de sodium (95 mg, 0.90 mmol) sont solubilisés dans l'acétonitrile sec (5 mL). Après 5 minutes à température ambiante, **5d** (143 mg, 0.28 mmol) est ajouté, un bullage d'Argon est effectué pendant 5 minutes et le mélange réactionnel est chauffé à 60°C sous agitation pendant 4 jours. Le mélange est ensuite filtré sur fritté P3, rincé avec CH₃CN sec et les eaux mères sont évaporées. Le produit brut est purifié sur colonne chromatographique (Al₂O₃, CH₂Cl₂/MeOH, 100% à 97/3) afin d'obtenir le produit pur sous forme d'une poudre blanche (82 mg, 66%). **¹H NMR (300 MHz, CDCl₃, δ):** 7.77 (s, 3H, H³), 7.47 (s, 3H, H⁵), 6.64 (s, 6H, H⁹), 4.12 (t, 6H, J= 4.7 Hz, H¹⁴), 3.94 (s, 9H, H²²), 3.91 (s, 6H, H²³), 3.84 (t, 6H, J= 4.7 Hz, H¹⁵), 3.74-3.52 (m, 24H, H¹⁶/H¹⁷/H¹⁸/H¹⁹), 3.35 (s, 9H, H²⁰), 2.82 (s, 12H, H²⁴), 1.91 (s, 18H, H¹³) ; **¹³C NMR (75 MHz, CDCl₃, δ):** 166.0 (C²¹), 161.4 (C⁶), 158.3 (C¹⁰), 151.0 (C⁴), 147.5 (C²), 136.5 (C^{8/12}), 131.3 (C⁷), 127.8 (C⁵), 125.3 (C³), 113.8 (C^{9/11}), 72.0 (C¹⁹), 70.9 (C¹⁶), 70.7 (C¹⁷), 70.6 (C¹⁸), 69.8 (C¹⁵), 67.4 (C¹⁴), 64.6 (C²³), 59.1 (C²⁰), 55.6 (C²⁴), 52.9 (C²²), 21.0 (C¹³) ; **HRMS (ESI)** calculée pour C₇₅H₁₀₄N₆O₁₈ 688.3698. Exp 688.3686 [M+2H]²⁺ ; t_{R}= 13.09 min (méthode HPLC-MM-ACN, H₂O/CH₃CN 85/15 à 0/100 en 16 min).

### Composé 6e

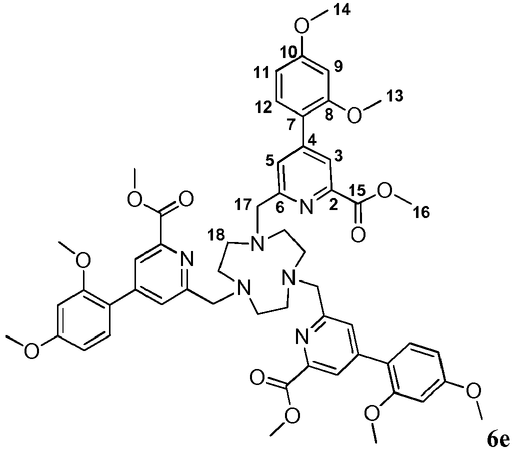

Le triazacyclononane (15 mg, 0.064 mmol) et le carbonate de sodium (68 mg, 0.64 mmol) sont solubilisés dans l'acétonitrile sec (4 mL). Après 10 minutes à température ambiante, **5e** (76 mg, 0.198 mmol) est ajouté et le mélange réactionnel est chauffé à 60°C sous agitation pendant 24h puis à température ambiante pendant 5 jours. Le mélange est ensuite filtré sur fritté P3, rincé avec CH₃CN sec et les eaux mères sont évaporées. Le produit brut est purifié sur colonne chromatographique (Al₂O₃, CH₂Cl₂/MeOH, 100% à 97/3) afin d'obtenir le produit pur sous forme d'une poudre blanche (41 mg, 65%). ***R_{f}*** (Al₂O₃, CH₂Cl₂/MeOH, 95/5) = 0.54 ; **¹H NMR (500 MHz, CDCl₃, δ):** 8.15 (s, 3H, H³), 7.92 (s, 3H, H⁵), 7.25 (d, 3H, J = 8.0 Hz, H¹²), 6.54-6.49 (m, 6H, H⁹/H¹¹), 3.99 (s, 6H, H¹⁷), 3.97 (s, 9H, H¹⁶), 3.84 (s, 9H, H¹³), 3.75 (s, 9H, H¹⁴), 2.99 (s, 12H, H¹⁸) ; **¹³C NMR (125 MHz, CDCl₃, δ):** 166.5 (C¹⁵), 161.9 (C¹⁰), 160.9 (C⁶), 158.0 (C⁸), 147.8 (C⁴), 147.2 (C²), 131.4 (C¹²), 126.6 (C⁵), 124.4 (C³), 119.9 (C⁷), 105.3 (C⁹), 99.2 (C¹¹), 65.0 (C¹⁷), 56.2 (C¹⁸), 55.7 (C¹³), 55.6 (C¹⁴), 53.0 (C¹⁶).

### Composé 7c

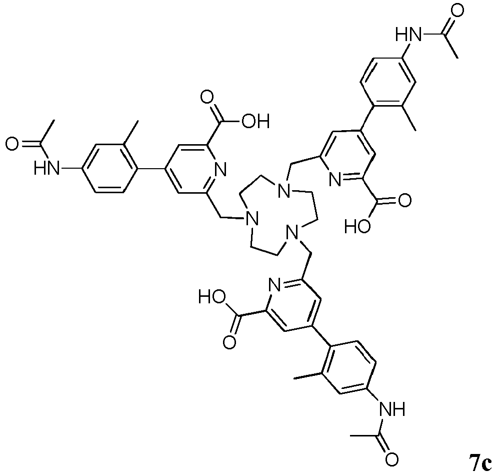

**6c** (47 mg, 0.05 mmol) est dissout dans le THF (3 mL) puis NaOH 1M (3 mL) est ajouté jusqu'à pH >12. Le mélange réactionnel est laissé sous agitation à température ambiante pendant 24h. Un suivi LC-MS permet d'observer la déprotection complète des esters méthyliques. Le produit est utilisé pour la complexation sans purification supplémentaire. **HRMS** (ESI) calculée pour C₅₄H₅₈N₉O₉ 976.4352. Exp 976.4306 [M+H]⁺ ; **t_{R}**= 6.24 min (méthode HPLC-MM-ACN, H₂O/CH₃CN 85/15 à 0/100 en 16 min).

### Composé 7d

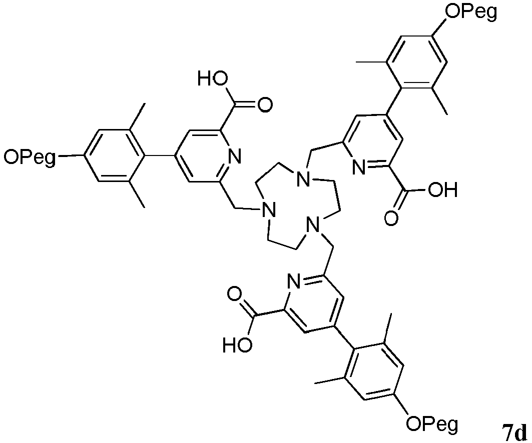

**6d** (38.7 mg, 0.028 mmol) est dissout dans le tetrahydrofurane (3 mL) et NaOH 1M (3 mL) est ajouté jusqu'à pH 14. Le mélange réactionnel est laissé sous agitation à température ambiante pendant 6h30. Le suivi par spectrométrie de masse montre la disparition du produit de départ. Le mélange est utilisé pour l'étape de complexation sans purification supplémentaire. **HRMS (ESI)** calculée pour C₇₅H₉₈N₆O₁₈ 667.3463. Exp 667.3461 [M+2H]²⁺ ; t_{R}= 9.57 min (méthode HPLC-ESI-ACN, H₂O/CH₃CN 85/15 à 0/100 en 16 min).

### Complexe Ia.1

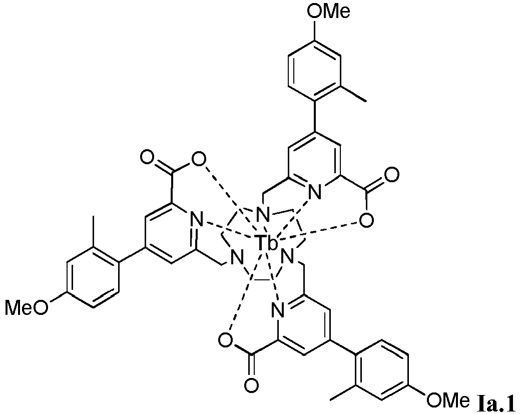

Le ligand **6a** (70 mg, 0.075 mmol) est dissout dans THF (10 mL) puis NaOH 1M (5 mL) est ajouté et la solution est agitée à température ambiante pendant 1h30. Le pH est ensuite ajusté à pH 4 avec HCl 1M (5 mL) et le mélange est concentré sous vide. Le milieu est dilué avec MeOH puis le pH est ajusté à pH 8-9 à l'aide d'une solution de Na₂CO₃ₛₐₜ. Enfin, TbCl₃.6H₂O (84 mg, 0.224 mmol) est ajouté et le mélange réactionnel est agité à température ambiante pendant 72h. Le milieu est concentré sous pression réduite puis le complexe est précipité avec ajout d'H₂O (2 x) et centrifugé pour obtenir un solide blanc correspondant au complexe de terbium(III) (79 mg, quant.). **HRMS (ESI)** calculée pour C₅₁H₅₃N₆O₉Tb 526.1558. Exp 526.1557 [M+2H]⁺⁺. Calc pour C₅₁H₅₂N₆O₉Tb 1051.3044. Exp 1051.3011 [M+H]⁺.

### Complexe Ia.2

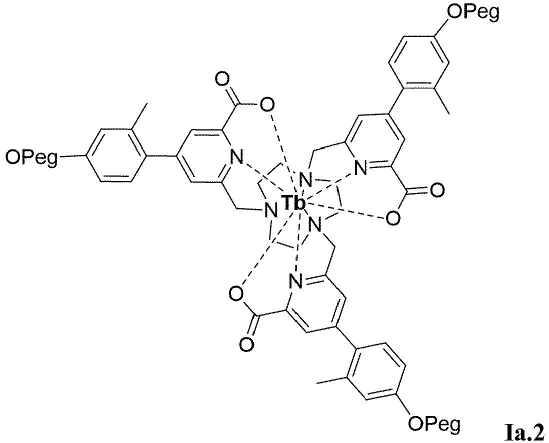

Le ligand 6b (50 mg, 0.038 mmol) est solubilisé dans MeOH (5 mL) puis NaOH 1M (2 mL) est ajouté et la solution est laissée sous agitation à température ambiante pendant 1h. Puis le pH est ajusté à pH 3∼4 avec HCl 1M, puis à pH ∼7 avec Na₂CO₃ₛₐₜ. TbCl₃.6H₂O (21 mg, 0.056 mmol) est ajouté et la solution est laissée à température ambiante pendant une nuit. Après concentration sous pression réduite, le mélange est purifié par extractions/lavages avec DCM/H₂O, et la phase organique évaporée pour donner le produit sous forme d'un solide jaune pâle (55 mg, quant.). **HRMS** (ESI) calculée pour C₆₉H₈₇N₆Na₂O₁₈Tb 746.2557. Exp 746.2565 [M+2Na]²⁺ ; **t_{R}**= 9.65 min (méthode HPLC-MM, H₂O/CH₃CN 85/15 à 0/100 en 16 min).

### Complexe Ia.3

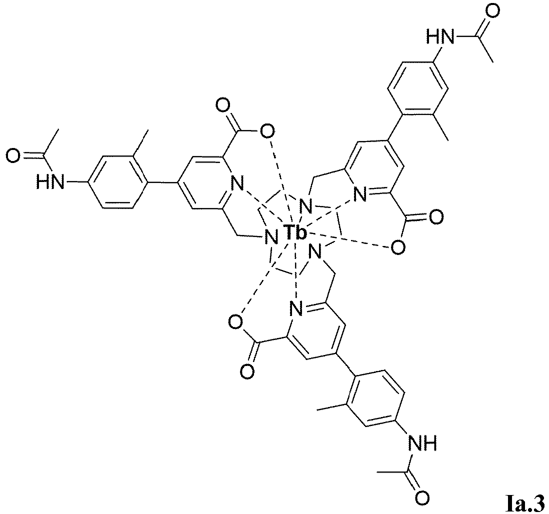

Le pH de la solution de **7c** est ajusté à pH 6 par ajout d'une solution de HCl 1M puis TbCl₃.6H₂O est ajouté à température ambiante. Le milieu réactionnel est laissé sous agitation pendant 48h puis le THF est évaporé sous pression réduite. Le précipité formé est centrifugé dans H₂O (3x) afin d'éliminer l'excès de sels présents puis le solide blanc est séché sous vide (43 mg, 83%). **HRMS** (ESI) calculée pour C₅₄H₅₅N₉O₉Tb 1132.3371. Exp 1132.3323 [M+H]⁺, C₅₄H₅₄N₉O₉TbNa 1154.3190, Exp 1154.3133 [M+Na]⁺; **t_{R}**= 6.67 min (méthode HPLC-MM-ACN, H₂O/CH₃CN 85/15 à 0/100 en 16 min).

### Complexe Ia.4

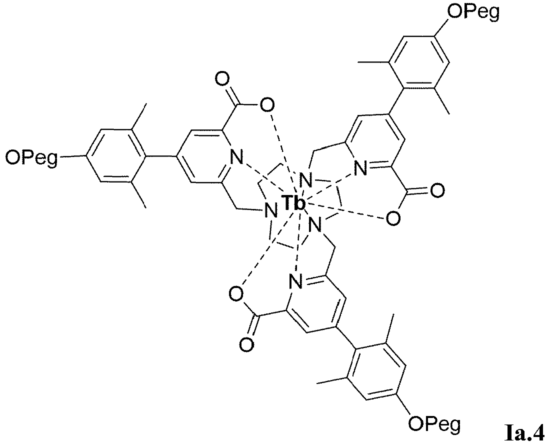

Le pH de la solution **7d** est ajusté à pH 6 avec HCl 1M puis TbCl₃.6H₂O (12 mg, 0.031 mmol) est ajouté et la solution est laissée sous agitation à température ambiante pendant 6 jours. Le THF est ensuite évaporé et une extraction CH₂Cl₂/H₂O est effectuée. Les phases organiques sont rassemblées et évaporées pour obtenir le complexe de terbium(III) pur (38 mg, 90%). **HRMS (ESI)** calculée pour C₇₂H₉₅N₆O₁₈Tb 745.2973. Exp 745.2966 [M+2H]²⁺, C₇₂H₉₄N₆O₁₈TbNa 756.2882. Exp 756.2882 [M+H+Na]²⁺ ; **t_{R}**= 9.64 min (méthode HPLC-ESI-ACN-365, H₂O/CH₃CN 85/15 à 0/100 en 16 min).

### Complexe 8 (hors invention)

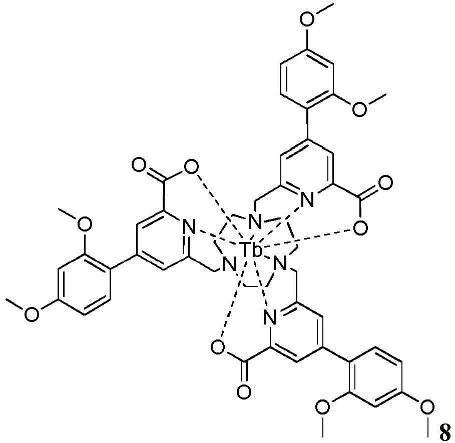

Le ligand 6e (31 mg, 0.032 mmol) est dissout dans MeOH (5 mL) puis NaOH 1M (2 mL) est ajouté et la solution est agitée à température ambiante pendant 2h. Le pH est ensuite ajusté à pH 3-4 avec HCl 1M (2 mL) puis à pH 7 à l'aide d'une solution de Na₂CO₃ₛₐₜ. TbCl₃.6H₂O (18 mg, 0.047 mmol) est enfin ajouté et le mélange réactionnel est agité à température ambiante pendant 14h. Le milieu est concentré sous pression réduite puis le complexe est précipité avec ajout d'H₂O (2 x) et centrifugé. Puis le solide obtenu est de nouveau précipité dans Et₂O et centrifugé pour obtenir un nouveau précipité blanc correspondant au complexe de terbium(III) (30 mg, 85%). **MS** Calc pour C₅₁H₅₃N₆O₁₂Tb 550.1488. Exp 550.1482 [M+2H]²⁺; calc pour C₅₁H₅₂N₆NaO₁₂Tb 561.1392. Exp 561.1405 [M+H+Na]²⁺; calc pour C₅₁H₅₁N₆Na₂O₁₂Tb 572.1301. Exp 572.1323 [M+2Na]²⁺.

### Exemple 2 : Complexe de Dysprosium Ia.5 et Ia.6

### Complexe Ia.5

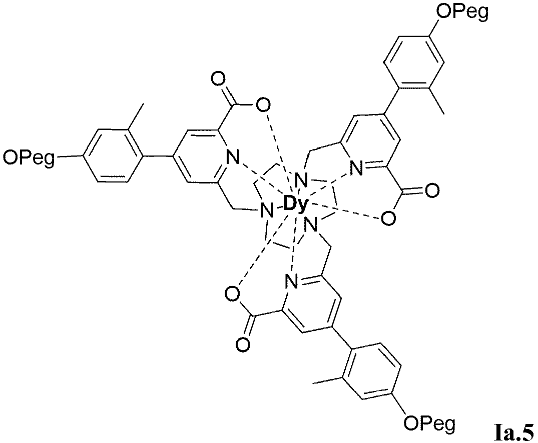

Le ligand **6b** (50 mg, 0.038 mmol) est solubilisé dans MeOH (5 mL) puis NaOH 1M (2 mL) est ajouté et la solution est laissée sous agitation à température ambiante pendant 1h. Puis le pH est ajusté à pH 3∼4 avec HCl 1M, puis à pH ∼7 avec Na₂CO₃ₛₐₜ. Dy(NO₃)₃.5H₂O (20 mg, 0.056 mmol) est ajouté et la solution est laissée à température ambiante pendant une nuit. Après concentration sous pression réduite, le mélange est purifié par extractions/lavages avec DCM/H₂O, et la phase organique évaporée pour donner le produit sous forme d'un solide jaune pâle (50 mg, 92%). **HRMS (ESI)** calculée pour C₆₉H₈₇DyN₆Na₂O₁₈ 748.7577. Exp 748.7576 [M+2Na]²⁺.

### Complexe Ia.6

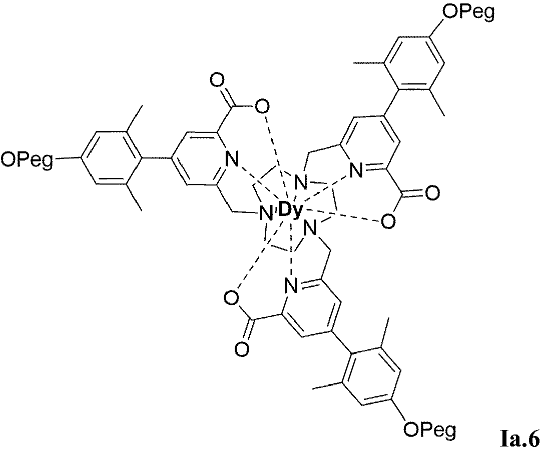

**6d** (9 mg, 0.007 mmol) est dissout dans le tetrahydrofurane (2 mL) et NaOH 1M (2 mL) est ajouté jusqu'à pH 14. Le mélange réactionnel est laissé sous agitation à température ambiante pendant 24h. Le pH de la solution est ensuite ajusté à pH 6 avec HCl 1M puis DyCl₃.6H₂O (3.2 mg, 0.008 mmol) est ajouté et la solution est laissée sous agitation à température ambiante pendant 3 jours. Le THF est ensuite évaporé et une extraction CH₂Cl₂/H₂O est effectuée. Les phases organiques sont rassemblées et évaporées pour obtenir le complexe de dysprosium(III) pur (8 mg, 77%). **HRMS (ESI)** calculée pour C₇₂H₉₃N₆O₁₈DyNa₂ 769.7811. Exp 769.7818 [M+2Na]²⁺, C₇₂H₉₃N₆O₁₈DyNa₃ 520.8505. Exp 520.8521 [M+3Na]³⁺ ; **t_{R}**= 9.57 min (méthode HPLC-ESI-ACN-365, H₂O/CH₃CN 85/15 à 0/100 en 16 min).

### B. EVALUATION DES COMPLEXES

### Exemple 3 : propriétés spectroscopiques

Les propriétés des complexes **Ia.1-Ia.6** et **8** ont été évaluées dans le méthanol (**Tableau 1**) et dans l'eau (**Tableau 2**). Ces propriétés ont été comparées à celles du complexe **9,** décrit dans le document, *Dalton Trans.* **2015,** *44,* 4918, et de structure suivante :

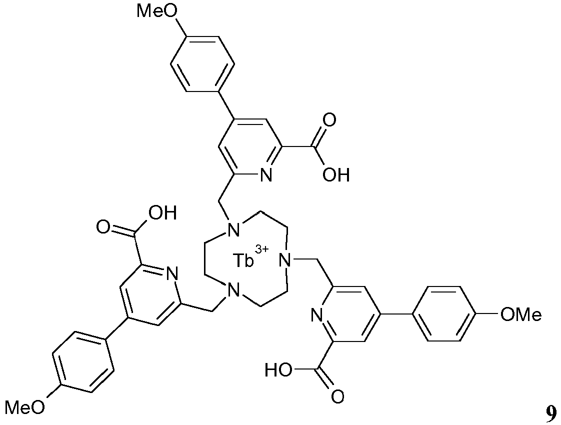

Les mesures photophysiques des complexes montrent que les complexes de terbium **Ia.1-Id.4** selon l'invention ont une absorption intense dans le méthanol (**Tableau 1**).

De plus, ces complexes possèdent des rendements quantiques très importants, et bien supérieurs à celui du complexe 9 hors invention, qui ne possède pas de substituant autre que des hydrogènes en alpha de la liaison pyridine-phényle (positions R2 et R3). La forte limitation, voire la suppression, de la rotation interne autour de la liaison pyridine-phényle permet ainsi d'améliorer significativement le rendement quantique. La brillance des complexes selon l'invention est alors supérieure à celle du complexe **9.**

Différents groupes électrodonneurs ont été testés : méthoxy, PEG ou amide. De moins bons résultats ont été observés avec un groupe amide (complexe **Ia.3**), en raison d'un moins bon rendement quantique, mais demeurent tout de même satisfaisants en termes de brillance.

En revanche, la présence d'un second groupe électrodonneur de type méthoxy sur le groupe phényle (complexe **8**) provoque une chute du rendement quantique, comparé à un groupe méthyle (complexe **Ia.1**).

L'introduction d'un second groupement méthyle a un effet majeur sur le coefficient d'extinction molaire avec une baisse de plus de 50 %. Ainsi, en dépit d'un rendement quantique excellent, **Ia.4** présente une brillance à 330 nm inférieure à celle de son homologue **Ia.2.**

Les propriétés spectroscopiques des complexes **Ia.2** et **Ia.4** sont conservées dans l'eau (**Tableau 2**).

La brillance du complexe **Ia.2** est très élevée, et comparable à celle du complexe TbLumi-4, issus des travaux du groupe de K. Raymond (J. Xu et al. J. Am. Chem. Soc. 2011, 133, 19900-19910), qui figure parmi les meilleurs complexes actuellement sur le marché (Φ = 59%, ε de l'ordre de 25000 L.mol⁻¹. cm⁻¹ et une brillance aux alentours des 15000 L.mol⁻¹. cm⁻¹).

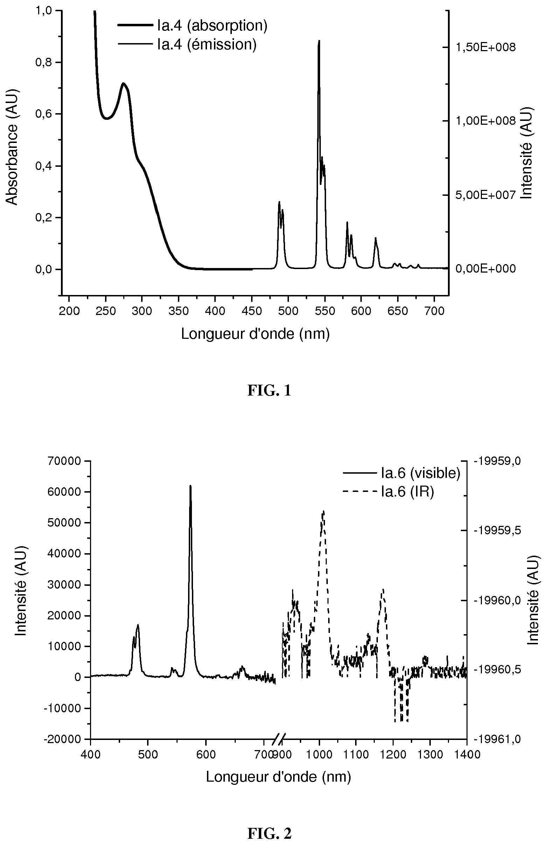

La **figure 1** présente le spectre d'absorption et d'émission du complexe **Ia.4** dans l'eau à température ambiante.

**Tableau 1-propriétés photophysiques des complexes Ia.1-Ia.6, 8 et 9 mesurées dans le méthanol**

| complexe | λ max (nm) | ε (L.mol⁻¹. cm⁻¹) | Φ (%) | T (ms) | B à λ max (L.mol⁻¹. cm⁻¹) | B à 330 nm (L.mol⁻¹.cm^{- 1}) |
|---|---|---|---|---|---|---|
| **Ia.1** | 306 | 21000 | 74 | 1.33 | 15500 | n.d. |
| **Ia.2** | 305 | 34800 | 74 | 1.32 | 26000 | 10000 |
| **Ia.3** | 305 | 34800 | 35 | 0.79 | 12000 | 5600 |
| **Ia.4** | 274 | 19000 | 65 | 1.59 | 12000 | 3400 |
| | 302 | 13000 | | | 8500 | |
| **8 (hors invention)** | 325 | 51000 | 13 | 0.22 | 6600 | 6500 |
| **Ia.5** | 306 | n.d. | 2.8 | 0.021 | n.d. | n.d. |
| **Ia.6** | 301 | n.d. | 2.6 | 0.020 | n.d. | n.d. |
| **9 (hors invention)** | 308 | 33000 | 12 | n.d. | 3900 | n.d. |

**Tableau 2 - propriétés photophysiques des complexes Ia.2 et Ia.4-Ia.6 mesurées dans l'eau**

| complexe | λ max (nm) | ε (L.mol⁻¹. cm⁻¹) | Φ (%) | T (ms) | B à λ max (L.mol⁻¹. cm⁻¹) | B à 330 nm (L.mol⁻¹.cm⁻¹) |
|---|---|---|---|---|---|---|
| **Ia.2** | 302 | 30400 | 74 | 1.36 | 23000 | 8900 |
| **Ia.4** | 275 | 21000 | 66 | 1.46 | 14000 | 2500 |
| | 300 | 11800 | | | 7800 | |
| **Ia.5** | 302 | n.d. | 1.8 | 0.017 | n.d. | n.d. |
| **Ia.6** | 300 | n.d. | 2.5 | 0.028 | n.d. | n.d. |

Les complexes de Dysprosium **Ia.5** et **Ia.6** présentent également des propriétés photophysiques remarquables, dans le méthanol et dans l'eau. Ces complexes sont parmi les complexes les plus brillants rapportés dans la littérature.

La **figure 2** présente le spectre d'absorption et d'émission du complexe **Ia.6** dans un mélange méthanol/éthanol à température ambiante.

### Exemple 4 : imagerie cellulaire

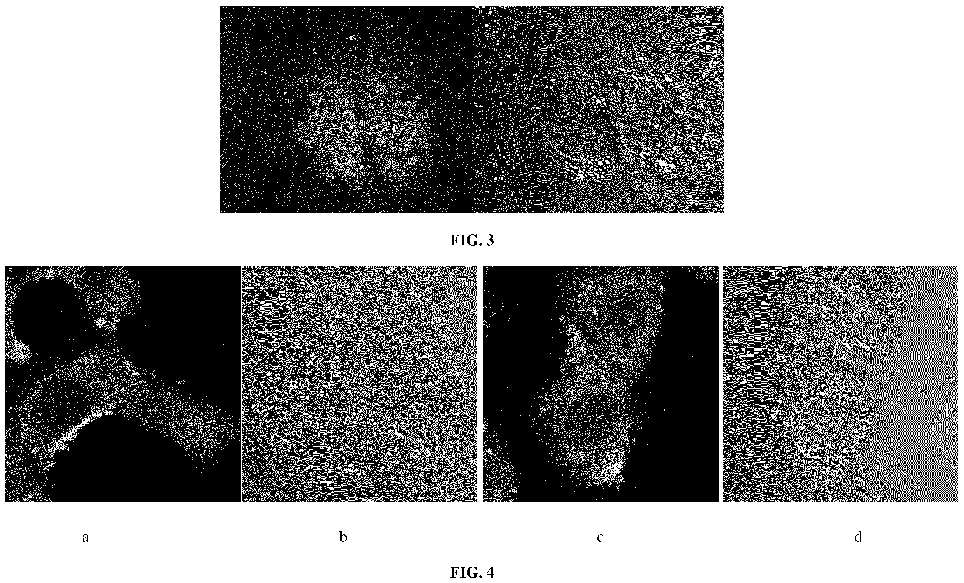

Le complexe **Ia.2** a montré un intérêt important en imagerie par microscopie de fluorescence à un ou deux photons. La **figure 3** représente des images de cellules fixées au PFA et marquées par le complexe **Ia.2** (concentration = 10⁻⁵ mol.L⁻¹, excitation biphotonique, λₑₓ = 720 nm), qui montre bien son internalisation dans la cellule. Les spectres d'émission confirment la présence de **Ia.2** à l'intérieur de la cellule et non dans le tampon.
La **figure 4** représente des images de cellules T24 fixées au PFA et marquées par le complexe **Ia.5** (concentration = 10⁻⁵ mol.L⁻¹, excitation biphotonique, λₑₓ = 720 nm). De même que pour le complexe **Ia.2,** l'internalisation du complexe **Ia.5** dans la cellule est confirmée par les spectres d'émission.

## Revendications

1. Complexe de lanthanide comportant un agent chélatant, formé d'un macrocycle ou d'un ligand, complexant un ion lanthanide **Ln³⁺, caractérisé en ce que** le lanthanide est choisi parmi le terbium et le dysprosium et **en ce que** l'agent chélatant comprend au moins un groupe (**B**) de structure suivante : 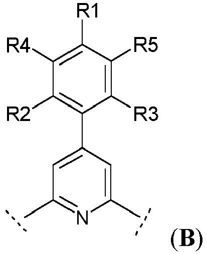 dans laquelle :
- **-R1** représente un hydrogène, un groupe **-R6,** ou un groupe électrodonneur **-E1,**
- **-R2** représente un hydrogène, un groupe **-R7,** ou un groupe électrodonneur **-E2,**
- **-R3** représente un hydrogène, un groupe **-R8,** ou un groupe électrodonneur **-E3,**
- **-R4** et **-R5,** identiques ou différents, représentent indépendamment l'un de l'autre un hydrogène ou un groupe **-R9** ou -O**R9,**
- **-E1, -E2** et **-E3** sont choisis indépendamment les uns des autres parmi les groupes **-OR10, -SR10,** -NH(CO)**R10**, -NH(CO)N**R10R'10**, -NH(CS)N**R10 R'10** et -NH(CS)NH**R10**,
- **-R6, -R7, -R8, -R9, -R10** et **R'10** identiques ou différents, représentent indépendamment les uns des autres un groupe (C1-C6) alkyle, éventuellement substitué par un groupe **-X1** ou un groupe **-Y,**
- **-X1** est un groupe réactif permettant son couplage par liaison covalente à une biomolécule,
- **-Y** est un groupe hydrosolubilisant, choisi parmi les groupes -SO₃⁻, -COO⁻, sulfobétaïne et -O-[(CH₂)₂-O]m-CH₃, m étant un nombre entier allant de 1 à 10,
- étant entendu que :
∘ au moins l'un des substituants **-R2** et **-R3** n'est pas un hydrogène,
∘ un seul des groupes **-R1, -R2** et **-R3** représente un groupe électrodonneur,
∘ ledit agent chélatant comprend au plus un groupe réactif.

2. Complexe de lanthanide selon la revendication 1 choisi parmi les complexes de lanthanide de formule (**I**) : 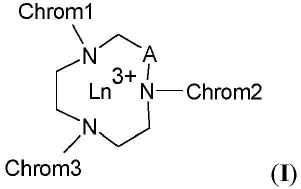 dans lesquels :
- **Ln** est un lanthanide choisi parmi Tb et Dy,
- **-A-** représente -CH₂- ou -CH(**L_{A}-X2**)-,
- **-L_{A}-** est une liaison covalente, ou un groupe (C1-C20) alkylène, linéaire ou ramifié, contenant éventuellement une ou plusieurs doubles ou triples liaisons, et éventuellement substitué par un à trois groupes -SO₃H, ou **-L_{A}-** est un groupe (C5-C8) cycloalkylène ou un groupe (C6-C14) arylène,
- **-X2** est un groupe réactif permettant son couplage par liaison covalente à une biomolécule,
- **-Chrom1, -Chrom2** et **-Chrom3** sont identiques ou différents et choisis indépendamment les uns des autres parmi les groupes de formule (**B1**) : 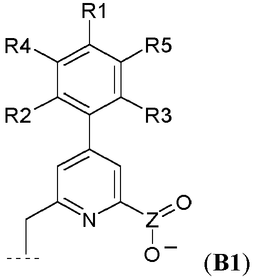
- **-R1, -R2, -R3, -R4,** et **-R5** sont tels que définis à la revendication 1,
- **-Z-** représente -C- ou -P(**R_{Z}**)-, et
- **-R_{Z}** représente un groupe phényle, benzyle, méthyle, éthyle, propyle, n-butyle, sec-butyle, iso-butyle ou tert-butyle, et de préférence un groupe phényle ou méthyle,
- étant entendu que si **-A-** est -CH(**L_{A}-X2**)-, alors aucun des groupes **-Chrom1, -Chrom2** et **-Chrom3** de formule (**B1**) ne comprend de groupe **-X1.**

3. Complexe de lanthanide selon la revendication 2, **caractérisé en ce que -A-** représente -CH₂- et **-Z-** représente -C-.

4. Complexe de lanthanide selon la revendication 2 ou 3, **caractérisé en ce que** :
- **-Chrom1, -Chrom2** et **-Chrom3** sont identiques,
- **-R1** représentant un groupe électrodonneur **-E1** tel que défini à la revendication 1,
- **-R2** étant un groupe **-R7** tel que défini à la revendication 1, et
- **-R3** étant un groupe **-R8** tel que défini à la revendication 1.

5. Complexe de lanthanide selon la revendication 2 ou 3 dans lesquels :
- **-A-** représente -CH₂-,
- **-Chrom1** et **-Chrom2** sont identiques et sont de structure (**B2**) : 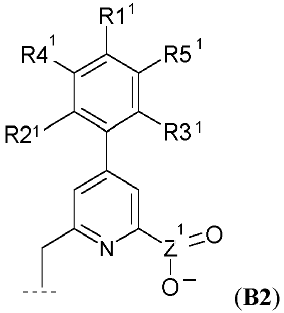 avec **-R1¹, -R2¹, -R3¹, -R4¹, -R5¹** et **-Z¹-** tels que définis respectivement pour **R1, -R2, -R3, -R4, -R5** et **-Z-** dans l'une des revendications 1 à 3, étant entendu que **Chrom1** et **-Chrom2** ne comprennent pas de groupe réactif, et
- **-Chrom3** est différent de **-Chrom1** et de **-Chrom2** et est de structure (**B3**) : 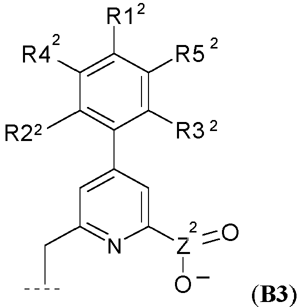
- avec **-R1², -R2², -R3², -R4², -R5²** et **-Z²-** tels que définis respectivement pour **-R1, -R2, -R3, -R4, -R5** et **-Z-** dans l'une des revendications 1 à 3, étant entendu que-**Chrom3** comprend un groupe réactif.

6. Complexe de lanthanide selon la revendication 1 choisi parmi les complexes de lanthanides de formule (**II**) : 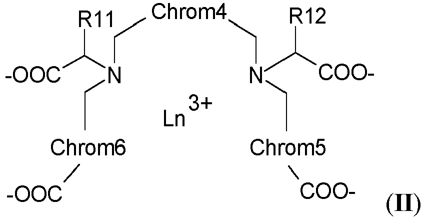 dans lesquels :
- **Ln** est un lanthanide choisi parmi Tb et Dy,
- **-R11** et **-R12,** identiques ou différents, sont choisis indépendamment l'un de l'autre parmi **-X1, -Y,** un atome d'hydrogène ou un groupe (C1-C6) alkyle,
- **-Chrom4-, -Chrom5-,** et **-Chrom6-,** identiques ou différents, représentent indépendamment l'un de l'autre un groupe de formule (**B**), tel que défini à la revendication 1.

7. Complexe de lanthanide selon la revendication 6, **caractérisé en ce que** :
- **-Chrom4-, -Chrom5-** et **-Chrom6-** sont identiques,
- **-R1** représentant un groupe électrodonneur **-E1** tel que défini à la revendication 1,
- **-R2** étant un groupe **-R7** tel que défini à la revendication 1, et
- **-R3** étant un groupe **-R8** tel que défini à la revendication 1.

8. Complexe de lanthanide selon la revendication 6, **caractérisé en ce que** :
• soit :
- **-Chrom5-** et **-Chrom6-** sont identiques et sont de structure (**B10**) : 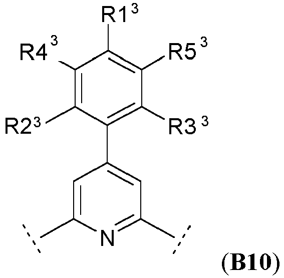 avec **-R1³, -R2³, -R3³, -R4³,** et **-R5³** tels que définis respectivement pour **-R1, -R2, -R3, -R4,** et **-R5** dans la revendication 1, étant entendu que **-Chrom5-** et **-Chrom6-**ne comprennent pas de groupe réactif,
- **-Chrom4-** est différent de **-Chrom5-** et de **-Chrom6-** et est de structure (**B11**) : 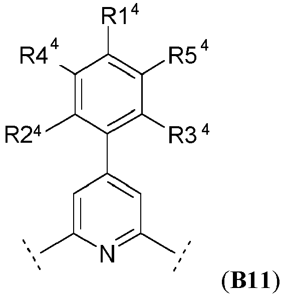 avec **-R1⁴, -R2⁴, -R3⁴, -R4⁴** et **-R5⁴** tels que définis respectivement pour **-R1, -R2, -R3, -R4** et **-R5** dans la revendications 6, étant entendu que **-Chrom4-** comprend un groupe réactif **-X1** permettant son couplage par liaison covalente à une biomolécule,
• soit :
- **-Chrom4-** et **-Chrom6-** sont identiques et sont de structure (**B12**) : 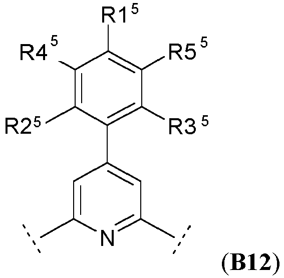 avec **-R1⁵, -R2⁵, -R3⁵, -R4⁵** et **-R5⁵** tels que définis respectivement pour **-R1, -R2, -R3, -R4** et **-R5** dans la revendication 1, étant entendu que **-Chrom4-** et **-Chrom6-**ne comprennent pas de groupe réactif, et
- **-Chrom5-** est différent de **-Chrom4-** et de **-Chrom6-** et est de structure (**B13**) : 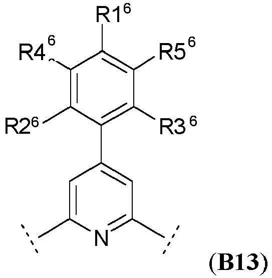 avec **-R1⁶, -R2⁶, -R3⁶, -R4⁶** et **-R5⁶** tels que définis respectivement pour **-R1, -R2, -R3, -R4** et **-R5** dans la revendication 1, étant entendu que **-Chrom5-** comprend un groupe réactif **-X1** permettant son couplage par liaison covalente à une biomolécule.

9. Complexe de lanthanide selon l'une quelconque des revendications 1 à 8, **caractérisé en ce que -R4** et **-R5** représentent des hydrogènes.

10. Complexe de lanthanide selon l'une quelconque des revendications 1 à 9, **caractérisé en ce que** les groupements réactifs **-X1** et **-X2** sont indépendamment choisis parmi - COOH, -NH₂, un acrylamide, une amine activée, un ester activé, un aldéhyde, un halogénure d'alkyle, un anhydride, une aniline, un azide, une aziridine, un acide carboxylique, un diazoalcane, un haloacétamide, une halotriazine, une hydrazine, un imido ester, un isocyanate, un isothiocyanate, un maléimide, un halogénure de sulfonyle, un thiol, une cétone, un halogénure d'acide, un ester d'hydroxysuccinimidyle, un ester de succinimidyle, un ester d'hydroxysulfosuccinimidyle, un azidonitrophényle, un azidophényle, un 3-(2-pyridyl dithio)-propionamide, un glyoxal, une triazine, un groupe acétylénique, et les groupes de formule : 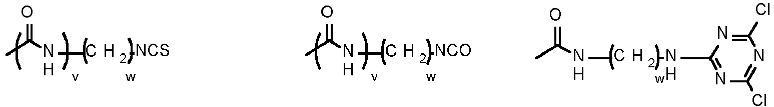 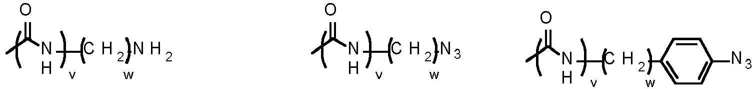 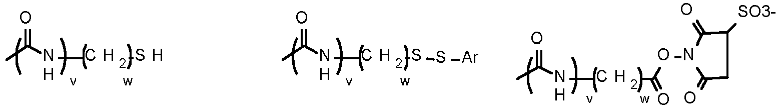 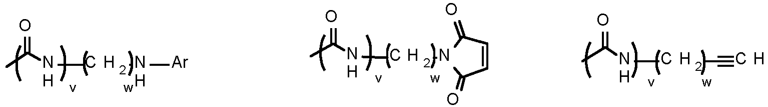 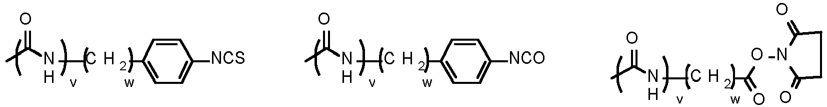 dans lesquels **w** est un entier appartenant à la gamme allant de 0 à 8 et v est égal à 0 ou 1, et **Ar** est un hétérocycle à 5 ou 6 chaînons saturé ou insaturé, comprenant 1 à 3 hétéroatomes, éventuellement substitué par un atome d'halogène.

11. Complexe de lanthanide selon l'une quelconque des revendications 1 à 10, **caractérisé en ce qu'**il comprend un seul groupe réactif permettant son couplage par liaison covalente à une biomolécule, ledit groupe réactif étant, de préférence, choisie parmi - COOH, -NH₂, un acrylamide, une amine activée, un ester activé, un aldéhyde, un halogénure d'alkyle, un anhydride, une aniline, un azide, une aziridine, un acide carboxylique, un diazoalcane, un haloacétamide, une halotriazine, une hydrazine, un imido ester, un isocyanate, un isothiocyanate, un maléimide, un halogénure de sulfonyle, un thiol, une cétone, un halogénure d'acide, un ester d'hydroxysuccinimidyle, un ester de succinimidyle, un ester d'hydroxysulfosuccinimidyle, un azidonitrophényle, un azidophényle, un 3-(2-pyridyl dithio)-propionamide, un glyoxal, une triazine, un groupe acétylénique, et les groupes de formule : 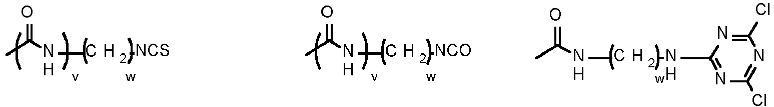 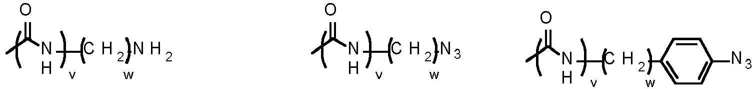 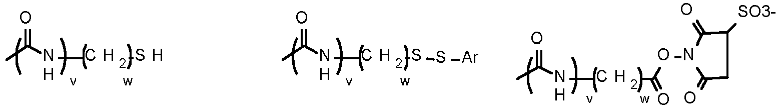 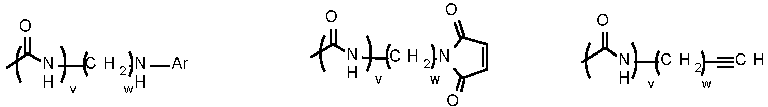 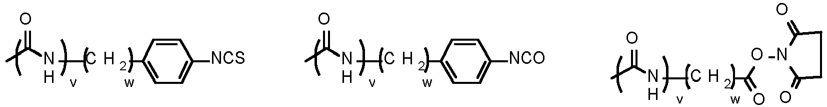 dans lesquels **w** est un entier appartenant à la gamme allant de 0 à 8 et v est égal à 0 ou 1, et **Ar** est un hétérocycle à 5 ou 6 chaînons saturé ou insaturé, comprenant 1 à 3 hétéroatomes, éventuellement substitué par un atome d'halogène ;
les groupes réactifs choisis parmi -COOH, -NH₂, les esters de succinimidyle, les haloacétamides, les azides, les hydrazines, les isocyanates et les maléimides étant préférées.

12. Complexe de lanthanide selon l'une quelconque des revendications 1 à 11, **caractérisé en ce que** le groupe **Y** est choisi parmi -SO₃⁻, -COO⁻, les groupes sulfobétaine et -O-[(CH₂)₂-O]ₘ-CH₃, m étant un nombre entier allant de 1 à 10, de préférence m=3.

13. Complexe de lanthanide selon l'une quelconque des revendications 1 à 12, **caractérisé en ce que Ln** est du terbium.

14. Agent chélatant de formule (**III**) ou de formule (**IV**) : 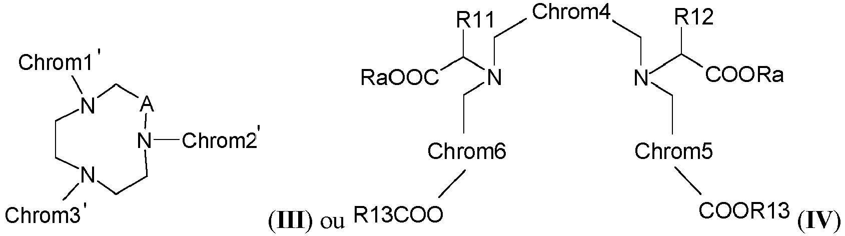
- avec **-Chrom1', -Chrom2',** et **-Chrom3',** identiques ou différents, et choisis indépendamment les uns des autres parmi les groupes de formule (**B5**) : 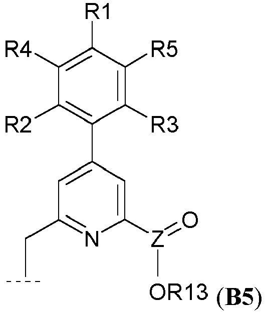
- avec **R1, R2, R3, R4** et **R5** tels que définis à la revendication 1,
- avec **-Chrom4-, -Chrom5-** et **-Chrom6-** tels que définis à l'une quelconque des revendications 6 à 8,
- et **-R13** un groupe protecteur des fonctions acide, ou sous la forme d'un sel.

15. Procédé de détection d'une biomolécule comprenant la détection de la luminescence d'un conjugué de ladite biomolécule avec un complexe luminescent selon l'une quelconque des revendications 1 à 13, comprenant un groupe réactif, et obtenu par couplage de ladite biomolécule avec ledit complexe luminescent sur son groupe réactif.

## Patentansprüche

1. Lanthanidkomplex, umfassend einen Chelatbildner, gebildet von einem Makrocyclus oder einem Ligand, ein Lanthanid-Ion **Ln³⁺** komplexierend, **dadurch gekennzeichnet, dass** das Lanthanid aus Terbium und Dysprosium ausgewählt ist und dass der Chelatbildner mindestens eine Gruppe (**B**) mit der folgenden Struktur umfasst: 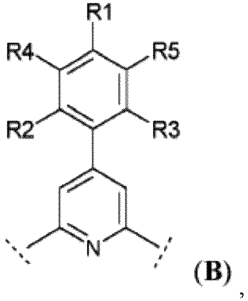 in der:
- **-R1** für einen Wasserstoff, eine Gruppe **-R6** oder eine Elektronendonatorgruppe **-E1** steht,
- **-R2** für einen Wasserstoff, eine Gruppe **-R7** oder eine Elektronendonatorgruppe **-E2** steht,
- **-R3** für einen Wasserstoff, eine Gruppe **-R8** oder eine Elektronendonatorgruppe **-E3** steht,
- **-R4** und **-R5,** gleich oder verschieden, unabhängig voneinander für einen Wasserstoff oder eine Gruppe **-R9** oder **-OR9** stehen,
- **-E1, -E2** und **-E3** unabhängig voneinander aus den Gruppen **-OR10, -SR10,** -NH(CO)**R10**, -NH(CO)N**R10R'10**, -NH(CS)N**R10R'10** und -NH(CS)NH**R10** ausgewählt sind,
- **-R6, -R7, -R8, -R9, -R10** und **R'10,** gleich oder verschieden, unabhängig voneinander für eine (C1-C6)-Alkylgruppe stehen, eventuell durch eine Gruppe **-X1** oder eine Gruppe **-Y** substituiert,
- **-X1** eine reaktive Gruppe ist, seine Kopplung an ein Biomolekül durch kovalente Bindung ermöglichend,
- **-Y** eine wasserlöslich machende Gruppe ist, ausgewählt aus den Gruppen -SO₃⁻, -COO⁻, Sulfobetain und -O-[(CH₂)₂-O]m-CH₃ ausgewählt, wobei m eine ganze Zahl ist, die von 1 bis 10 reicht,
- mit der Maßgabe, dass:
∘ mindestens einer der Substituenten **-R2** und **-R3** kein Wasserstoff ist,
∘ eine einzige der Gruppen **-R1, -R2** und **-R3** für eine Elektronendonatorgruppe steht,
∘ der Chelatbildner höchstens eine reaktive Gruppe umfasst.

2. Lanthanidkomplex nach Anspruch **1,** ausgewählt aus den Lanthanidkomplexen der Formel (**I**): 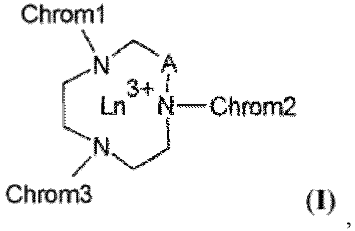 in denen:
- **Ln** ein Lanthanid ist, ausgewählt aus Tb und Dy,
- **-A-** für -CH₂- oder -CH(**L_{A}-X2**)- steht,
- **-L_{A}-** eine kovalente Bindung oder eine (C1-C20)-Alkylengruppe ist, linear oder verzweigt, eventuell eine oder mehrere Doppel- oder Dreifachbindungen enthaltend und eventuell durch eine bis drei Gruppen -SO₃H substituiert, oder **-L_{A}**-eine (C5-C8)-Cycloalkylengruppe oder eine (C6-C14)-Arylengruppe ist,
- **-X2** eine reaktive Gruppe ist, seine Kopplung an ein Biomolekül durch kovalente Bindung ermöglichend,
- **-Chrom1, -Chrom2** und **-Chrom3** gleich oder verschieden sind und unabhängig voneinander aus den Gruppen der Formel (**B1**) ausgewählt sind: 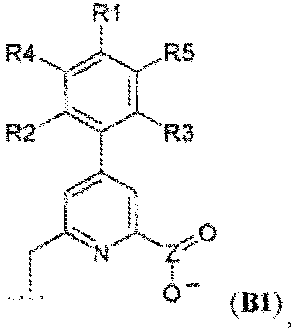
- wobei **-R1, -R2, -R3, -R4** und **-R5** wie in Anspruch **1** definiert sind,
- **-Z-** für -C- oder -P(**Rz**)- steht und
- **-Rz-** für eine Phenyl-, Benzyl-, Methyl-, Ethyl-, Propyl-, n-Butyl-, sek.-Butyl-, Isobutyl- oder tert.-Butylgruppe und vorzugsweise eine Phenyl- oder Methylgruppe steht,
- mit der Maßgabe, dass, wenn **-A-** -CH(**L_{A}-X2**)- ist, keine der Gruppen **-Chrom1, -Chrom2** und **-Chrom3** der Formel (**B1**) eine Gruppe **-X1** umfasst.

3. Lanthanidkomplex nach Anspruch **2, dadurch gekennzeichnet, dass -A-** für -CH₂- steht und **-Z-** für -C- steht.

4. Lanthanidkomplex nach Anspruch **2** oder **3, dadurch gekennzeichnet, dass**:
- **-Chrom1, -Chrom2** und **-Chrom3** gleich sind,
- **-R1** für eine Elektronendonatorgruppe **-E1** wie in Anspruch **1** definiert steht,
- **-R2** für eine Gruppe **-R7** wie in Anspruch **1** definiert steht und
- **-R3** für eine Gruppe **-R8** wie in Anspruch **1** definiert steht.

5. Lanthanidkomplex nach Anspruch **2** oder **3,** in dem:
- **-A-** für -CH₂- steht,
- **-Chrom1** und **-Chrom2** gleich sind und die Struktur (**B2**) aufweisen: 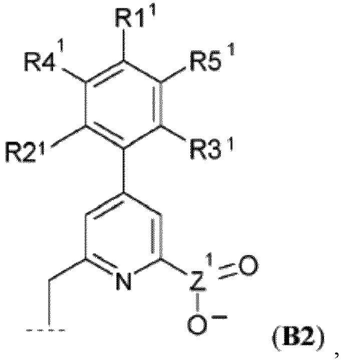 wobei **-R1¹, -R2¹, -R3¹, -R4¹, -R5¹** und **-Z¹-** wie für **R1, -R2, -R3, -R4, -R5** bzw. **-Z-** in einem der Ansprüche **1** bis **3** definiert sind, mit der Maßgabe, dass **Chrom1** und **-Chrom2** keine reaktive Gruppe umfassen, und
- **-Chrom3** sich von **-Chrom1** und **-Chrom2** unterscheidet und die Struktur (**B3**) aufweist: 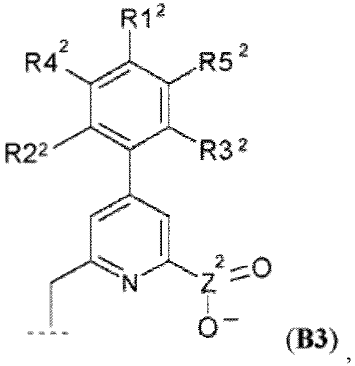
- wobei **-R1², -R2², -R3², -R4², -R5²** und **-Z²-** wie für **-R1, -R2, -R3, -R4, -R5** bzw. **-Z-** in einem der Ansprüche **1** bis **3** definiert sind, mit der Maßgabe, dass **-Chrom3** eine reaktive Gruppe umfasst.

6. Lanthanidkomplex nach Anspruch **1,** ausgewählt aus den Lanthanidkomplexen der Formel (**II**): 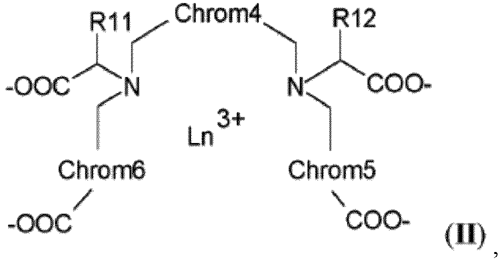 in denen:
- **Ln** ein Lanthanid ist, ausgewählt aus Tb und Dy,
- **-R11** und **-R12,** gleich oder verschieden, unabhängig voneinander aus **-X1, -Y,** einem Wasserstoffatom oder einer (C1-C6)-Alkylgruppe ausgewählt sind,
- **-Chrom4-, -Chrom5-** und **-Chrom6-,** gleich oder verschieden, unabhängig voneinander für eine Gruppe der Formel (**B**) wie in Anspruch **1** definiert stehen.

7. Lanthanidkomplex nach Anspruch **6, dadurch gekennzeichnet, dass**:
- **-Chrom4-, -Chrom5-** und **-Chrom6-** gleich sind,
- **-R1** für eine Elektronendonatorgruppe **-E1** wie in Anspruch **1** definiert steht,
- **-R2** eine Gruppe **-R7** wie in Anspruch **1** definiert ist und
- **-R3** eine Gruppe **-R8** wie in Anspruch **1** definiert ist.

8. Lanthanidkomplex nach Anspruch 6, **dadurch gekennzeichnet, dass**:
• entweder:
- **-Chrom5-** und **-Chrom6-** gleich sind und die Struktur (**B10**) aufweisen: 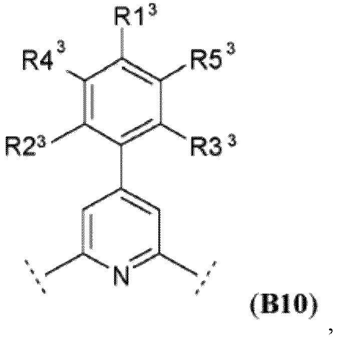 wobei **-R1³, -R2³, -R3³, -R4³** und **-R5³** wie für **-R1, -R2, -R3, -R4** bzw. **-R5** in Anspruch 1 definiert sind, mit der Maßgabe, dass **-Chrom5-** und **-Chrom6-** keine reaktive Gruppe umfassen,
- **-Chrom4-** sich von **-Chrom5-** und **-Chrom6-** unterscheidet und die Struktur (**B11**) aufweist: 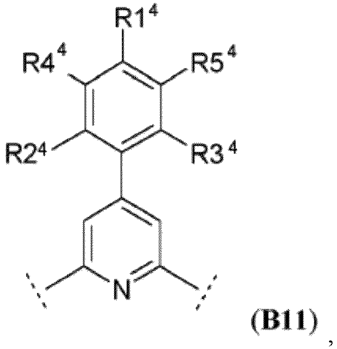 wobei **-R1⁴, -R2⁴, -R3⁴, -R4⁴** und **-R5⁴** wie für **-R1, -R2, -R3, -R4** bzw. **-R5** in Anspruch **6** definiert sind, mit der Maßgabe, dass **-Chrom4-** eine reaktive Gruppe **-X1** umfasst, seine Kopplung an ein Biomolekül durch kovalente Bindung ermöglichend,
• oder:
- **-Chrom4-** und **-Chrom6-** gleich sind und die Struktur **(B12)** aufweisen: 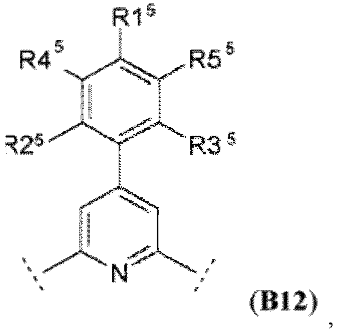 wobei **-R1⁵, -R2⁵, -R3⁵, -R4⁵** und **-R5⁵** wie für **-R1, -R2, -R3, -R4** bzw. **-R5** in Anspruch **1** definiert sind, mit der Maßgabe, dass **-Chrom4-** und **-Chrom6-** keine reaktive Gruppe umfassen, und
- **-Chrom5-** sich von **-Chrom4-** und **-Chrom6-** unterscheidet und die Struktur (**B13**) aufweist: 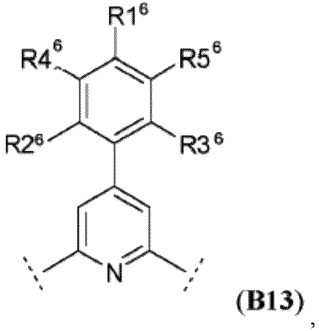 wobei **-R1⁶, -R2⁶, -R3⁶, -R4⁶** und **-R5⁶** wie für **-R1, -R2, -R3, -R4** bzw. **-R5** in Anspruch **1** definiert sind, mit der Maßgabe, dass **-Chrom5-** eine reaktive Gruppe -**X1** umfasst, seine Kopplung an ein Biomolekül durch kovalente Bindung ermöglichend.

9. Lanthanidkomplex nach einem der Ansprüche **1** bis **8, dadurch gekennzeichnet, dass -R4** und **-R5** für Wasserstoffe stehen.

10. Lanthanidkomplex nach einem der Ansprüche **1** bis **9, dadurch gekennzeichnet, dass** die reaktiven Gruppierungen -**X1** und -**X2** unabhängig aus -COOH, -NH₂, einem Acrylamid, einem aktivierten Amin, einem aktivierten Ester, einem Aldehyd, einem Alkylhalogenid, einem Anhydrid, einem Anilin, einem Azid, einem Aziridin, einer Carbonsäure, einem Diazoalkan, einem Haloacetamid, einem Halotriazin, einem Hydrazin, einem Imidoester, einem Isocyanat, einem Isothiocyanat, einem Maleinimid, einem Sulfonylhalogenid, einem Thiol, einem Keton, einem Säurehalogenid, einem Hydroxysuccinimidylester, einem Succinimidylester, einem Hydroxysulfosuccinimidylester, einem Azidonitrophenyl, einem Azidophenyl, einem 3-(2-Pyridyldithio)propanamid, einem Glyoxal, einem Triazin, einer Acetylengruppe und den Gruppen der Formel: 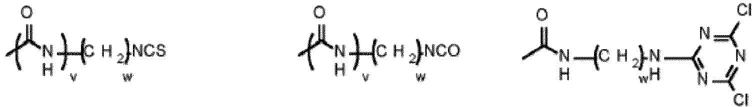 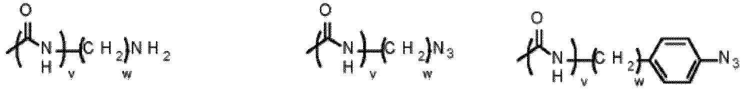 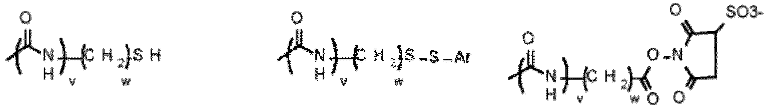 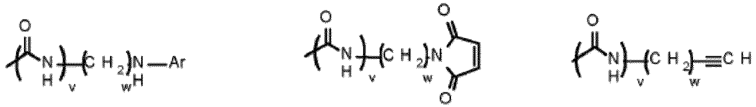 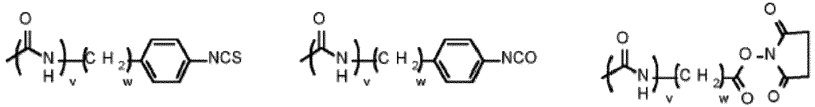 ausgewählt sind, in denen **w** eine ganze Zahl ist, die zu dem Bereich gehört, der von 0 bis 8 reicht, und **v** gleich 0 oder 1 ist und **Ar** ein gesättigter oder ungesättigter Heterocyclus mit 5 oder 6 Kettengliedern ist, umfassend 1 bis 3 Heteroatome, eventuell durch ein Halogenatom substituiert.

11. Lanthanidkomplex nach einem der Ansprüche **1** bis **10, dadurch gekennzeichnet, dass** er eine einzige reaktive Gruppe umfasst, seine Kopplung an ein Biomolekül durch kovalente Bindung ermöglichend, wobei die reaktive Gruppe vorzugsweise aus -COOH, -NH₂, einem Acrylamid, einem aktivierten Amin, einem aktivierten Ester, einem Aldehyd, einem Alkylhalogenid, einem Anhydrid, einem Anilin, einem Azid, einem Aziridin, einer Carbonsäure, einem Diazoalkan, einem Haloacetamid, einem Halotriazin, einem Hydrazin, einem Imidoester, einem Isocyanat, einem Isothiocyanat, einem Maleinimid, einem Sulfonylhalogenid, einem Thiol, einem Keton, einem Säurehalogenid, einem Hydroxysuccinimidylester, einem Succinimidylester, einem Hydroxysulfosuccinimidylester, einem Azidonitrophenyl, einem Azidophenyl, einem 3-(2-Pyridyldithio)propanamid, einem Glyoxal, einem Triazin, einer Acetylengruppe und den Gruppen der Formel: 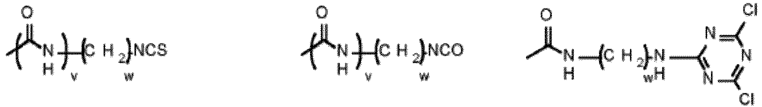 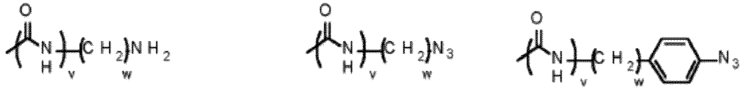 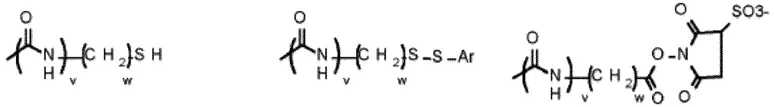 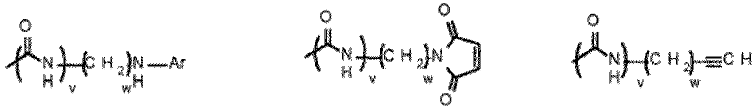 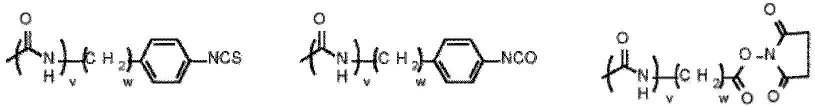 ausgewählt ist, in denen **w** eine ganze Zahl ist, die zu dem Bereich gehört, der von 0 bis 8 reicht, und **v** gleich 0 oder 1 ist und **Ar** ein gesättigter oder ungesättigter Heterocyclus mit 5 oder 6 Kettengliedern ist, umfassend 1 bis 3 Heteroatome, eventuell durch ein Halogenatom substituiert;
wobei die reaktiven Gruppen, die aus -COOH, -NH₂, Succinimidylestern, Haloacetamiden, Aziden, Hydrazinen, Isocyanaten und Maleinimiden ausgewählt sind, bevorzugt sind.

12. Lanthanidkomplex nach einem der Ansprüche **1** bis **11, dadurch gekennzeichnet, dass** die Gruppe **Y** aus -SO₃⁻, -COO⁻, Sulfobetaingruppen und -O-[(CH₂)₂-O]ₘ-CH₃ ausgewählt ist, wobei m eine ganze Zahl ist, die von 1 bis 10 reicht, vorzugsweise m = 3.

13. Lanthanidkomplex nach einem der Ansprüche **1** bis **12, dadurch gekennzeichnet, dass Ln** Terbium ist.

14. Chelatbildner der Formel **(III)** oder der Formel **(IV)**: 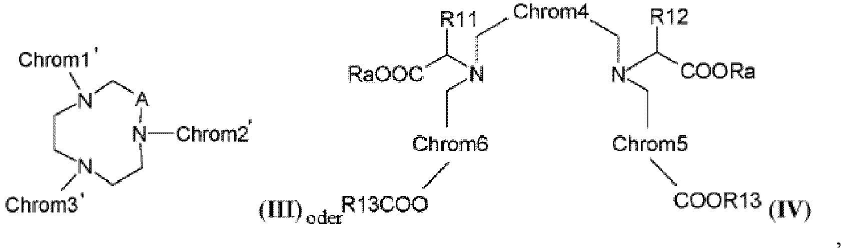
- wobei **-Chrom1'**, **-Chrom2'** und **-Chrom3',** gleich oder verschieden, unabhängig voneinander aus den Gruppen der Formel (**B5**) ausgewählt sind: 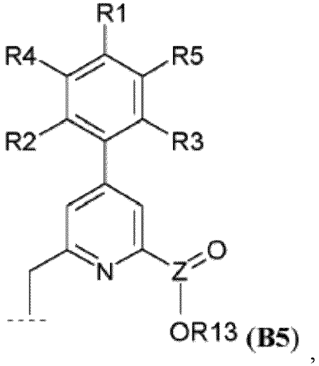
- wobei **R1**, **R2**, **R3**, **R4** und **R5** wie in Anspruch **1** definiert sind,
- wobei **-Chrom4-, -Chrom5-** und **-Chrom6-** wie in einem der Ansprüche **6** bis **8** definiert sind
- und **-R13** eine Schutzgruppe von Säurefunktionen ist oder in der Form eines Salzes ist.

15. Verfahren zum Nachweis eines Biomoleküls, umfassend den Nachweis der Lumineszenz eines Konjugats des Biomoleküls mit einem lumineszierenden Komplex nach einem der Ansprüche **1** bis **13,** umfassend eine reaktive Gruppe, und erhalten durch Kopplung des Biomoleküls mit dem lumineszierenden Komplex an seiner reaktiven Gruppe.

## Claims

1. A lanthanide complex comprising a chelating agent, formed of a macrocycle or a ligand, complexing a lanthanide ion **Ln³⁺**, **characterized in that** the lanthanide is selected from terbium and dysprosium and **in that** the chelating agent includes at least a group (B) of the following structure: 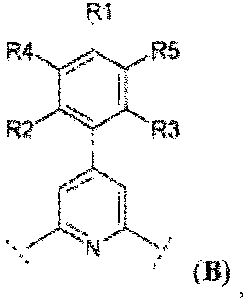 wherein:
- **-R1** represents a hydrogen, a group **-R6** or an electron-donating group **-E1**,
- **-R2** represents a hydrogen, a group **-R7** or an electron-donating group **-E2,**
- **-R3** represents a hydrogen, a group **-R8** or an electron-donating group **-E3,**
- **-R4** and **-R5,** identical or different, independently represent a hydrogen or a group -R9 or **-OR9,**
- **-E1**, **-E2** and **-E3** are independently selected from the groups: **-OR10**, **-SR10,** -NH(CO)**R10**, -NH(CO)N**R10R'10**, -NH(CS)N**R10R'10** and -NH(CS)NH**R10**,
- **-R6**, **-R7, -R8, -R9, -R10** and **R'10,** identical or different, independently represent a (C1-C6)-alkyl group, optionally substituted by a group -**X1** or a group **-Y,**
- -**X1** is a reactive group allowing its coupling by a covalent bond to a biomolecule,
- **-Y** is a water-solubilizing group selected from the groups SO₃⁻, -COO⁻", sulfobetaine and -O-[(CH₂)₂-O]m-CH₃, m being an integer ranging from 1 to 10,
- provided that:
∘ At least one of the substituents **-R2** and **-R3** is not hydrogen,
∘ Only one of groups **-R1, -R2** and **-R3** is an electron-donating group,
∘ Said chelating agent comprises at most one reactive group.

2. Lanthanide complex according to claim **1,** selected from lanthanide complexes of Formula (**I**): 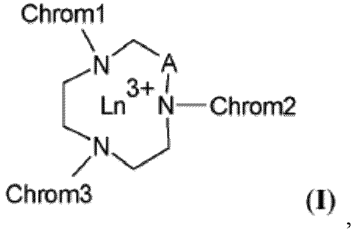 wherein:
- **Ln** is a lanthanide selected from Tb and Dy,
- **-A-** represents -CH₂- or -CH(**L_{A}-X2**)-,
- **-L_{A}-** is a covalent bond, or a (C1-C20)-Alkylene group, linear or branched, optionally comprising one or more double or triple bonds, and optionally substituted by one to three groups -SO₃H, or **-L_{A}-** is a (C5-C8)-cycloalkylene group or a (C6-C14)-arylene group,
- **-X2** is a reactive group allowing its coupling by a covalent bond to a biomolecule, - **-Chrom1**, **-Chrom2** and **-Chrom3** are identical or different, and independently selected from the groups of Formula (**B1**): 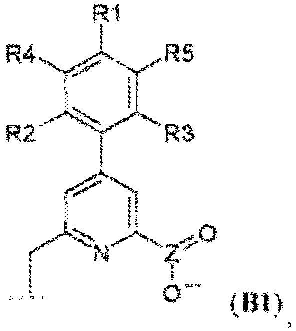
- **-R1**, **-R2, -R3, -R4** and **-R5** are as defined in claim **1,**
- **-Z-** represents -C- or -P(**Rz**)- and
- **-Rz-** represents a group phenyl, benzyl, methyl, ethyl, propyl, n-butyl, sec.-butyl, isobutyl or tert.-butyl; and preferably a group phenyl or methyl,
- provided that if **-A-** is -CH(**L_{A}-X2**)-, then none of the groups **-Chrom1**, **-Chrom2** and **-Chrom3** of Formula **(B1)** comprise a group **-X1**.

3. Lanthanide complex according to claim **2,** wherein **-A-** represents -CH₂- and **-Z-**represents -C-.

4. Lanthanide complex according to claim **2** or **3,** wherein:
- **-Chrom1**, **-Chrom2** and **-Chrom3** are identical,
- **-R1** represents an electron-donating group **-E1** as defined in claim **1,**
- **-R2** is a group **-R7** as defined in claim **1** and
- **-R3** is a group **-R8** as defined in claim **1.**

5. Lanthanide complex according to claim **2** or **3,** wherein:
- **-A-** represents -CH₂-,
- **-Chrom1** and **-Chrom2** are identical and are of structure (**B2**): 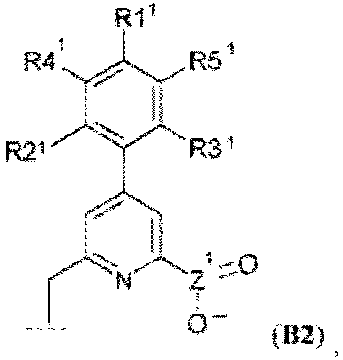 wherein -**R1¹**, **-R2¹**, **-R3¹**, **-R4¹**, **-R5¹** and **-Z¹-** are as defined respectively for **R1**, **-R2, -R3, -R4, -R5** and **-Z-** in any one of claims **1** to **3,** provided that **Chrom1** and **-Chrom2** do not comprise any reactive group, and
- **-Chrom3** is different from **-Chrom1** and **-Chrom2,** and is of structure (**B3**): 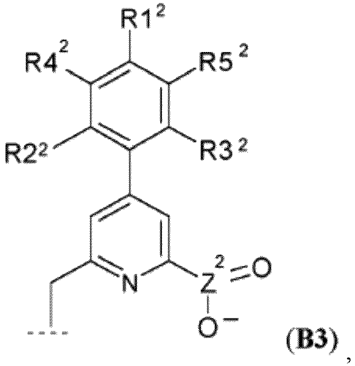
- wherein -**R1²**, **-R2²**, **-R3²**, **-R4²**, **-R5²** and **-Z²-** are as defined respectively for **-R1, -R2, -R3, -R4, -R5** and **-Z-** according to any one of claims **1** to **3,** provided that **-Chrom3** comprises a reactive group.

6. Lanthanide complex according to claim **1,** selected from Lanthanide complexes of Formula (**II**): 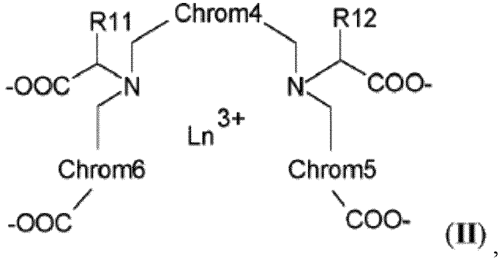 wherein:
- **Ln** is a Lanthanide selected from Tb and Dy,
- **-R11** and **-R12,** identical or different, are independently selected from -**X1**, **-Y,** a hydrogen atom or a (C1-C6)-Alkyl group,
- **-Chrom4-, -Chrom5-** and **-Chrom6-,** identical or different, independently represent a group of Formula (**B**) as defined to claim **1.**

7. Lanthanide complex according to claim **6,** wherein:
- **-Chrom4-, -Chrom5-** and **-Chrom6-** are identical,
- -**R1** represents an electron-donating group **-E1** as defined in claim **1,**
- **-R2** is a group **-R7** as defined in claim **1,** and
- **-R3** is a group **-R8** as defined in claim **1.**

8. Lanthanide complex according to claim **6,** wherein:
• either:
- **-Chrom5-** and **-Chrom6-** are identical and are of structure **(B10)**: 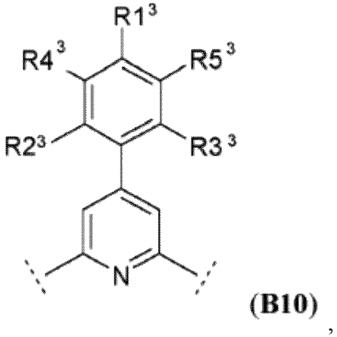 with **-R1³, -R2³, -R3³**, **-R4³** and **-R5³** are as defined respectively for **-R1, -R2, -R3, -R4** and **-R5** in claim **1,** provided that **-Chrom5-** and **-Chrom6-** do not comprise a reactive group,
- **-Chrom4-** is different from **-Chrom5-** and from **-Chrom6-** and is of structure **(B11)**: 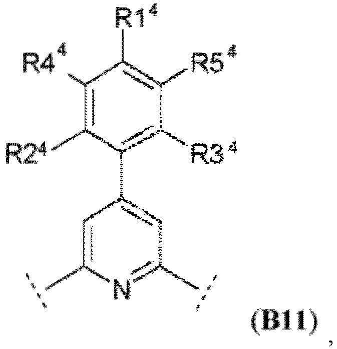 with **-R1⁴, -R2⁴, -R3⁴, -R4⁴** and **-R5⁴** are as defined respectively for **-R1, -R2, -R3, -R4** and **-R5** in claim **6,** provided that **-Chrom4-** comprises a reactive group -**X1** allowing its coupling by a covalent bond to a biomolecule,
• or:
- **-Chrom4-** and **-Chrom6-** are identical and are of structure **(B12)**: 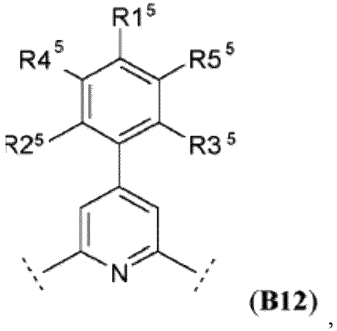 with **-R1⁵, -R2⁵, -R3⁵, -R4⁵** and **-R5⁵** are as defined respectively for **-R1, -R2, -R3, -R4** and **-R5** in claim **1,** provided that **-Chrom4-** and **-Chrom6-** do not comprise a reactive group, and
- **-Chrom5-** is different from **-Chrom4-** and from **-Chrom6-** and is of structure (**B13**): 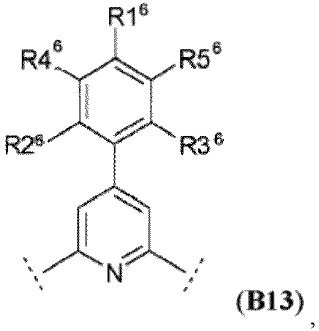 with **-R1⁶, -R2⁶, -R3⁶, -R4⁶** and **-R5⁶** are as defined respectively for **-R1, -R2, -R3, -R4** and **-R5** in claim **1,** provided that **-Chrom5-** comprises a reactive group -**X1** allowing its coupling by a covalent bond to a biomolecule.

9. Lanthanide complex according to any one of claims **1** to **8,** wherein **-R4** and **-R5** represent a hydrogen atom.

10. Lanthanide complex according to any one of claims **1** to **9,** wherein the reactive groups -**X1** and **-X2** are independently selected from -COOH, -NH₂, an acrylamide, an activated amine, an activated ester, an aldehyde, an alkyl halide, an anhydride, an aniline, an azide, an aziridine, a carbonylic acid, a diazoalkane, a halo acetamide, a halo triazine, a hydrazine, an imidoester, an isocyanate, an isothiocyanate, a maleimide, a sulfonyl halide, a thiol, a cetone, an acid halide, a hydroxysuccinimidyl ester, a succinimidyl ester, a hydroxysulfosuccinimidyl ester, an azidonitrophenyl, an azidophenyl, a 3-(2-Pyridyldithio)propionamide, a glyoxal, a triazine, an acetylenic group and the groups of Formula: 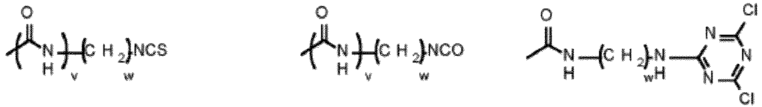 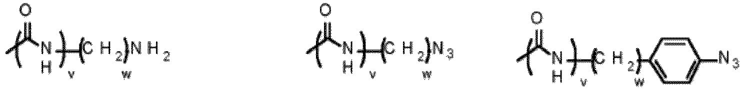 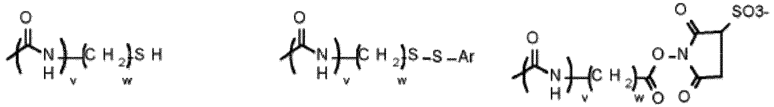 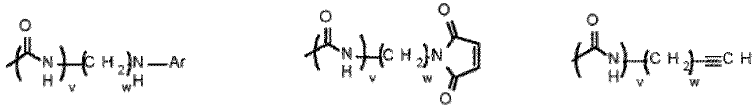 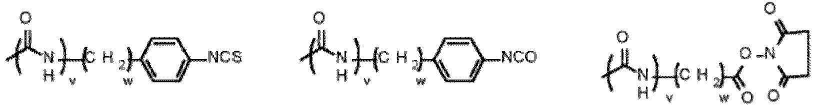 wherein **w** is an integer ranging from 0 to 8, and **v** equals to 0 or 1 and **Ar** is a heterocycle with 5 or 6 links, saturated or unsaturated, comprising 1 to 3 heteroatoms, optionally substituted by a halo atom.

11. Lanthanide complex according to any one of claims **1** to **10,** wherein its comprises only one reactive group allowing its coupling by a covalent bond to a biomolecule; said reactive group being, preferably, selected from COOH, -NH₂, an acrylamide, an activated amine, an activated ester, an aldehyde, an alkyl halide, an anhydride, an aniline, an azide, an aziridine, a carboxylic acid, a diazoalkane, a haloacetamide, a halotriazine, a hydrazine, an imidoester, an isocyanate, an isothiocyanate, a maleimide, a sulfonyl halide, a thiol, a cetone, an acid halide, a hydroxysuccinimidylester, a succinimidylester, a hydroxysulfosuccinimidylester, an azidonitrophenyl, an azidophenyl, a 3-(2-Pyridyldithio)propanamide, a glyoxal, a triazine, an acetylenic group and the groups of Formula: 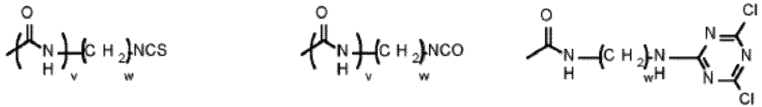 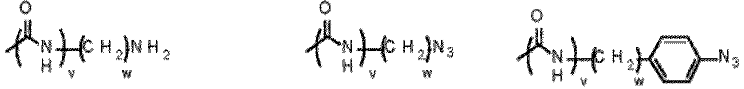 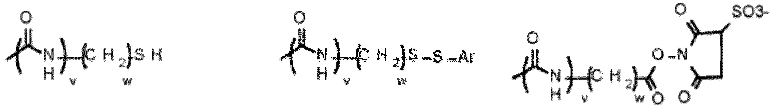 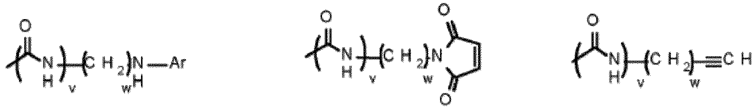 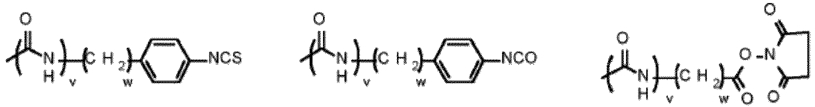 wherein **w** is an integer ranging from 0 to 8, and **v** equals to 0 or 1, and **Ar** is a heterocycle with 5 or 6 links, saturated or unsaturated, comprising 1 to 3 heteroatoms, optionally substituted by a halo atom;
the reactive groups being preferably selected from -COOH, -NH₂, succinimidyl esters, haloacetamide, azides, hydrazines, isocyanates and maleimides.

12. Lanthanide complex according to any one of claims **1** to **11,** wherein the group **Y** is selected from -SO₃⁻, -COO⁻, sulfobetaine groups and -O-[(CH₂)₂-O]ₘ-CH₃, m being an integer ranging from 1 to 10, preferably m = 3.

13. Lanthanide complex according to any one of claims **1** to **12,** wherein **Ln** is Terbium.

14. Chelating agent of Formula (**III**) or of Formula (**IV**): 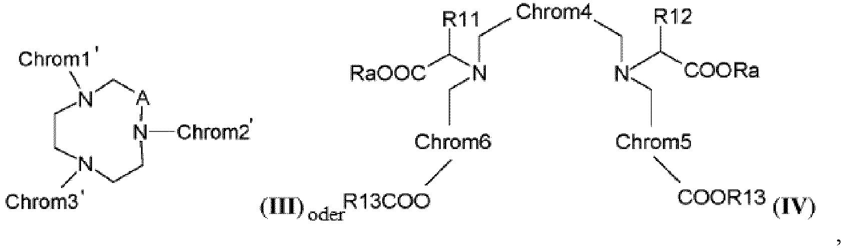
- with **-Chrom1'**, **-Chrom2'** and **-Chrom3',** identical or different, and are independently selected from the groups of Formula (**B5**): 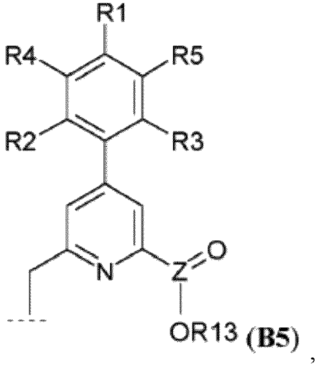
- with **R1, R2, R3, R4** and **R5** are as defined in claim **1,**
- with **-Chrom4-, -Chrom5-** and **-Chrom6-** are as defined in any one of claims **6** to **8,**
- and **-R13** a protecting group of acid functions or under the form of a salt.

15. A method for detecting a biomolecule comprising detecting the luminescence of a conjugate of said biomolecule with a luminescent complex according to any one of claims **1** to **13,** comprising a reactive group, and obtained by the coupling of said biomolecule with said luminescent complex on its reactive group.
